# EUROPEAN PATENT SPECIFICATION

(11) **EP 1 529 038 B1**
(45) Date of publication and mention of the grant of the patent: **07.10.2009**
(21) Application number: 03787754.5
(22) Date of filing: 08.08.2003
(51) Int. Cl.: C07D 277/68, C07D 417/12, A61K 31/428, A61P 29/00

(54) **BENZOTHIAZOLE DERIVATIVES HAVING BETA-2-ADRENORECEPTOR AGONIST ACTIVITY**
BENZOTHIAZOLDERIVATE MIT BETA-2-ADRENOREZEPTOR-AGONISTENWIRKUNG
DERIVES DE BENZOTHIAZOLE AYANT UNE ACTIVITE D'AGONISTE DU BETA-2-ADRENORECEPTEUR

(30) Priority: 09.08.2002 GB 0218629; 10.09.2002 GB 0220955
(43) Date of publication of application: 11.05.2005
(73) Proprietor: NOVARTIS AG, 4056 Basel (CH); Novartis Pharma GmbH, 1230 Wien (AT)
(72) Inventor: CUENOUD, Bernard, CH-1005 Lausanne (CH); FAIRHURST, Robin, Alec, Horsham West Sussex RH12 5AB (GB); TAYLOR, Roger, John, Horsham West Sussex RH12 5AB (GB)
(74) Representative: Bridle, Andrew Barry
(86) International application number: PCT/EP2003/008824
(87) International publication number: WO 2004/016601

(56) References cited:
- US-A- 5 648 370
- SUZUKI, H. ET AL: "The effects of S1319, a novel marine sponge-derived.beta.2-adrenoceptor agonist, on IgE-mediated activation of human cultured mast cells" INFLAMMATION RESEARCH (2000),49(2), 86-94, XP002258317
- SUZUKI H ET AL: "S1319: a novel beta2-adrenoceptor agonist from a marine sponge Dysidea sp" BIOORGANIC & MEDICINAL CHEMISTRY LETTERS, OXFORD, GB, vol. 9, no. 10, 17 May 1999 (1999-05-17), pages 1361-1364, XP004164892 ISSN: 0960-894X

## Description

This invention relates to organic compounds, their preparation and use as pharmaceuticals.

US 5648370 discloses arylalkylaminoalkyl derivatives that are said to be β₂-adrenoreceptor agonists and dopamine DA₂-agonists and useful for treating reversible obstructive airways diseases.

A compound designated S1319 is disclosed in Suzuki et al, Inflamn. Res. 2000 and Suzuki et al, Bio. & Med. Chem. Let. 1999. This compound, namely 4-hydroxy-7-[1-(1-hydroxy-2-methylamino)ethyl]-1,3-benzothiazol-2(3H)-one, is a short acting β₂-adrenoreceptor agonist that is isolated from a marine sponge *Dysidea sp*. from the southwest of Japan.

In a first aspect the present invention provides compounds of formula I 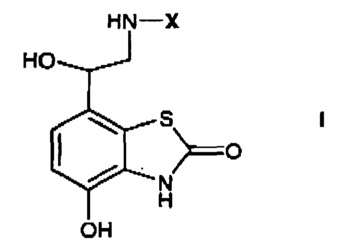 in free or salt or solvate form, wherein
X is -R¹-Ar-R² or -Rₐ-Y;
Ar denotes a phenylene group optionally substituted by halo, hydroxy, C₁-C₁₀-alkyl, C₁-C₁₀-alkoxy, C₁-C₁₀-alkoxy-C₁-C₁₀-alkyl, phenyl, C₁-C₁₀-alkyl substituted by phenyl, C₁-C₁₀-alkoxy substituted by phenyl, C₁-C₁₀-alkyl-substituted phenyl or by C₁-C₁₀-alkoxy-substituted phenyl;
R¹ and R² are attached to adjacent carbon atoms in Ar, and
   either R¹ is C₁-C₁₀-alkylene and R² is hydrogen, C₁-C₁₀-alkyl, C₁-C₁₀-alkoxy or halogen or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₁-C₁₀-alkoxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₁-C₁₀-alkoxy, C₃-C₁₀-cycloalkyl, C₇-C₁₄-aralkyl, C₇-C₁₄-aralkyloxy or C₆-C₁₀-aryl, where C₃-C₁₀-cycloalkyl, C₇-C₁₄-aralkyl, C₇-C₁₄-aralkyloxy or C₆-C₁₀-aryl are optionally substituted by halo, hydroxy, C₁-C₁₀-alkyl, C₁-C₁₀-alkoxy or halo-C₁-C₁₀-alkyl;
   C₆-C₁₀-aryl optionally substituted by halo, hydroxy, C₁-C₁₀-alkyl, C₁-C₁₀-alkoxy, C₁-C₁₀-haloalkyl, phenoxy, C₁-C₁₀-alkylthio, C₆-C₁₀-aryl, 4- to 10- membered heterocyclic ring having at least one ring nitrogen, oxygen or sulphur atom, or by NR^{b}R^{c} where R^{b} and R^{c} are each independently C₁-C₁₀-alkyl optionally substituted by hydroxy, C₁-C₁₀-alkoxy or phenyl or R^{b} may additionally be hydrogen;
   phenoxy optionally substituted by C₁-C₁₀-alkyl, C₁-C₁₀-alkoxy or by phenyl optionally substituted by C₁-C₁₀-alkyl or C₁-C₁₀-alkoxy;
   a 4- to 10-membered heterocyclic ring having at least one ring nitrogen, oxygen or sulphur atom, said heterocyclic ring being optionally substituted by halo, C₁-C₁₀-alkyl, C₁-C₁₀-alkoxy, halo-C₁-C₁₀-alkyl, C₆-C₁₀-aryl, C₇-C₁₄-aralkyl, C₇-C₁₄-aralkyloxy, C₁-C₁₀-alkoxycarbonyl or a 4- to 10-membered heterocyclyl-C₁-C₁₀-alkyl;
   -NR^{d}R^{e} where R^{d} is hydrogen or C₁-C₁₀-alkyl and R^{e} is C₁-C₁₀-alkyl optionally substituted by hydroxy, or R^{e} is C₆-C₁₀-aryl optionally substituted by halo, or R^{e} is a 4-to 10-membered heterocyclic ring having at least one ring nitrogen, oxygen or sulphur atom which ring is optionally substituted by phenyl or halo-substituted phenyl or R^{e} is C₆-C₁₀-arylsulfonyl optionally substituted by C₁-C₁₀-alkylamino or di(C₁-C₁₀-alkyl)amino;
   -SR^{f} where R^{f} is C₆-C₁₀-aryl or C₇-C₁₄-aralkyl optionally substituted by halo, C₁-C₁₀-alkyl, C₁-C₁₀-alkoxy or C₁-C₁₀-haloalkyl; or
   -CONHR^{g} where R^{g} is C₁-C₁₀-alkyl, C₃-C₁₀-cycloalkyl or C₆-C₁₀-aryl.

Terms used in the specification have the following meanings:
"Halo" or "halogen" as used herein denotes a element belonging to group 17 (formerly group VII) of the Periodic Table of Elements, which may be, for example, fluorine, chlorine, bromine or iodine. Preferably halo or halogen is fluorine or chlorine.
"C₁-C₁₀-alkyl" as used herein denotes straight chain or branched alkyl that contains one to ten carbon atoms, which may be, for example, methyl, ethyl, n-propyl, isopropyl, n-butyl, isobutyl, sec-butyl, tert-butyl, straight or branched pentyl, straight or branched hexyl, straight or branched heptyl, straight or branched octyl, straight or branched nonyl, or straight or branched decyl. Preferably, C₁-C₁₀-alkyl is C₁-C₄-alkyl.
"C₁-C₁₀-alkylene" as used herein denotes a straight chain or branched alkylene that contains one to ten carbon atoms, for example, methylene, ethylene, trimethylene, methylethylene, tetramethylene, -CH(CH₃)CH₂CH₂-, -CH₂CH(CH₃)CH₂-, straight or branched pentylene, straight or branched hexylene, straight or branched heptylene, straight or branched octylene, straight or branched nonylene, or straight or branched decylene. Preferably C₁-C₁₀-alkylene is C₁-C₄ alkylene, especially ethylene or methylethylene.
"C₂-C₁₀-alkenyl" as used herein denotes straight chain or branched hydrocarbon chains that contain two to ten carbon atoms and one or more carbon-carbon double bonds, for example, ethenyl, propenyl, isopropenyl, butenyl, isobutenyl, sec-butenyl, tert-butenyl, straight or branched pentenyl, straight or branched hexenyl, straight or branched heptenyl, straight or branched octenyl, straight or branched nonenyl, or straight or branched decenyl. Preferably "C₂-C₁₀-alkenyl" is "C₂-C₄-alkenyl".
"C₂-C₁₀-alkynyl" as used herein denotes straight chain or branched hydrocarbon chains that contain two to ten carbon atoms and one or more carbon-carbon triple bonds, for example, ethynyl, propynyl, isopropynyl, butynyl, isobutynyl, sec-butynyl, tert-burynyl, straight or branched pentynyl, straight or branched hexynyl, straight or branched heptynyl, straight or branched octynyl, straight or branched nonynyl, or straight or branched decynyl. Preferably "C₂-C₁₀-alkynyl" is "C₂-C₄-alkynyl".
"C₃-C₁₀-cycloakyl" as used herein denotes cycloalkyl having 3 to 10 ring carbon atoms, for example a monocyclic group such as a cyclopropyl, cyclobutyl, cyclopentyl, cyclohexyl, cycloheptyl, cyclooctyl, cyclononyl or cyclodecyl, any of which can be substituted by one or more, usually one or two, C₁-C₄-alkyl groups, or a bicyclic group such as bicycloheptyl or bicyclooctyl. Preferably C₃-C₁₀-cycloalkyl is C₃-C₆-cycloalkyl, for example cyclopropyl, cyclobutyl, cyclopentyl, cyclohexyl or cycloheptyl.
"C₁-C₁₀-haloalkyl" as used herein denotes C₁-C₁₀-alkyl as hereinbefore defined substituted by one or more halogen atoms, preferably one, two or three halogen atoms.
"C₁-C₁₀-alkylamino" and "di(C₁-C₁₀-alkyl)amino" as used herein denote amino substituted respectively by one or two C₁-C₁₀-alkyl groups as hereinbefore defined, which may be the same or different. Preferably C₁-C₁₀-alkylamino and di(C₁-C₁₀-alkyl)amino are respectively C₁-C₄-alkylamino and di(C₁-C₄-alkyl)amino.
"C₁-C₁₀-alkylkthio" as used herein denotes straight chain or branched C₁-C₁₀-alkylthio, which may be, for example, methylthio, ethylthio, n-propylthio, isopropylthio, n-butylthio, isobutylthio, sec-butylthio, tert-butylthio, straight or branched pentylthio, straight or branched hexylthio, straight or branched heptylthio, straight or branched octylthio, straight or branched nonylthio, or straight or branched decylthio. Preferably, C₁-C₁₀-alkylthio is C₁-C₄-alkylthio.
"C₁-C₁₀-alkoxy" as used herein denotes straight chain or branched alkoxy that contains one to ten carbon atoms which may be, for example, merhoxy, ethoxy, n-propoxy, isopropoxy, n-butoxy, isobutoxy, sec-butoxy, tert-butoxy, straight or branched pentoxy, straight or branched hexyloxy, straight or branched heptyloxy, straight or branched octyloxy, straight or branched nonyloxy, or straight or branched decyloxy. Preferably, C₁-C₁₀-alkoxy is C₁-C₄-alkoxy.
"C₁-C₁₀-alkoxy-C₁-C₁₀-alkyl" as used herein denotes C₁-C₁₀-alkyl as hereinbefore defined substituted by C₁-C₁₀-alkoxy. Preferably, C₁-C₁₀-alkoxy-C₁-C₁₀-alkyl is C₁-C₄-alkoxy-C₁-C₄-alkyl.
"C₁-C₁₀-alkoxycarbonyl" as used herein denotes C₁-C₁₀-alkoxy as hereinbefore defined linked through an oxygen atom thereof to a carbonyl group.
"C₆-C₁₀-aryl" as used herein denotes a monovalent carbocyclic aromatic group that contains 6 to 10 carbon atoms and which may be, for example, a monocyclic group such as phenyl or a bicyclic group such as naphthyl. Preferably C₆-C₁₀-aryl is C₆-C₈-aryl, especially phenyl.
"C₆-C₁₀-arylsulfonyl" as used herein denotes C₆-C₁₀-aryl as hereinbefore defined linked through a carbon atom thereof to a sulfonyl group. Preferably C₆-C₁₀-arylsulfonyl is C₆-C₈-arylsulfonyl.
"C₇-C₁₄-aralkyl as used herein denotes alkyl, for example C₁-C₄-alkyl as hereinbefore defined, substituted by aryl, for example C₆-C₁₀-aryl as hereinbefore defined. Preferably, C₇-C₁₄-aralkyl is C₇-C₁₀-aralkyl such as phenyl-C₁-C₄-alkyl, particularly benzyl or 2-phenylethyl.
"C₇-C₁₄-aralkyloxy" as used herein denotes alkoxy, for example C₁-C₄-alkoxy as hereinbefore defined, substituted by aryl, for example C₆-C₁₀-aryl. Preferably, C₇-C₁₄-aralkyloxy is C₇-C₁₀-aralkyloxy such as phenyl-C₁-C₄-alkoxy, particularly benzyloxy or 2-phenylethoxy.
Ar as used herein may be, for example, phenylene which is unsubstituted or substituted by one or more substituents selected from halogen, hydroxy, C₁-C₁₀-alkyl, C₁-C₁₀-alkoxy, C₁-C₁₀-alkoxy-C₁-C₁₀-alkyl, phenyl, or C₁-C₁₀-alkyl substituted by phenyl, C₁-C₁₀-alkoxy substituted by phenyl, C₁-C₁₀-alkyl-substituted phenyl and C₁-C₁₀-alkoxy-substituted phenyl. Preferably Ar is phenylene which is unsubstituted or substituted by one or two substituents selected from halogen, C₁-C₄-alkyl, C₁-C₄-alkoxy, or C₁-C₄-alkoxy substituted by phenyl. Preferably one substituent in Ar is para to R¹ and optional second and third substituents in Ar are meta to R¹.
"4- to 10-membered heterocyclic ring having at least one ring nitrogen, oxygen or sulphur atom" as used herein may be, for example, pyrrole, pyrrolidine, pyrazole, imidazole, triazole, tetrazole, thiadiazole, oxazole, isoxazole, thiophene, thiazole, isothiazole, oxadiazole, pyridine, pyrazine, pyridazine, pyrimidine, piperidine, piperazine, triazine, oxazine, morpholino, quinoline, isoquinoline, naphthyridine, indane or indene. Preferred heterocyclic rings include thiazole, pyrrolidine, piperidine, azacycloheptane and isoxazole.
"4 to 10-membered heterocyclyl-C₁-C₁₀-alkyl" denotes alkyl, for example C₁-C₁₀-alkyl as hereinbefore defined, substituted by a 4- to 10-membered heterocyclic ring as hereinbefore defined. Preferably, 4- to 10-membered heterocyclyl-C₁-C₁₀-alkyl is C₁-C₄-alkyl substituted by a 4- to 8-membered heterocyclic ring having at least one ring nitrogen, oxygen or sulphur atom.
"Optionally substituted" as used herein means the group referred to can be substituted at one or more positions by any one or any combination of the radicals listed thereafter.
R¹ and R² together with the carbon atoms to which they are attached as a cycloaliphatic ring may be, for example, a cyclopentane ring, optionally substituted by one or two C₁-C₄-alkyl groups, a cyclohexane ring, optionally substituted by one or two C₁-C₄-alkyl groups, or a cycloheptane ring, preferably a cyclopentane ring.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps. Preferred compounds of formula I in free or salt or solvate form include those wherein
X is -R¹-Ar-R² or -R^{a}-Y;
Ar denotes a phenylene group optionally substituted by halo, C₁-C₁₀-alkyl, C₁-C₁₀-alkoxy or by C₁-C₁₀-alkoxy substituted by phenyl;
R¹ and R² are attached to adjacent carbon atoms in Ar, and
either R¹ is C₁-C₁₀-alkylene and R² is hydrogen,
or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₃-C₁₀-cycloalkyl, C₇-C₁₄-aralkyl, C₇-C₁₄-aralkyloxy optionally substituted by halo, or by C₆-C₁₀-aryl optionally substituted by C₁-C₁₀-alkyl or C₁-C₁₀-alkoxy; C₆-C₁₀-aryl optionally substituted by halo, hydroxy, C₁-C₁₀-alkyl, phenoxy, C₁-C₁₀-alkylthio, C₆-C₁₀-aryl, a 4- to 10-membered heterocyclic ring having at least one ring nitrogen atom, or by NR^{b}R^{c} where R^{b} and R^{c} are each independently C₁-C₁₀-alkyl optionally substituted by hydroxy or phenyl or R^{b} may additionally be hydrogen; phenoxy optionally substituted by C₁-C₁₀-alkoxy; a 4- to 10-membered heterocyclic ring having at least one ring nitrogen or oxygen atom, said heterocyclic ring being optionally substituted by C₁-C₁₀-alkyl, C₆-C₁₀-aryl, C₇-C₁₄-aralkyl, C₁-C₁₀-alkoxycarbonyl or by a 4- to 10-membered heterocyclyl-C₁-C₁₀-alkyl; -NR^{d}R^{e} where R^{d} is hydrogen or C₁-C₁₀-alkyl and R^{e} is C₁-C₁₀-alkyl, or R^{e} is a 4- to 10-membered heterocyclic ring having at least one ring nitrogen or oxygen atom which ring is optionally substituted by halo-substituted phenyl or R^{e} is C₆-C₁₀-arylsuffonyl optionally substituted by di(C₁-C₁₀-alkyl)amino;
-SR^{f} where R^{f} is C₆-C₁₀-aryl or C₇-C₁₄-aralkyl optionally substituted by halo or C₁-C₁₀-haloalkyl; or -CONHR^{g} where R^{g} is C₃-C₁₀-cycloalkyl or C₆-C₁₀-aryl,

Especially preferred compounds of formula I in free or salt or solvate form include those wherein
X is -R¹-Ar-R² or -R^{a}-Y;
Ar denotes a phenylene group optionally substituted by halo, C₁-C₄-alkyl, C₁-C₄-alkoxy or by C₁-C₄-alkoxy substituted by phenyl;
R¹ and R² are attached to adjacent carbon atoms in Ar, and
either R¹ is C₁-C₄-alkylene and R² is hydrogen,
or R¹ and R² together with the carbon atoms in Ar to which they are attached denote a 5-, 6-or 7-membered cycloaliphatic ring, especially a 5-membered cycloaliphatic ring;
R^{a} is a bond or C₁-C₄-alkylene optionally substituted by hydroxy, C₆-C₈-aryl or C₇-C₁₀-aralkyl; and
Y is C₃-C₆-cycloalkyl optionally fused to one or more benzene rings and optionally substituted by C₁-C₆-akyl C₃-C₆-cycloalkyl, C₇-C₁₀-arakyl, C₇-C₁₀-aralkyloxy optionally substituted by halo, or by C₆-C₈-aryl optionally substituted by C₁-C₄-alkyl or C₁-C₄-alkoxy; C₆-C₈-aryl optionally substituted by halo, hydroxy, C₁-C₄-alkyl, phenoxy, C₁-C₄-alkylthio, C₆-C₈-aryl, a 4- to 8-membered heterocyclic ring having at least one ring nitrogen atom, or by NR^{b}R^{c} where R^{b} and R^{c} are each independently C₁-C₄-alkyl optionally substituted by hydroxy or phenyl or R^{b} may additionally be hydrogen; phenoxy optionally substituted by C₁-C₄-alkoxy; a 4- to 8-membered heterocyclic ring having at least one ring nitrogen or oxygen atom, said heterocyclic ring being optionally substituted by C₁-C₄-alkyl, C₆-C₈-aryl, C₇-C₁₀-aralkyl, C₁-C₄-alkoxycarbonyl or by a 4- to 8-membered heterocyclyl-C₁-C₄-alkyl; -NR^{d}R^{e} where R^{d} is hydrogen or C₁-C₄-alkyl and R^{e} is C₁-C₄-alkyl, or R^{e} is a 4- to 8-membered heterocyclic ring having at least one ring nitrogen or sulphur atom which ring is optionally substituted by halo-substituted phenyl or R^{e} is C₆-C₈-arylsulfonyl optionally substituted by di(C₁-C₄-alkyl)amino; - SR^{f} where R^{f} is C₆-C₈-aryl or C₇-C₁₀-aralkyl optionally substituted by halo or C₁-C₄-haloalkyl; or -CONHR^{g} where R^{g} is C₃-C₆-cycloa!kyl or C₆-C₈-aryl.

In a second aspect the present invention provides compounds of formula I in free or salt or solvate form, wherein
X is -R¹-Ar-R² or -R^{a}-Y;
Ar denotes a phenylene group optionally substituted by halo, hydroxy, C₁-C₁₀-alkyl, C₁-C₁₀-alkoxy, C₁-C₁₀-alkoxy-C₁-C₁₀-alkyl, phenyl, C₁-C₁₀-alkyl substituted by phenyl, C₁-C₁₀-alkoxy substituted by phenyl, C₁-C₁₀-alkyl-substituted phenyl or by C₁-C₁₀-alkoxy-substituted phenyl; R¹ and R² are attached to adjacent carbon atoms in Ar, and
either R¹ is C₁-C₁₀-alkylene and R² is hydrogen, C₁-C₁₀-alkyl, C₁-C₁₀-alkoxy or halogen or R¹ and R² together with the carbon atoms in Ar to which they are attached denote a 5-, 6-or 7-membered cycloaliphatic ring;
R^{a} is a C₁-C₁₀-alkylene optionally substituted by hydroxy, C₁-C₁₀-alkoxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₁-C₁₀-alkoxy, C₃-C₁₀-cycloalkyl, C₁-C₁₄-aralkyl, C₇-C₁₄-aralkyloxy or C₆-C₁₀-aryl optionally substituted by halo, hydroxy, C₁-C₁₀-alkyl, C₁-C₁₀-alkoxy or halo-C₁-C₁₀-alkyl;
C₆-C₁₀-aryl optionally substituted by halo, hydroxy, C₁-C₁₀-alkyl, C₁-C₁₀-alkoxy, C₁-C₁₀-haloalkyl, phenoxy, C₁-C₁₀-alkylthio, C₆-C₁₀-aryl, 4- to 10- membered heterocyclic ring having at least one ring nitrogen, oxygen or sulphur atom, or by NR^{b}R^{c} where R^{b} and R^{c} are each independently C₁-C₁₀-alkyl optionally substituted by hydroxy, C₁-C₁₀-alkoxy or phenyl or R^{b} may additionally be hydrogen;
phenoxy optionally substituted by C₁-C₁₀-alkyl, C₁-C₁₀-alkoxy or by phenyl optionally substituted by C₁-C₁₀-alkyl or C₁-C₁₀-alkoxy;
a 4- to 10-membered heterocyclic ring having at least one ring nitrogen, oxygen or sulphur atom, said heterocyclic ring being optionally substituted by halo, C₁-C₁₀-alkyl, C₁-C₁₀-alkoxy, halo-C₁-C₁₀-alkyl, C₆-C₁₀-aryl, C₇-C₁₄-aralkyl, C₇-C₁₄-aralkyloxy, C₁-C₁₀-alkoxycarbonyl or a 4- to 10-membered heterocyclyl-C₁-C₁₀-alkyl;
-NR^{d}R^{e} where R^{d} is hydrogen or C₁-C₁₀-alkyl and R^{e} is C₁-C₁₀-alkyl optionally substituted by hydroxy, or R^{e} is C₆-C₁₀-aryl optionally substituted by halo, or R^{e} is a 4-to 10-membered heterocyclic ring having at least one ring nitrogen, oxygen or sulphur atom which ring is optionally substituted by phenyl or halo-substituted phenyl or R^{e} is C₆-C₁₀-arylsulfonyl optionally substituted by C₁-C₁₀-alkylamino or di(C₁-C₁₀-alkyl)-amino;
-SR^{f} where R^{f} is C₆-C₁₀-aryl or C₇-C₁₄-aralkyl optionally substituted by halo, C₁-C₁₀-alkyl, C₁-C₁₀-alkoxy or C₁-C₁₀-haloalkyl; or
-CONHR^{g} where R^{g} is C₁-C₁₀-alkyl, C₃-C₁₀-cycloalkyl or C₆-C₁₀-aryl.

Preferred compounds of formula I in free or salt or solvate form include those wherein
X is -R¹-Ar-R² or -R^{a}-Y;
Ar denotes a phenylene group optionally substituted by halo, C₁-C₁₀-alkyl, C₁-C₁₀-alkoxy or by C₁-C₁₀-alkoxy substituted by phenyl;
R¹ and R² are attached to adjacent carbon atoms in Ar, and
either R¹ is C₁-C₁₀-alkylene and R² is hydrogen,
or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₃-C₁₀-cycloalkyl, C₇-C₁₄-aralkyl, C₇-C₁₄-aralkyloxy or C₆-C₁₀-aryl; C₆-C₁₀-aryl optionally substituted by halo, hydroxy, C₁-C₁₀-alkyl, phenoxy, C₁-C₁₀-alkylthio, C₆-C₁₀-aryl, a 4- to 10-membered heterocyclic ring having at least one ring nitrogen atom, or by NR^{b}R^{c} where R^{b} and R^{c} are each independently C₁-C₁₀-alkyl optionally substituted by hydroxy or phenyl or R^{b} may additionally be hydrogen; phenoxy optionally substituted by C₁-C₁₀-alkoxy; a 4- to 10-membered heterocyclic ring having at least one ring nitrogen or oxygen atom; said heterocyclic ring being optionally substituted by C₁-C₁₀-alkyl, C₆-C₁₀-aryl, C₇-C₁₄-aralkyl, C₁-C₁₀-alkoxycarbonyl or by a 4- to 10-membered heterocyclyl-C₁-C₁₀-alkyl; -NR^{d}R^{e} where R^{d} is hydrogen or C₁-C₁₀-alkyl and R^{e} is C₁-C₁₀-alkyl, or R^{e} is a 4- to 10-membered heterocyclic ring having at least one ring nitrogen or oxygen atom which ring is optionally substituted by halo-substituted phenyl or R^{e} is C₆-C₁₀-arylsulfonyl optionally substituted by di(C₁-C₁₀-alkyl)amino; -SR^{f} where R^{f} is C₆-C₁₀-aryl or C₇-C₁₄-aralkyl optionally substituted by halo or C₁-C₁₀-haloalkyl; or -CONHR^{g} where R^{g} is C₃-C₁₀-cycloalkyl or C₆-C₁₀-aryl.

Especially preferred compounds of formula I in free or salt or solvate form include those wherein
X is -R¹-Ar-R² or R^{a}-Y;
Ar denotes a phenylene group optionally substituted by halo, C₁-C₄-alkyl, C₁-C₄-alkoxy or by C₁-C₄-alkoxy substituted by phenyl;
R¹ and R² are attached to adjacent carbon atoms in Ar, and
either R¹ is C₁-C₄-alkylene and R² is hydrogen,
or R¹ and R² together with the carbon atoms in Ar to which they are attached denote a 5-, 6-or 7-membered cycloaliphatic ring, especially a 5-membered cycloaliphatic ring;
R^{a} is a bond or C₁-C₄-alkylene optionally substituted by hydroxy, C₆-C₈-aryl or C₇-C₁₀-aralkyl; and
Y is C₃-C₆-cycloalkyl optionally fused to one or more benzene rings and optionally substituted by C₁-C₄-alkyl, C₃-C₆-cycloalkyl, C₇-C₁₀-aralkyl, C₇-C₁₀-aralkyloxy or C₆-C₈-aryl; C₆-C₈-aryl optionally substituted by halo, hydroxy, C₁-C₄-alkyl, phenoxy, C₁-C₄-alkylthio, C₆-C₈-aryl, a 4- to 8-membered heterocyclic ring having at least one ring nitrogen atom, or by NR^{b}R^{c} where R^{b} and R^{c} are each independently C₁-C₄-alkyl optionally substituted by hydroxy or phenyl or R^{b} may additionally be hydrogen; phenoxy optionally substituted by C₁-C₄-alkoxy; a 4- to 8-membered heterocyclic ring having at least one ring nitrogen or oxygen atom, said heterocyclic ring being optionally substituted by C₁-C₄-alkyl, C₆-C₈-aryl, C₇-C₁₀-aralkyl, C₁-C₄-alkoxycarbonyl or by a 4- to 8-membered heterocyclyl-C₁-C₄-alkyl; -NR^{d}R^{e} where R^{d} is hydrogen or C₁-C₄-alkyl and R^{e} is C₁-C₄-alkyl, or R^{e} is a 4- to 8-membered heterocyclic ring having at least one ring nitrogen or sulphur atom which ring is optionally substituted by halo-substituted phenyl or R^{e} is C₆-C₈-arylsulfonyl optionally substituted by di(C₁-C₄-alkyl)amino; - SR^{f} where R^{f} is C₆-C₈-aryl or C₇-C₁₀-aralkyl optionally substituted by halo or C₁-C₄-haloalkyl; or -CONHR^{g} where R^{g} is C₃-C₆-cycloalkyl or C₆-C₈-aryl.

In a third aspect the present invention provides compounds of formula II 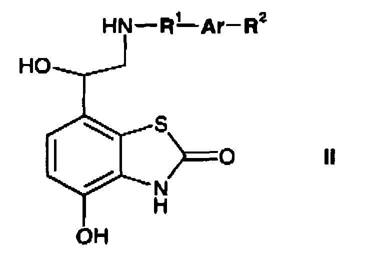 in free or salt or solvate form, where
Ar denotes a phenylene group optionally substituted by one or more substituents selected from halogen, C₁-C₈-alkyl, C₁-C₈-alkoxy, C₁-C₈-alkoxy-C₁-C₈-alkyl, or C₁-C₈-alkoxy substituted by phenyl, C₁-C₈-alkyl-substituted phenyl or by C₁-C₈-alkoxy-substituted phenyl,
R¹ and R² are attached to adjacent carbon atoms in Ar, and
either R¹ is C₁-C₈-alkylene and R² is hydrogen, C₁-C₈-alkyl, C₁-C₈-alkoxy or halogen or R¹ and R² together with the carbon atoms in Ar to which they are attached denote a 5-, 6- or 7-membered cycloaliphatic ring.

Further preferred compounds of formula I in free or salt or solvate form include those of formula III 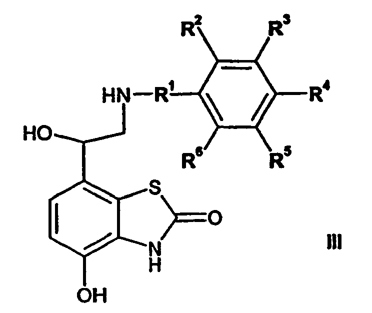 in free or salt or solvate form, where R¹ is C₂-C₄-alkylene and R² is hydrogen, or R¹ and R² together with the carbon atoms to which they are attached on the indicated benzene ring denote a 5-membered cycloaliphatic ring, R³ and R⁶ are each hydrogen, R⁴ is hydrogen, C₁-C₄-alkyl, C₁-C₄-alkoxy or C₁-C₄-alkoxy substituted by phenyl and R⁵ is hydrogen or C₁-C₄-alkyl. Especially preferred compounds of formula I in free or salt or solvate form include those of formula II where R¹ is C₂-C₃-alkylene, R², R³, R⁵ and R⁶ are each hydrogen, and R⁴ is C₁-C₄-alkyl, C₁-C₄-alkoxy or C₁-C₄-alkoxy substituted by phenyl, and those where R¹ and R² together with the carbon atoms to which they are attached on the indicated benzene ring denote a cyclopentyl group fused to the benzene ring, R³ and R⁶ are each hydrogen and R⁴ and R⁵ are each independently hydrogen or C₁-C₄-alkyl.

The compounds represented by formulae I, II or III are capable of forming acid addition salts, particularly pharmaceutically acceptable acid addition salts. Pharmaceutically acceptable acid addition salts of the compounds of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula I by known salt-forming procedures.

In formulae I, II and III, the carbon atom alpha to the phenolic ring carries a hydroxy group and so is asymmetric, so the compounds exist in individual optically active isomeric forms or as mixtures thereof, e.g. as racemic or diastereomeric mixtures. The invention embraces both individual optically active R and S isomers as well as mixtures, e.g. racemic or diastereomeric mixtures, thereof.

Specific especially preferred compounds of formulae I, II or III are those described hereinafter in the Examples.

The present invention also provides a process for the preparation of compounds of formula I in free or salt or solvate form which comprises:
(i) either (A) reacting a compound of formula IV 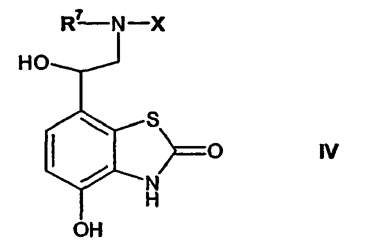 where X is as hereinbefore defined and R⁷ denotes a protecting group, to replace R⁷ by hydrogen,
   or (B) reacting a compound of formula V 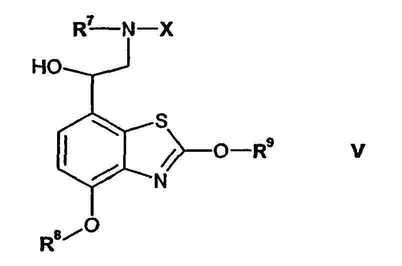 where X and R⁷ are as hereinbefore defined and R⁸ and R⁹ each independently denote a protecting group, to convert groups R⁷, R⁸ and R⁹ to hydrogen; and
(ii) recovering the compound of formula I in free or salt or solvate form.

Where reference is made herein to protected functional groups or to protecting groups, the protecting groups may be chosen in accordance with the nature of the functional group, for example as described in Protective Groups in Organic Synthesis, T.W. Greene and P.G.M. Wuts, John Wiley & Sons Inc, Second Edition, 1991, which reference also describes procedures suitable for replacement of the protecting groups by hydrogen.

The protecting group R⁷ may be, for example, a group chosen from known amine-protecting groups. Preferred protecting groups R⁷ include araliphatic groups such as benzyl. Protecting groups R⁸ and R⁹ may be chosen from known phenolic hydroxy - and alcoholic hydroxy-protecting groups respectively. Preferred groups R⁸ and R⁹ include C₁-C₄-alkyl groups, particularly branched groups such as isopropyl and tert-butyl.

Process variant (A) may be effected, for example, using known procedures for conversion of amine-protecting groups to hydrogen or analogous procedures. For example, where R⁷ is a benzyl group it may be converted to hydrogen by hydrogenolysis of the compound of formula II, e.g. with a carboxylic acid such as formic acid, preferably in the presence of a palladium catalyst. This de-protection reaction may be carried out using procedures as described hereinafter in the Examples or analogous procedures.

Process variant (B) may be effected using known procedures for conversion of hydroxy-protecting groups to hydrogen or analogous procedures. For example, where, R⁸ and R⁹ are alkyl groups, R⁸ and R⁹ may be converted to hydrogen by hydrogenolysis of the compounds of formula IV, e.g. with a carboxylic acid such as formic acid preferably in the presence of a palladium catalyst, for example as hereinbefore described for conversion of R⁷ to hydrogen, or by treatment with an acid alone such as formic acid, hydrochloric acid or trifluoroacetic acid, in either case the resulting 2-hydroxybenzothiazole compound being in tautomeric equilibrium with the benzothiazol-2-one form.

Compounds of formula IV may be prepared by reduction of a compound of formula VI 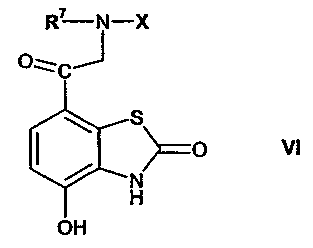 where X and R⁷ are as hereinbefore defined. The reduction may be effected using known methods for reduction of ketones to alcohols, or analogous methods, including asymmetric reductions. For example, the compounds of formula VI may be reacted with NaBH₄ in an inert solvent such as an aliphatic alcohol. Suitable reaction temperatures are from -80° C to 100° C, conveniently from -5° C to 5° C. The reduction may be effected using known procedures or analogously as described hereinafter in the Examples.

Compounds of formula V may be prepared by reacting a compound of formula VII 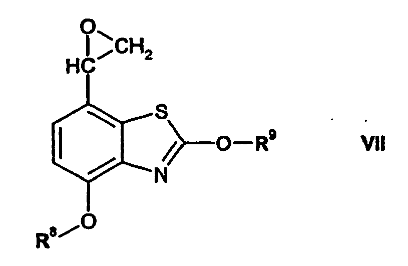 where R⁸ and R⁹ are as hereinbefore defined, with a compound of formula VIII 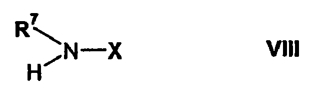 where X and R⁷ are as hereinbefore defined. The reaction of compounds of formulae VII and VIII may be effected using known procedures for epoxide-amine reactions or analogous procedures. The reaction is optionally effected in an inert organic solvent, conveniently an alcohol such a n-butanol. Suitable reaction temperatures are, for example, from 0° C to solvent reflux temperature. The reaction may be effected conveniently using a procedure as described hereinafter in the Examples, or analogously.

Compounds of formula VI may be prepared by reacting a compound of formula IX 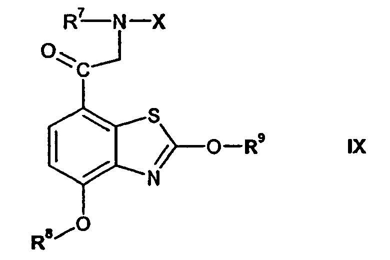 where X, R⁷, R⁸ and R⁹ are as hereinbefore described, with concentrated hydrochloric or hydrobromic acid. The reaction is preferably carried out in an inert organic solvent such as an aliphatic alcohol.

Compounds of formula VII and VIII may be prepared by known methods or analogously such as hereinafter described in the Examples.

Compounds of formula IX may be prepared by reaction of a compound of formula X 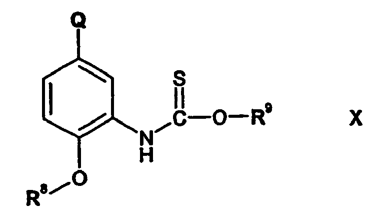 where Q is fluorine or chlorine and R⁸ and R⁹ are as hereinbefore defined, with a strong base, such as an alkyllithium, NaNH₂ or potassium tert-butoxide or a mixture of two or more thereof, and a compound of formula XI 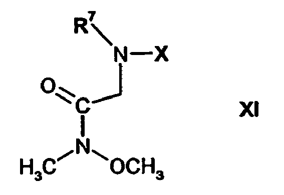 where X and R⁷ are as hereinbefore defined. The reaction is preferably effected in an inert organic solvent, for example an ether such as tetrahydrofuran (THF). Suitable reaction temperatures may be, for example, from -80° C to 80° C. The reaction may be effected using a procedure as described hereinafter in the Examples or analogous procedures.

Compounds of formulae X and XI may be prepared using known procedures or analogously, such as described hereinafter in the Examples.

Compounds of formula I in free form may be converted into salt form, and vice versa, in a conventional manner. The compounds in free or salt form can be obtained in the form of hydrates or solvates containing a solvent used for crystallisation. Compounds of formula I can be recovered from reaction mixtures and purified in a conventional manner. Isomers, such as enantiomers, may be obtained in a conventional manner, e.g. by fractional crystallisation or asymmetric synthesis from correspondingly asymmetrically substituted, e.g. optically active, starting materials.

Compounds of formula I in free or salt or solvate form are useful as pharmaceuticals.

Accordingly the invention also provides a compound of formula I in free or salt form for use as a pharmaceutical. The compounds of formula I in free or salt form, hereinafter referred to alternatively as "agents of the invention", have good β₂-adrenoreceptor agonist activity. The β₂ agonist activity, onset of action and duration of action of the agents of the invention may be tested using the guinea pig tracheal strip in vitro assay according to the procedure of R.A. Coleman and A. T. Nials, J. Pharmacol. Methods (1989), 21(1), 71-86. The binding potency can be measured by a classical filtration binding assay according to the procedure of Current Protocols in Pharmacology (S. J. Enna et al, John Wiley & Son, Inc, 1998), or by cAMP determination in cells expressing β₂-adrenoceptor, according to the procedure of B. January et al, British J. Pharmacol. 123: 701-711 (1998). For example, the compounds of Examples 1, 3, 4, 5 and 79 hereinbelow have Ki (β₂) values of 0.3 nM, 1.6 nM, 18.8 nM, 14.8 nM and 2.5 nM respectively.

The agents of the invention commonly have a rapid onset of action and have a prolonged stimulating action on the β₂-adrenoceptor, compounds of the Examples herein below having durations of action of the order of up to 24 hours. The compounds of Examples 4 and 5 have T(50%) times (in minutes) of 403 and 326 respectively at 10 nM concentration in the guinea pig tracheal strip assay, where T(50%) is the time for inhibition of contraction to decay to 50% of its maximum value.

Having regard to their β₂ agonist activity, the agents of the invention are suitable for use in the treatment of any condition which is prevented or alleviated by activation of the β₂-adrenoreceptor. In view of their long acting β₂ agonist activity, the agents of the invention are useful in the relaxation of bronchial smooth muscle and the relief of bronchoconstriction. Relief of bronchoconstriction can be measured in models such as the in vivo plethysmography models of Chong et al, J. Pharmacol. Toxicol. Methods 1998, 39, 163-168, Hammelmann et al, Am. J. Respir. Crit. Care Med., 1997, 156, 766-775 and analogous models. The agents of the invention are therefore useful in the treatment of obstructive or inflammatory airways diseases.

In view of their long duration of action, it is possible to administer the agents of the invention once-a-day in the treatment of such diseases. In another aspect, agents of the invention commonly exhibit characteristics indicating a low incidence of side effects commonly encountered with β₂ agonists such as tachycardia, tremor and restlessness, such agents accordingly being suitable for use in on demand (rescue) treatment as well as prophylactic treatment of obstructive or inflammatory airways diseases. The incidence of side effects may be determined, for example, as described by J. R. Fozard et al., Pulmonary Pharmacology & Therapeutics (2000) 14,289-295.

Treatment of a disease in accordance with the invention may be symptomatic or prophylactic treatment. Inflammatory or obstructive airways diseases to which the present invention is applicable include asthma of whatever type or genesis including both intrinsic (non-allergic) asthma and extrinsic (allergic) asthma. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Other inflammatory or obstructive airways diseases and conditions to which the present invention is applicable include acute lung injury (ALI), adult/acute respiratory distress syndrome (ARDS), chronic obstructive pulmonary, airways or lung disease (COPD, COAD or COLD), including chronic bronchitis, or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to their β₂ agonist activity, the agents of the invention are also useful in the treatment of a condition requiring relaxation of smooth muscle of the uterus or vascular system. They are thus useful for the prevention or alleviation of premature labour pains in pregnancy. They are also useful in the treatment of chronic and acute urticaria, psoriasis, rhinitis, allergic conjunctivitis, actinitis, hay fever, and mastocytosis.
The agents of the invention are also useful as co-therapeutic agents for use in combination with other drug substances such as anti-inflammatory, bronchodilatory or antihistamine drug substances, particularly in the treatment of obstructive or inflammatory airways diseases such as those mentioned hereinbefore, for example as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance. Such anti-inflammatory drugs include steroids, in particular glucocorticosteroids such as budesonide, beclamethasone, fluticasone, ciclesonide or mometasone or steroids described in WO 0200679 especially those of Examples 3, 11, 14, 17, 19, 26, 34, 37, 39, 51, 60, 67, 72, 73, 90, 99 and 101, LTB4 antagonists such as those described in US 5451700, LTD4 antagonists such as montelukast and zafirlukast, and PDE4 inhibitors such as Ariflo®(GlaxoSmith Kline), Roflumilast (Byk Gulden),V-11294A (Napp), BAY19-8004 (Bayer), SCH-351591 (Schering-Plough), Arofylline (Almirall Prodesfarma), PD189659 (Parke-Davis), AWD-12-281 (Asta Medica), CDC-801 (Celgene) and KW-4490 (Kyowa Hakko Kogyo) and A2a agonists such as those described in EP 1052264, EP 1241176, WO 0023457, WO0077018, WO 0123399, WO 0160835, WO 0194368, WO 0200676, WO 0222630, WO 0296462, WO 0127130, WO 0127131, WO 9602543, WO 9602553, WO 9828319, WO 9924449, WO 9924450, WO 9924451, WO 9938877, WO 9941267, WO 9967263, WO 9967264, WO 9967265, WO 9967266, WO 9417090, EP 409595A2 and WO 0078774 and A2b antagonists such as those described in WO 02/42298. Such bronchodilatory drugs include anticholinergic or antimuscarinic agents, in particular ipratropium bromide, oxitropium bromide and tiotropium bromide, but also those described in EP 424021, US 5171744 (Pfizer) and WO 01/04118 (Almirall Prodesfarma).

The agents of the invention are also useful as co-therapeutic agents for use in combination other beta-2 adrenoceptor agonists, for example as a rescue medication. Suitable beta-2 adrenoceptor agonists include salbutamol, terbutaline, salmeterol and, especially, formoterol and pharmaceutically acceptable salts thereof, and compounds (in free or salt or solvate form) of formula I of PCT International patent publication No. WO 00/75114, which document is incorporated herein by reference, preferably compounds of the Examples thereof, especially a compound of formula 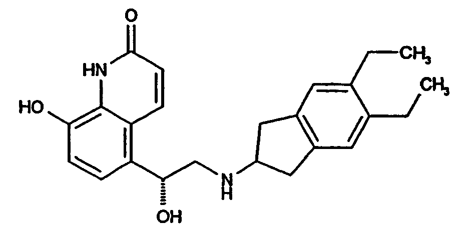 and pharmaceutically acceptable salts thereof.

Co-therapeutic antihistamine drug substances include cetirizine hydrochloride, acetaminophen, clemastine fumarate, promethazine, loratidine, desloratidine, diphenhydramine and fexofenadine hydrochloride.

Combinations of agents of the invention and steroids, beta-2 agonists, PDE4 inhibitors or LTD4 antagonists may be used, for example, in the treatment of COPD or, particularly, asthma. Combinations of agents of the invention and anticholinergic or antimuscarinic agents, PDE4 inhibitors, dopamine receptor agonists or LTB4 antagonists may be used, for example, in the treatment of asthma or, particularly, COPD.

In accordance with the foregoing, the present invention also provides a method for the treatment of an obstructive or inflammatory airways disease which comprises administering to a subject, particularly a human subject, in need thereof an effective amount a compound of formula I, or a pharmaceutically acceptable salt thereof, as hereinbefore described. In another aspect, the invention provides a compound of formula I, or a pharmaceutically acceptable salt thereof, as hereinbefore described for use in the preparation of a medicament for the treatment of an obstructive or inflammatory airways disease.

The agents of the invention may be administered by any appropriate route, e.g. orally, for example in the form of a tablet or capsule; parenterally, for example intravenously; topically to the skin, for example in the treatment of psoriasis; intranasally, for example in the treatment of hay fever; or, preferably, by inhalation, particularly in the treatment of obstructive or inflammatory airways diseases.

In a further aspect, the invention also provides a pharmaceutical composition comprising as active ingredient a compound of formula I in free form or in the form of a pharmaceutically acceptable salt or solvate thereof, optionally together with a pharmaceutically acceptable diluent or carrier therefor. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula I having a particle diameter up to 10 microns, optionally together with a diluent or carrier, such as lactose, of the desired particle size distribution and a compound that helps to protect against product performance deterioration due to moisture. When the composition comprises a nebulised formulation, it preferably contains, for example, the compound of formula I either dissolved, or suspended, in a vehicle containing water, a co-solvent such as ethanol or propylene glycol and a stabiliser, which may be a surfactant.

The invention also includes (A) a compound of formula I as hereinbefore described in free form, or a pharmaceutically acceptable salt or solvate thereof, in inhalable form; (B) an inhalable medicament comprising such a compound in inhalable form together with a pharmaceutically acceptable carrier in inhalable form; (C) a pharmaceutical product comprising such a compound in inhalable form in association with an inhalation device; and (D) an inhalation device containing such a compound in inhalable form.

Dosages employed in practising the invention will of course vary depending, for example, on the particular condition to be treated, the effect desired and the mode of administration. In general, suitable daily dosages for administration by inhalation are of the order of from 0.1 to 5000 µg.

The invention is illustrated by the following Examples.

### EXAMPLES

Certain compounds that are used to prepare compounds of the Examples that are not readily commercially available are prepared as follows:

### Preparation 1: [4-(4-phenyl-butoxy)-phenyl]-acetonitrile

1-Chloro-4-phenylbutane is added to a suspension of 4-hydroxyphenylacetonitrile (1.91 g), K₂CO₃ (4.64 g) and sodium iodide (600 mg) in acetonitrile (30 ml) and refluxed for 68 hours. Filtration, evaporation followed by silica gel flash column chromatography, eluent 4:1 hexane: CH₃CO₂CH₂CH₃, gives the title compd. ¹H nmr (d₆-DMSO, 400 MHz); 7.30-7.13 (m, 7H), 6.95-6.87 (m, 2H), 3.97 (t, J=6 Hz, 2H), 3.92 (s, 2H), 2.62 (t, J=7 Hz, 2H), 1.77-1.63 (m, 4H).

### Preparation 2: 2-[4-(4-phenyl-butoxy)-phenyl]-ethylamine

The title compound (395 mg) is prepared from [4-(4-phenyl-butoxy)-phenyl]-acetonitrile (500 mg) by the procedure of B. Staskun et al J. Chem Soc. (C) 1966, 531. ¹H nmr (d₆-DMSO, 400 MHz); 7.32-7.10 (m, 5H), 7.10-7.00 (m, 2H), 6.83-6.75 (m, 2H), 3.97-3.80 (m, 2H), 3.70-2.87 (br s, 2H), 2.73-2.45 (m, 6H), 1.77-1.60 (m, 4H).

### Preparation 3: benzyl-{2-[4-(4-phenyl-butoxy)-phenyl]-ethyl}-amine

The title compound (2.1 g) is prepared from 2-[4-(4-phenyl-butoxy)-phenyl]-ethylamine by the procedure of A. F. Abdel-Magid J. Org. Chem. 1996, 61, 3849. MS (ES+) 361.

### Prep. 4: (benzyl-{2-[4-(4-phenyl-butoxy)-phenyl]-ethyl}-amino)-acetie acid tert-butyrl ester

Butyl bromoacetate (4.94 ml) is added to a solution of benzyl-{2-[4-(4-phenyl-butoxy)-phenyl]-ethyl}-amine (10 g) and N,N-diisopropylethylamine (10.2 ml) in tetrahydrofuran (THF) (40 ml) at 0° C. After 18 hours at room temperature the reaction mixture is partitioned between aqueous NaHCO₃ and CH₃CO₂CH₂CH₃, followed by evaporation of the CH₃CO₂CH₂CH₃ layers and silica gel column chromatography, eluent 9:1 hexane:CH₃CO₂CH₂CH₃, to give the title compound. MS (ES+) 474.

### Preparation 5: tert-butoxy-5-fluoro-phenylamine

A suspension of platinum oxide (17 g) in a solution of 1-tert-butoxy-4-fluoro-2-nitro-benzene (225 g, prepared by procedure T. F. Woiwode et al J. Org. Chem. 1998, 63, 9594.) in CH₃OH (1.5 l) is stirred under an atmosphere of hydrogen for 18 hours. Filtration through Celite and evaporation gives the title compound. ¹⁹F nmr (CDCl₃, 376 MHz); -43.4.

### Preparation 6: (benzyl-{2-[4-(4-phenyl-butoxy)-phenyl]-ethyl}-amino)-acetic acid

A solution of (benzyl-{2-[4-(4-phenyl-butoxy)-phenyl]-ethyl}-amino)-acetic acid tert-butyl ester (12.1 g) in CH₂Cl₂ (50 ml) and CF₃CO₂H (30 ml) is stirred for 18 hours at room temperature after which evaporation gives the title compound. MS (ES+) 418.

### Preparation 7: 1-tert-butoxy-4-fluoro-2-isothiocyanato-benzene

Carbon disulphide (38.6 ml) is added to a solution of 2-tert-butoxy-5-fluoro-phenylamine (58.8 g) and triethylamine (89.5 ml) in toluene (66 ml) and the reaction mixture stirred at room temperature for 18 hours, then evaporated. Chloroform (200 ml) and triethylamine (44.9 ml) are added to the residue, which is cooled before the addition of ethyl chloroformate (30.8 ml). After 15 minutes at 0°C, the reaction mixture is washed sequentially with aqueous 3N HCl, saturated brine, saturated NaHCO₃ and saturated brine, then evaporated to give the title compound. ¹H nmr (CDCl₃, 400 MHz); 7.10-7.03 (m, 1H), 6.93-6.87 (m, 1H), 6.86-6.80 (m, 1H), 1.43 (s, 9H).

### Preparation 8: 2-(benzyl-{2-[4-(4-phenyl-butoxy)-phenyl]-ethyl}-amino)-N-methoxy-N-methylacetamide

1-(3-dimethylaminopropyl)-3-ethylcarbodiimide hydrochloride (5.30 g) is added to a solution of (benzyl-{2-[4-(4-phenyl-butoxy)-phenyl]-ethyl}-amino)-acetic acid (12.56 g), N,N-dimethylaminopyridine (3.38 g), N,O-dimethylhydroxylamine (8.09 g) and N-methylmorpholine (6.08 ml) in tetrahydrofuran (150 ml). After refluxing for 4 hours, the reaction mixture is partitioned between water and CH₃CO₂CH₂CH₃. Evaporation of the CH₃CO₂CH₂CH₃ layers and silica gel column chromatography, eluent 9:1 hexane: CH₃CO₂CH₂CH₃, gives the title compound. MS (ES+) 461.

### Preparation 9: (2-tert-butoxy-5-fluoro-phenyl)-thiocarbamic acid O-isopropyl ester

A solution of 1-tert-butoxy-4-fluoro-2-isothiocyanato-benzene (50.0 g) and triethylamine (31 ml) in isopropanol (170 ml) is refluxed for 48 hours. Evaporation of the reaction mixture followed by silica gel flash column chromatography, eluent 20:1 hexane: CH₃CO₂CH₂CH₃ gives the title compound. ¹H nmr (CDCl₃, 400 MHz); 8.60 (br s, 1H), 7.38 (br s, 1H), 7.50-6.87 (m, 1H), 6.67-6.58 (m, 1H), 5.64-5.50 (m, 1H), 1.43-1.32 (m, 6H), 1.32-1.25 (s, 9H).

### Preparation 10: 2-(benzyl-{2-[4-(4-phenyl-butoxy)-phenyl]-ethyl}-amino)-1-(4-tert-butoxy-2-isopropoxy-benzothiazol-7-yl)-ethanone

A solution of tert. butyl lithium in pentane (12.3 ml, 1.7 M) is added to a solution of (2-tert-butoxy-5-fluoro-phenyl)-thiocarbamic acid O-isopropyl ester (3.20 g) in tetrahydrofuran (10 ml) at -78° C, the solution warmed to -20° C over 1 hour then re-cooled -78° C and a solution of 2-(benzyl-{2-[4-(4-phenyl-butoxy)-phenyl]-ethyl}-amino)-N-methoxy-N-methylacetamide in tetrahydrofuran (10 ml) added at -78° C. The reaction mixture is warmed to room temperature and partitioned between aqueous NH₄Cl and CH₃CO₂CH₂CH₃. Evaporation of the CH₃CO₂CH₂CH₃ layers and silica gel column chromatography, eluent 4:1 hexane: CH₃CO₂CH₂CH₃, gives the title compound. MS (ES+) 665.

### Preparation 11: 7-[(benzyl-{2-[4-(4-phenyl-butoxy)-phenyl]-ethyl}-amino)-acetyl]-4-hydroxy-3H-benzothiazol-2-one

A solution of 2-(benzyl-{2-[4-(4-phenyl-butoxy)-phenyl]-ethyl}-amino)-1-(4-tert-butoxy-2-isopropoxy-benzothiazol-7-yl)-ethanone (2.49 g) in isopropanol (20 ml) and concentrated hydrobromic acid (20 ml) is heated at 50° C. After 3 hours the reaction mixture is partitioned between CH₃CO₂CH₂CH₃ and water, and the CH₃CO₂CH₂CH₃ layer washed with aqueous NaHCO₃ then brine. Evaporation of the CH₃CO₂CH₂CH₃ layers and silica gel column chromatography, eluent 4:1 hexane: CH₃CO₂CH₂CH₃, gives the title compd. MS (ES+) 567.

### Preparation 12: 7-[2-(benzyl-{2-[4-(4-phenyl-butoxy)-phenyl]-ethyl}-amino)-1-hydroxy-ethyl]-4-hydroxy-3H-benzothiazol-2-one

NaBH₄ (2.67 g) is added portion-wise to a solution of 7-[(benzyl-{2-[4-(4-phenyl-butoxy)-phenyl]-ethyl}-amino)-acetyl]-4-hydroxy-3H-benzothiazol-2-one (0.40 g) in CH₃OH (15 ml) at 0° C. After 30 minutes the reaction mixture is partitioned between CH₃CO₂CH₂CH₃ and water. Evaporation of the CH₃CO₂CH₂CH₃ layers and silica gel column chromatography, eluent 1:1 hexane: CH₃CO₂CH₂CH₃, gives the title compound. MS (ES+) 569.

### Preparation 13: benzyl-indan-2-ylamine

The title compound is prepared from indan-2-one by the procedure of A. F. Abdel-Magid et al J. Org. Chem. 1996, 61, 3849. MS (ES+) 224.

### Preparation 14: 2-(benzyl-indan-2-yl-amino)-N-methoxy-N-methyl-acetamide

The title compound is prepared from benzyl-indan-2-ylamine by procedures analogous to those of Preparations 4, 6 and 8. MS (ES+) 326.

### Prep. 15: 7-[2-(benzyl-{2-indan-2-yl}amino)-1-hydroxyethyl]-4-hdroxy-3H-enzothiazolone

The title compound is prepared from 2-(benzyl-indan-2-yl-amino)-N-methoxy-N-methylacetamide and (2-tert-butoxy-5-fluoro-phenyl)-thiocarbamic acid O-isopropyl ester using procedures analogous to those of Preparations 10, 11 and 12.

### Preparation 16: 2-[benzyl-(5,6-diethyl-indan-2-yl)-amino]-N-methoxy-N-methyl-acetamide

The title compound is prepared from 5,6-diethyl-indan-2-ylamine (prepared by the procedure of WO 0075114) by procedures analogous to those of Prep. 3,4, 6 and 8. MS (ES+) 382.

### Preparation 17: 2-{benzyl-[(R)-2-(4-methoxy-phenyl)-1-methyl-ethyl]-amino}-N-methoxy-N-methyl-acetamide

The title compound Is prepared from (R)-2-(4-methoxy-phenyl)-1-methyl-ethylamine (R. Hett et al Tetrahedron Lett. 1997, 38, 1125.) by procedures analogous to those of Preparations 3, 4, 6 and 8. MS (ES+) 358.

### Preparation 18: 2,2,2-trifluoro-N-phenethyl-acetamide

Trifluoroacetic anhydride (64.5 ml) is added dropwise to a solution of phenethylamine (52 ml) and triethylamine (58 ml) in CH₂Cl₂ at 0° C. After 18 hours at room temperature the reaction mixture is washed with aqueous citric acid, brine and aqueous NaHCO₃, dried with MgSO₄ and evaporated to give the title compound.

### Preparation 19: 2,2,2-trifluoro-N-[2-(4-isobutyryl-phenyl)-ethyl]-acetamide

Isobutryl chloride (19.7 ml) is added dropwise to a mixture of 2,2,2-trifluoro-N-phenethyl-acetamide (34.2 g) and aluminium chloride (48.2 g) in CH₂Cl₂ (450 ml) at 0° C. After 18 hours at room temperature the reaction mixture is poured onto ice (2000 g), extracted 3 times with CH₂Cl₂, dried with MgSO₄ and evaporated to give the title compound which is used without further purification.

### Preparation 20: 2,2,2-trifluoro-N-[2-(4-isobutyl-phenyl)-ethyl]-acetamide

A solution of 2,2,2-trifluoro-N-[2-(4-isobutyryl-phenyl)-ethyl]-acetamide (19.4 g) in ethanol (200 ml) and conc. hydrochloric acid (5 ml) is stirred for 23 hours under 1 atm of hydrogen over a palladium on carbon catalyst (1.9 g). Filtration, evaporation and purification by silica gel chromatography, eluting with chloroform, gives the title compound. ¹³C nmr (CDCl₃, 101 MHz); 157.55, 140.90, 135.10, 130.03, 128.78, 45.40, 41.46, 34.94, 30.61, 22.72.

### Preparation 21: 2-(4-isobutyl-phenyl)-ethylamine

Potassium carbonate (18.5 g) is added to a solution of 2,2,2-trifluoro-N-[2-(4-isobutylphenyl)-ethyl]-acetamide (12.2 g) in methanol (45 ml) and water (19 ml) at room temp. The mixture is heated at 45° C for 8 hours. Dilution with water (200 ml), extraction with dichloromethane, drying over MgSO₄ and evaporation gives the title compound. ¹³C nmr (CDCl₃, 101 MHz); 140.10, 137.40, 129.56, 128.94, 45.45, 43.99, 40.01, 30.45, 22.79.

### Preparation 22: {benzyl-[2-(4-isobutyl-phenyl)-ethyl]-amino}-acetic acid

The title compound is prepared from 2-(4-isobutyl-phenyl)-ethylamine by procedures analogous to those of Preparations 3, 4 and 6. MS (ES+) 326.

### Prep. 23: 2-{benzyl-[2-(4-isobutyl-phenyl)-ethyl]-amino}-N-methoxy-N-methyl-acetamide

Isobutyl chloroformate (0.54 ml) is added to a solution of {benzyl-[2-(4-isobutyl-phenyl)-ethyl]-amino}-acetic acid (1.5 g) and Hunig's base (3.61 ml) in dichloromethane (21 ml) at 0°C. After 2 hours N,O-dimethylhydroxylamine hydrochloride (0.49 g) is added, the reaction stirred a further 30 minutes at 0°C then partitioned between aqueous NaHCO₃ and CH₂Cl₂, dried over MgSO₄, evaporated and purified by silica gel chromatography, eluting with 10% ethyl acetate in CH₂Cl₂, to give the title compound. MS (ES+) 370.

### Prep. 24: 2-{benzyl-[2-(4-propyl-phenyl)-ethyl]-amino}-N-methoxy-N-methyl-acetamide

The title compound is prepared from 2,2,2-trifluoro-N-phenethyl-acetamide by procedures analogous to those of Preparations 19, 20, 21, 3, 4, 6 and 22. MS (ES+) 356 (96%), 266.

### Preparation 25: N-benzyl-2-(4-bromo-phenyl)-acetamide

4-Bromophenylacetic acid (23.24 g) is dissolved in dichloromethane (400 ml). 1-(3-Dimethylaminopropyl)-3-ethylcarbodiimide hydrochloride (20.70 g) is added followed by DMAP (0.13 g) and the reaction mixture stirred at room temperature for 10 minutes. Benzylamine (12.14 g), dissolved in dichloromethane (100 ml), is then slowly added and the reaction mixture stirred at room temperature. After 1 hour the reaction is shown to be complete by TLC. The reaction mixture is washed with 1M HCl (3 x 200 ml), water (3 x 200 ml) and brine (200 ml). The organic layer dried is over MgSO₄, filtered and the solvent removed in vacuo. The title compound is obtained following crystallisation from ethylacetate. MS (ES⁺) m/e 304 (MH⁺ - Br⁷⁹) and 306 (MH⁺ - Br⁸¹).

### Preparation 26: N-benzyl-2-(4-propyl-phenyl)-acetamide

1,1'-Bis(diphenylphosphino)ferrocenedichloro palladium(II) (0.39 g) is placed in a flask under an atmosphere of argon. The flask is cooled to -78°C and then propylzinc bromide (200 ml, 0.5 M in THF) is slowly added. N-Benzyl-2-(4-bromo-phenyl)-acetamide (14.48 g, 47.62 mmol), dissolved in THF (500 ml), is then slowly added and the reaction mixture stirred at room temperature. After 24 hours further propylzinc bromide (10 ml, 0.5 M in THF) is added and the reaction mixture stirred at room temperature. The reaction is shown to be complete by TLC after 24 hours and is quenched by the addition of 2M HCl (50 ml) and then 80-90% of the solvent is removed in vacuo. The residue is partitioned between ethyl acetate (250 ml) and water (250 ml). The organic layer is washed with water (250 ml) and brine (250 ml), dried over MgSO₄, filtered and the solvent removed in vacuo. Recrystallisation from cyclohexane gives the title compound. MS (ES+) m/e 268 (MH⁺).

### Preparation 27: benzyl-[2-(4-propyl-phenyl)-ethyl]-amine hydrochloride

DIBAL (47.5 ml, 1.5M in toluene) is slowly added to N-benzyl-2-(4-propyl-phenyl)-acetamide (9.50 g) in toluene (200 ml) cooled on an ice-bath. The reaction mixture is stirred at room temperature until shown to be complete by TLC. The reaction mixture is recooled on an ice-bath and quenched by the addition of water (10 ml), washed further with water (2 x 100 ml) and brine (100 ml), dried over MgSO₄, filtered and the solvent removed in vacuo. The residue is taken up in hexane : ethylacetate (5 :1) (100 ml) and any insoluble material is filtered off. The solvent is removed in vacuo and the residue dissolved in Et₂O. 1M HCl in Et₂O (30ml) is added and the title compound obtained by filtration. MS (ES+) m/e 254 (MH⁺).

### Preparation 28: 1-(4-tert.butoxy-2-isopropoxy-benzothiazol-7-yl)-2-chloro-ethanone

Tert. butyllithium (22.7 ml, 1.7 M in pentane) is added dropwise to a solution of (2-tert. butoxy-5-fluoro-phenyl)-thiocarbamic acid O-isopropyl ester (5.00 g) in THF (20 ml) at -78° C. This solution is then allowed to warm to -20° C and a dried mixture of lithium chloride (2.12 g) and copper (I) cyanide (2.24 g) in THF (50 ml) is added. After 15 minutes chloroacetyl chloride (4.36 g) is added and the reaction mixture allowed to warm to 0°C. This temperature is maintained for 1 hour and then the reaction mixture is quenched by the addition of saturated aqueous NH₄Cl (5 ml). The reaction mixture is partitioned between ethyl acetate (250 ml) and water (250 ml). The organic layer is washed with water (250 ml) and brine (250 ml), dried over MgSO₄, filtered and the solvent removed in vacuo. The tide compd is obtained by flash column chromatography (silica, iso-hexane / ethyl acetate 10:1). MS (ES+) m/e 341 (MH⁺).

### Preparation 29: (R)-1-(4-tert-butoxy-2-isopropoxy-benzothiazol-7-yl)-2-chloro-ethanol

Borane-THF complex, (14.64 ml, 1M in THF) is added dropwise to a solution of (1R, 2S)-(+)-1-amino-2-indanol (0.22 g) in THF (50 ml) and the solution is stirred at room temperature for 15 minutes. A solution of 1-(4-tert.butoxy-2-isopropoxy-benzothiazol-7-yl)-2-chloro-ethanone (5.00 g) in THF (50 ml) is then added dropwise over a period of 1 hour. The reaction mixture is stirred at room temperature for a further 15 minutes and then quenched by the addition of 0.2M H₂SO₄ (5 ml). The reaction mixture is partitioned between ethyl acetate (200 ml) and 0.2M H₂SO₄ (200 ml). The organic layer is washed with water (200 ml) and brine (200 ml), dried over MgSO₄, filtered and the solvent removed in vacuo to give the title compound. MS (ES+) m/e 344 (MH⁺).

### Preparation 30: 4-tert.butoxy-2-isopropoxy-7-(R)-oxiranyl-benzothiazole

A mixture of (R)-1-(4-tert.butoxy-2-isopropoxy-benzothiazol-7-yl)-2-chloro-ethanol (4.70 g) and potassium carbonate (7.48 g) in acetone (250 ml) is refluxed for 48 hours. The reaction mixture is allowed to cool, filtered and the solvent removed in vacuo to give the title compound. MS (ES+) m/e 308 (MH⁺).

### Preparation 31: (R)-2-{benzyl-[2-(4-propyl-phenyl)-ethyl]-amino}-1-(4-tert-butoxy-2-isopropoxy-benzothiazol-7-yl)-ethanol

A solution of 4-tert.butoxy-2-isopropoxy-7-(R)-oxiranyl-benzothiazole (3.50 g) and benzyl-[2-(4-propyl-phenyl)-ethyl]-amine (3.03 g) in 1-butanol (25 ml) is stirred at 110° C. The reaction is shown to be complete by TLC after 18 hours. The title compound is obtained after purification by flash column chromatography (silica, iso-hexane / ethyl acetate 10:1). MS (ES+) m/e 561 (MH⁺).

### Preparation 32: (S)-1-(4-tert-butoxy-2-isopropoxy-benzothiazol-7-yl)-2-chloro-ethanol

The title compound is prepared by a procedure analogous to that of Prep 29 using Borane-THF complex, (14.64 ml, 1 M in THF), (1S, 2R)-(-)-1-amino-2-indanol (0.22 g) and 1-(4-tertbut-oxy-2-isopropoxy-benzothiazol-7-yl)-2-chloro-ethanone (5.00 g). MS (ES+) m/e 344 (MH⁺).

### Preparation 33: (5,6,7,8-Tetrahydro-naphthalen-2-yl)-acetic acid

The title compound is prepared from 6-acetyltetralin by the procedure of G. Giardina et al J. Med. Chem. 1994, 37, 3482. ¹H nmr (CDCl₃, 400 MHz); 7.00 (m, 3H), 3.55 (s, 2H), 2.75 (m, 4H), 1.75 (m, 4H).

### Preparation 34: N-Benzyl-2-(5,6,7,8-tetrahydro-naphthalen-2-yl)-acetamide

The title compound is prepared by a procedure analogous to that of Preparation 25 using (5,6,7,8-Tetrahydro-naphthalen-2-yl)-acetic acid. ¹H nmr (CDCl₃, 400 MHz); 7.30-7.15 (m, 5H), 7.05-6.90 (m, 3H), 5.70 (br s, 1H), 3.55 (s, 2H), 2.70 (m, 4H), 1.75 (m, 4H).

### Preparation 35: Benzyl-[2-(5,6,7,8-tetrahydro-naphthalen-2-yl)-ethyl]-amine hydrochloride

The title compound is prepared by a procedure analogous to that of Preparation 27 using N-Benzyl-2-(5,6,7,8-tetrahydro-naphthalen-2-yl)-acetamide. ¹H nmr (d₆-DMSO, 400 MHz); 9.40 (br s, 2H), 7.55 (m, 2H), 7.40 (m, 3H), 7.00-6.85 (m, 3H), 4.10 (m, 2H), 3.05 (m, 2H), 2.90 (m, 2H), 2.65 (m, 4H), 1.70 (m, 4H).

### Preparation 36: (R)-2-{Benzyl-[2-(5,6,7,8-tetrahydro-naphthalen-2-yl)-ethyl]-amino}-1-(4-tert-butoxy-2-isopropoxy-benzothiazol-7-yl)-ethanol

The title compound is prepared by a procedure analogous to that of Preparation 31 using Benzyl-[2-(5,6,7,8-tetrahydro-naphthalen-2-yl)-ethyl]-amine. ¹H nmr (CDCl₃, 400 MHz); 7.35-7.20 (m, 5H), 7.00-6.95 (m, 3H), 6.90-6.80 (m, 2H), 5.45 (m, 1H), 4.70 (m, 1H), 3.93 (d, 1H), 3.55 (d, 1H), 2.85 (m, 6H), 2.70 (m, 4H), 1.75 (m, 4H), 1.45 (m, 6H), 1.35 (s, 9H).

### Preparation 37: 1-(3,4-Diethyl-phenyl)-ethanone

1,2-Diethylbenzene (9.24 g, 69 mmol) and acetyl chloride (5.42 g, 69 mmol) are added dropwise to AlC1₃ (20.63 g, 155 mmol) in nitromethane (50 ml) over 30 minutes. The reaction mixture is stirred at room temperature for 2 hours, after which 200 g of ice and 15 ml concentrated hydrochloric acid are added. The aqueous phase is extracted with ether, and the combined organic phases extracted with 2 N HCI and saturated aqueous NaCl. The organic phase is dried over magnesium sulphate, filtered, and the solvent removed *in vacuo* to give the title compound. ¹H nmr (CDCl₃, 400 MHz); 7.75 (d, 1H), 7.70 (d of d, 1H), 7.15 (d, 1H), 2.70 (m, 4H), 2.55 (s, 3H), 1.20 (m, 6H).

### Preparation 38: Benzyl-[2-(3,4-diethyl-phenyl)-ethyl]-amine hydrochloride

The title compound is prepared from 1-(3,4-Diethyl-phenyl)-ethanone using procedures analogous to those of Preparations 33, 25 and 27. ¹H nmr (d₆-DMSO, 400 MHz); 9.40 (br s, 2H), 7.55 (m, 2H), 7.40 (m, 3H), 7.10 (m, 1H), 7.00- (m, 2H), 4.15 (m, 2H), 3.05 (m, 2H), 2.90 (m, 2H), 2.55 (m, 4H), 1.10 (m, 6H).

### Preparation 39: Benzyl-[2-(4-ethoxy-3-methoxy-phenyl)-ethyl]-amine hydrochloride

The title compound is prepared from (4-Ethoxy-3-methoxy-phenyl)-acetic acid using procedures analogous to those of Preparations 25 and 27. ¹H nmr (d₆-DMSO, 400 MHz); 9.40 (br s, 2H), 7.55 (m, 2H), 7.40 (m, 3H), 6.85 (m, 2H), 6.70 (m, 1H), 4.15 (m, 2H), 3.95 (q, 2H), 3.75 (s, 3H), 3.10 (m, 2H), 2.90 (m, 2H), 1.30 (t, 4H).

### Preparation 40: (R)-2-{Benzyl-[2-(4-ethoxy-3-methoxy-phenyl)-ethyl]-amino}-1-(4-tert-butoxy-2-isopropoxy-benzothiazol-7-yl)-ethanol

The title compound is prepared by a procedure analogous to that of Preparation 31 using Benzyl-[2-(4-ethoxy-3-methoxy-phenyl)-ethyl]-amine. ¹H nmr (CDCl₃, 400 MHz); 7.35-7.20 (m, 5H), 6.95 (m, 2H), 6.80 (d, 1H), 6.65 (m, 2H), 5.45 (m, 1H), 4.70 (m, 1H), 4.05 (q, 2H), 3.95 (d, 1H), 3.80 (s, 3H), 3.55 (d, 1H), 2.90-2.70 (m, 6H), 1.45 (m, 9H), 1.35 (s, 9H).

### Preparation_41: 1,2-Dipropylbenzene

1,1'-Bis(diphenylphosphino)ferrocenedichloro palladium(II) (0.35 g) is placed in a flask under an atmosphere of argon. The flask is cooled to 0°C and then propylzinc bromide (169.5 ml, 0.5 M in THF) is slowly added. 1,2-Dibromobenzene (5.00 g, 21.19 mmol), dissolved in THF (10 ml), is then slowly added and the reaction mixture stirred at 50°C. The reaction is shown to be complete by TLC after 24 hours and is quenched by the addition of 2M HCl (50 ml) and then 80-90% of the solvent is removed *in vacuo*. The residue is partitioned between ethyl acetate (250 ml) and water (250 ml). The organic layer is washed with water (250 ml) and brine (250 ml), dried over MgSO₄, filtered and the solvent removed *in vacuo* to give the title compound. ¹H nmr (CDCl₃, 400 MHz); 7.10 (m, 4H), 2.60 (m, 4H), 1.60 (m, 4H), 1.00 (m, 6H).

### Preparation 42: Benzyl-[2-(3,4-dipropyl-phenyl)-ethyl]-amine hydrochloride

The title compound is prepared from 1,2-Dipropylbenzene using procedures analogous to those of Preparations 37, 33, 25 and 27. ¹H nmr (d₆-DMSO, 400 MHz); 9.40 (br s, 2H), 7.55 (m, 2H), 7.40 (m, 3H), 7.10 (d, 1H), 6.95 (m, 2H), 4.15 (m, 2H), 3.10 (m, 2H), 2.90 (m, 2H), 2.50 (m, 4H), 1.50 (m, 4H), 0.90 (m, 6H).

### Preparation 43: (R)-1-(4-tert-Butoxy-2-isopropoxy-benzothiazol-7-yl)-2-(1,1dimethyl-2-phenyl-ethylamino)-ethanol

A solution of phentermine (0.728 g, 4.89 mmol) and N,O-bis(trimethylsilyl)acetamide (0.496 g, 2.44 mmol) in dry DMF (1 ml) is stirred at room temperature for 30 minutes. A solution of 4-tert.butoxy-2-isopropoxy-7-(R)-oxiranyl-benzothiazole (0.75 g, 2.44 mmol) in dry DMF (1 ml) is added and the reaction mixture is stirred at 80° C. The reaction is shown to be complete by TLC after 18 hours. The title compound is obtained after purification by flash column chromatography (silica, iso-hexane / ethyl acetate 1:1). MS (ES+) *m*/*e* 457 (MH⁺).

### Preparation 44: Benzyl-[2-(3-propyl-phenyl)-ethyl]-amine hydrochloride

The title compound is prepared from 3-Bromophenylacetic acid using procedures analogous to those of Preparations 25, 26 and 27. MS (ES+) *m*/*e* 254 (MH⁺).

### Preparation 45: Benzyl-[2-(3-butyl-phenyl)-ethyl]-amine hydrochloride

The title compound is prepared from 3-Bromophenylacetic acid using procedures analogous to those of Preparations 25, 26 and 27. MS (ES+) *m*/*e* 268 (MH⁺).

### Preparation 46: N-Benzyl-2-(4-hydroxy-phenyl)-acetamide

The title compound is prepared from 4-hydroxyphenylacetic acid using procedures analogous to that of Preparation 25 and purification by flash column chromatography (silica, iso-hexane / ethyl acetate 2:1). MS (ES+) *m*/*e* 242 (MH⁺).

### Preparation 47: N-Benzyl-2-(4-butoxy-phenyl)-acetamide

N-Benzyl-2-(4-hydroxy-phenyl)-acetamide(2.00 g, 8.25 mmol) is suspended in acetonitrile (30 ml). Caesium carbonate (5.40 g, 16.58 mmol), butyl bromide (1.07 ml, 9.95 mmol) and finally potassium iodide (0.41 g, 2.49 mmol) were added and the reaction mixture was refluxed. The reaction was shown to be complete by HPLC after 16 hours. The reaction mixture is partitioned between ethyl acetate (100 ml) and water (100 ml). The organic layer is washed with water (150 ml) and brine (150 ml), dried over MgSO₄, filtered and the solvent removed *in vacuo*. Recrystallisation from cyclohexane/ ethyl acetate gives the title compound. MS (ES+) *m*/*e* 298 (MH⁺).

### Preparation 48: Benzyl-[2-(4-butoxy-phenyl)-ethyl]-amine

Borane-THF complex (10 ml, 1M in THF) is added to N-Benzyl-2-(4-butoxy-phenyl)-acetamide 1.00 g, 3,36 mmol). The resulting slurry is stirred at room temperature. The reaction was shown to be complete by HPLC after 3 hours. Concentrated hydrochloric acid (5 ml) is slowly added to the ice-cooled reaction mixture and is then refluxed for 1.5 hours. The reaction mixture is cooled and treated with 4M NaOH until a basic solution is obtained. The reaction mixture is extracted with ethyl acetate and the organic layer is washed with water (150 ml) and brine (150 ml), dried over MgSO₄, filtered and the solvent removed *in vacuo*. The tide compound is obtained after purification by flash column chromatography (silica, iso-hexane / ethyl acetate 2:1). MS (ES+) *m*/*e* 284 (MH⁺).

### Preparation 49: Benzyl-[2-(3-butoxy-phenyl)-ethyl]-amine

The title compound is prepared from 3-hydroxyphenylacetic acid using procedures analogous to those of Preparations 46, 47 and 48. MS (ES+) *m*/*e* 284 (MH⁺).

### Preparation 50: Benzyl-[2-(3-pentyrl-phenyl)-ethyl]-amine hydrochloride

The title compound is prepared from 3-Bromophenylacetic acid using procedures analogous to those of Preparations 25, 26 and 27. MS (ES+) *m*/*e* 282 (MH⁺).

### Preparation 51: (3-Bromo-phenyl)-acetic acid methyl ester

3-Bromophenylacetic acid (14.38g, 66.90mmol) is dissolved in methanol (100 ml). Concentrated sulfuric acid (2 ml) is added dropwise and the reaction mixture is stirred at room temperature for 18 hours. 80% of the solvent is removed *in vacuo* and the reaction mixture is partitioned between ethyl acetate (100 ml) and water (100 ml). The organic layer is washed with water (150 ml) and brine (150 ml), dried over MgSO₄, filtered and the solvent removed *in vacuo* to give the title compound. ¹H nmr (CDCl₃, 400 MHz); 7.20 (m, 2H), 6.95 (m, 2H), 3.50 (s, 3H), 3.35 (s, 2H)

### Preparation 52: 2-(3-Bromo-phenyl)-acetamide

(3-Bromo-phenyl)-acetic acid methyl ester (15.33 g, 66.90 mmol) is dissolved in methanol (50 ml) and ammonium hydroxide (100 ml). The reaction mixture is stirred at room temperature. The reaction is shown to be complete by TLC after 18 hours. The methanol is removed *in vacuo* and the product precipitates out. The solid is filtered off, washed with water and dried to give the title compd. MS (ES+) *m*/*e* 214 (MH⁺ - Br⁷⁹), 216 (MH⁺ - Br⁸¹).

### Preparation 53: 2-(3'-Methoxy-biphenyl-3-yl)-acetamide

2-(3-Bromo-phenyl)-acetamide (1.07 g, 5.00 mmol) is dissolved in THF (30 ml) and cooled to 0°C. 2-methoxyphenylboronic acid (0.76 g, 5.00 mmol) is added then sodium carbonate (1.06 g, 10.00 mmol) dissolved in water (24 ml). The reaction mixture is evacuated and charged with argon (3x). Tetrakis(triphenylphosphine)palladium (0.29 g, 0.25 mmol) is added and the reaction mixture is evacuated and charged with argon (3x). The reaction mixture is stirred at 80°C for 18 hours. The reaction mixture is extracted with ethyl acetate and the organic layer is washed with water (150 ml) and brine (150 ml), dried over MgSO₄, filtered and the solvent removed *in vacuo*. The title compound is obtained after purification by flash column chromatography (silica, ethyl acetate). MS (ES+) mle 242 (MH⁺).

### Preparation 54: 2-(3'-Methoxy-biphenyl-3-yl)-ethylamine hydrochloride

The title compound is prepared from 2-(3'-methoxy-biphenyl-3-yl)-acetamide using a procedure analogous to that of Preparation 48 and treatment with 1M HCl/Et₂O. ¹H nmr (CDCl₃, 400 MHz); 8.30 (br s, 3H), 7.40 (m, 2H), 7.30 (m, 1H), 7.20 (m, 1H), 7.10 (m, 2H), 7.05 (m, 1H), 6.75 (m, 1H), 3.75 (s, 3H), 3.05 (m, 2H), 2.95 (m, 2H).

### Preparation 55: Benzyl-[2-(4-bromo-phenyl)-1-methyl-ethyl]-amine

4-bromophenylacetone (18.30 g, 85.91 mmol) and benzylamine (9.19 g, 85.91 mmol) are dissolved in ethanol (100 ml). 5%Pt-C (0.5 g) is added and the reaction mixture is stirred under an atmosphere of hydrogen. The reaction is shown to be complete by TLC after 18 hours. The catalyst is filtered off and the solvent is removed in *vacuo* to give the title compound. MS (ES+) *m*/*e* 304 (MH⁺ - Br⁷⁹) and 306 (MH⁺ - Br⁸¹).

### Preparation 56: Benzyl-[2-(4-bromo-phenyl)-1-methyl-ethyl]-carbamic acid benzyl ester

Benzyl-[2-(4-bromo-phenyl)-1-methyl-ethyl]-amine (17.00 g, 55.92 mmol) is dissolved in dichloromethane (250 ml). Triethylamine (6.21 g, 61.51 mmol) is added, then benzylchloroformate (10.49 g, 61.51 mmol), and the reaction mixture is stirred at room temperature. The reaction is shown to be complete by TLC after 18 hours. The reaction mixture is washed with 2M HCl, water and brine, dried over MgSO₄, filtered and the solvent removed *in vacuo*. The title compound is obtained after purification by flash column chromatography (silica, iso-hexane / ethyl acetate 4:1). MS (ES+) *m*/*e* 438 (MH⁺ - Br⁷⁹) and 440 (MH⁺ - Br⁸¹).

### Preparation 57: Benzyl-[1-methyl-2-(4-propyl-phenyl)-ethyl]-carbamic acid benzyl ester

The title compound is prepared from benzyl-[2-(4-bromo-phenyl)-1-methyl-ethyl]-carbamic acid benzyl ester using a procedure analogous to that of Preparation 26 and purification by flash column chromatography (silica, iso-hexane / ethyl acetate 10:1). ¹H nmr (CDCl₃, 400 MHz); 7.40- 6.85(m, 16H), 5.10 (m, 2H), 4.50 (m, 1H), 4.20 (m, 1H), 2.90 (m, 1H), 2.65 (m, 1H), 2.50 (m, 2H), 1.60 (m, 2H), 1.10 (m, 3H), 0.90 (t, 3H)

Preparation 58: 1-Methyl-2-(4-propyl-phenyl)-ethylamine

Benzyl-[2-(4-propyl-phenyl)-1-methyl-ethyl]-carbamic acid benzyl ester (4.00 g, 9.13 mmol) is dissolved in methanol (100 ml) and the compound is deprotected by adding a catalytic amount of 10% palladium on charcoal and placing the solution under an atmosphere of H₂. The reaction is shown to be complete by TLC after 18 hours. The catalyst is filtered off and the solvent is removed *in vacuo* to give the title compound. MS (ES+) *m*/*e* 178 (MH⁺).

### Preparation 59 (R)-2-(4-Methoxy-phenyl)-1-methyl-ethylamine

The title compound is prepared by the procedure of R. Hett et al Organic Process Research & Development (1998), 2(2), 96-99.

### Preparation 60: (S)-2-(4-Methxy-phenyl)-1-methyl-ethylamine

The title compound is prepared by the procedure of R. Hett et al Organic Process Research & Development (1998), 2(2), 96-99.

### Preparation 61: 1-(3-Butyl-phenyl)-3-chloro-propan-1-one

Butylbenzene (44.78 g, 334 mmol) and propionyl chloride (42.44 g, 334 mmol) are added dropwise to AlC13 (22.3 g, 167.8 mmol) in nitromethane (75 ml) over 1 hour. The reaction mixture is stirred at room temperature for 3 hours, after which 400 g of ice and 60 ml concentrated hydrochloric acid are added. The aqueous phase is extracted with ether, and the combined organic phases extracted with 2N HCI and saturated aqueous NaCl. The organic phase is dried over magnesium sulphate, filtered, and the solvent removed *in vacuo* to give the title compound. ¹H nmr (CDCl₃, 400 MHz); 7.85(d, 2H), 7.30(d, 2H), 3.90 (t, 2H), 3.40 (t, 2H), 2.65 (t, 2H), 1.60 (m, 2H), 1.35 (m, 2H), 0.90 (t, 3H).

### Preparation 62: 6-Butyl-indan-1-one

1-(3-Butyl-phenyl)-3-chloro-propan-1-one (65.0 g, 290 mmol) is dissolved in concentrated sulphuric acid (250 ml) and heated to 90° C for 4 hours. The reaction mixture is cooled, ice (500 g) is added, and the aqueous solution extracted twice with toluene. The organic layer is washed with sodium bicarbonate, saturated aqueous NaCl, dried over magnesium sulphate. After filtration, the solvent is removed *in vacuo* to give the title compound.

### Preparation 63: 6-Butyl-indan-1,2-dione 2-oxime

6-Butyl-indan-1-one (35.0 g, 186 mmol) is dissolved in methanol (250 ml) and brought up to 40° C. N-butyl nitrite (21.1 g, 205 mmol) is added dropwise, followed by the addition of concentrated HCI (5 ml). The reaction is shown to be complete by TLC after 1 hour. The reaction is brought to room temperature and the precipitate is filtered off, washed with ice-cold methanol and dried to give the title compound. ¹H nmr (CDCl₃, 400 MHz); 8.80 (br s, 1H), 7.80(d, 1H), 7.30(s, 1H), 7.25(d, 1H), 3.85 (s, 2H), 2.70 (t, 2H), 1.60 (m, 2H), 1.35 (m, 2H), 0.90 (t, 3H).

### Preparation 64: 5-Butyl-indan-2-ylamine hydrochloride

6-Butyl-indan-1,2-dione 2-oxime (5.00 g, 23.04 mmol) is dissolved in acetic acid (75 ml) and concentrated sulfuric acid (5 ml). 10% Pd-C (1.00 g) is added and the reaction mixture is stirred under an atmosphere of hydrogen at 3 atmospheres. The reaction is shown to be complete by TLC after 18 hours. The catalyst is filtered off and the solution is made basic with 2M NaOH. The reaction mixture is extracted with ethyl acetate and the organic layer is washed with water and brine, dried over MgSO₄, filtered and the solvent removed *in vacuo*. The residue is dissolved in Et₂O (100 ml) and treated with 1M HCl/ Et₂O (25 ml). The precipitate is filtered off and dried to give the title compound. ¹H nmr (CDCl₃, 400 MHz); 8.50 (br s, 3H), 7.05(m, 1H), 6.95(m, 2H), 4.00 (m, 1H), 3.25 (m, 2H), 3.10 (m, 2H), 2.50 (t, 2H), 1.50 (m, 2H), 1.25 (m, 2H), 0.80 (t, 3H).

### Preparation 65: 2-(4-Amino-phenyl)-ethyl]-carbamic acid tert-butyl ester

The title compound is prepared from 4-(2-amino-ethyl)-phenylamine by the procedure of WO 01/42193. ¹H nmr (CDCl₃, 400 MHz); 6.95(d, 2H), 6.65(d, 2H), 4.50 (br s, 1H), 3.60 (s, 2H), 3.30 (m, 2H), 2.70 (m, 2H), 1.40 (s, 9H).

### Preparation 66: [2-(4-Pyrrolidin-1-yl-phenyl)-ethyl]-carbamic acid tert-butyl ester

The title compound is prepared from [2-(4-Amino-phenyl)-ethyl]-carbamic acid tert-butyl ester by a procedure analogous to that of G. Verardo et al Synthesis (1999), No.1, 74-79. MS (ES+) *m*/*e* 291 (MH⁺).

### Preparation 67: 2-(4-Pyrrolidin-1-yl-phenyl)-ethylamine

The title compd is prepared from [2-(4-pyrrolidin-1-yl-phenyl)-ethyl]-carbamic acid tert-butyl ester by a procedure analogous to that of WO 01/42193. ¹H nmr (CDCl₃, 400 MHz); 7.10 (d; 2H), 6.55(d, 2H), 3.30 (m, 4H), 2.90 (t, 2H), 2.65 (t, 2H), 2.00 (m, 4H), 1.50 (s, 2H).

### Preparation 68: 2-[4-(2-Amino-ethyl)-phenylamino]-1-phenyl-ethanol

The title compound is prepared by the procedure of WO 01/42193.

### Preparation 69: Cis-Bicyclopentyrl-2-ylamine and trans-Bicyclopentyl-2-ylamine

The title compounds are prepared from bicyclopentyl-2-one by procedures analogous to that of R. Hutchins et al J. Org. Chem. 1983, 48, 3412-3422.

### Preparation 70: (1R,2R)-Bicyclopentyl-2-ylamine and (1S,2S)-Bicyclopentyl-2-ylamine

The title compounds are prepared from Bicyclopentyl-2-one by the procedures of S. Hartmann et al Eur. J. Med. Chem. 2000, 35, 377-392.

### Preparation 71: Cis-2-Phenyl-cyclopentylamine

The title compound is prepared from 2-Phenyl-cyclopentanone by procedures analogous to that of R. Hutchins et al J. Org. Chem. 1983, 48, 3412-3422.

### Preparation 72: Trans-2-cyclohexyl-cyclopentanol

Cyclohexylmagnesium chloride (2M in Et₂O, 50 ml, 100 mmol) is placed in a flask with THF (100 ml) under an atmosphere of Argon. Copper (I) iodide (1.90 g, 10 mmol) is added and the reaction mixture is stirred at room temperature for 5 minutes. Cyclopentene oxide (8.40 g, 100 mmol) is then added dropwise (exothermic). The reaction mixture is stirred at room temperature for 4 hours and is quenched with saturated ammonium chloride solution and water (200 ml) is added. The reaction mixture is extracted with ethyl acetate (2 x 200 ml) and the organic layer is washed with water (150 ml) and brine (150 ml), dried over MgSO₄, filtered and the solvent removed *in vacuo*. The title compound is obtained after purification by flash column chromatography (silica, iso-hexane / ethyl acetate 10:1). ¹H nmr (CDCl₃, 400 MHz); 4.00 (s, 1H), 1.90-0.90 (m, 18H).

### Preparation 73: 2-Cyclohexyl-cyclopentanone

20% PCC-Al₂O₃ (103.9 g, 96.42 mmol) is placed in a flask with toluene (50 ml). Trans-2-Cyclohexyl-cyclopentanol (5.40 g, 32.14 mmol) is added with vigorous stirrng and the reaction mixture is stirred at room temperature for 18 hours. The reaction mixture is filtered through Celite and the solvent removed *in vacuo*. The title compound is obtained after purification by flash column chromatography (silica, iso-hexane / ethyl acetate 10:1). ¹H nmr (CDCl₃, 400 MHz); 2.35-1.00 (m, 18H).

### Preparation 74: ((1R,2R)-2-Cyclohexyl-cyclopentyrl)-((R)-1-phenyl-ethyl)-amine

2-Cyclohexyl-cyclopentanone (4.00 g, 24.10 mmol) and D-(+)-α-phenylethylamine (3.50 g, 28.92 mmol) are placed in a flask with 1,2-dichloroethane (15 ml) and acetic acid (1.73 g, 36.14 mmol). Sodium triacetoxyborohydride (7.66 g, 36.14 mmol) is added and the reaction mixture is stirred at room temperature for 48 hours. The reaction mixture is quenched with 0.2M NaOH (200 ml) and the organic layer is washed with water (150 ml) and brine (150 ml), dried over MgSO₄, filtered and the solvent removed *in vacuo*. The title compound is obtained after purification by flash column chromatography (silica, iso-hexane / ethyl acetate 20:1). ¹H nmr (CDCl₃, 400 MHz); 7.40-7.20 (m, 5H), 3.80 (q, 1H), 3.20 (t, 1H), 1.90 (m, 1H), 1.80-1.15 (m, 18H), 1.05-0.80 (m, 3H).

### Preparation 75: (1R,2R)-2-Cyclohexyl-cyclopentylamine

((1R,2R)-2-cyclohexyl-cyclopentyl)-((R)-1-phenyl-ethyl)-amine (2.10 g, 7.75 mmol) is dissolved in methanol (75 ml) and acetic acid (1 ml) under an atmosphere of Argon. 10% Pd-C (0.50 g) is added and the reaction mixture is stirred at room temperature under an atmosphere of hydrogen at 5 atmospheres. The reaction is shown to be complete by TLC after 18 hours. The catalyst is filtered off and the solvent removed *in vacuo*. The residue is partitioned between dichloromethane (200 ml) and 0.2M NaOH (200 ml). The organic layer is washed with water (150 ml) and brine (150 ml), dried over MgSO₄, filtered and the solvent removed *in vacuo* to give the title compound. ¹H nmr (CDCl₃, 400 MHz); 3.30 (m, 1H), 1.80-0.75 (m, 20H).

### Preparation 76: ((R)-1-Phenyl-ethyl)-((1R,2R)-2-o-tolyl-cyclopentyl)-amine

The title compound is prepared using procedures analogous to those of Preparations 72,73, 74 and purification by flash column chromatography (silica, iso-hexane / ethyl acetate 20:1). ¹H nmr (CDCl₃, 400 MHz); 7.40-7.20 (m, 9H), 3.35 (m, 1H), 3.25 (m, 1H), 3.10 (q, 1H), 2.40 (s, 3H), 2.10 (m, 1H), 2.00-1.80 (m, 3H), 1.50 (m, 2H), 1.20 (s, 1H), 0.90 (d, 3H).

### Preparation 77: (1R,2R)-2-o-Tolyl-cyclopentylamine

((R)-1-Phenyl-ethyl)-((1R,2R)-2-o-tolyl-cyclopentyl)-amine (1.55 g, 5.56 mmol) is dissolved in methanol (100 ml) and acetic acid (2 ml) under an atmosphere of Argon. 10% Pd-C (0.40 g) is added and the reaction mixture is stirred at room temperature under an atmosphere of hydrogen at 1 atmosphere. The reaction is shown to be complete by TLC after 18 hours. The catalyst is filtered off and the solvent removed *in vacuo*. The residue is partitioned between dichloromethane (200 ml) and 0.2M NaOH (200 ml). The organic layer is washed with water (150 ml) and brine (150 ml), dried over MgSO₄, filtered and the solvent removed *in vacuo* to give the title compound. ¹H nmr (CDCl₃, 400 MHz); 7.20 (m, 1H), 7.10-7.00 (m, 3H), 3.50 (m, 1H), 3.15 (m, 1H), 2.25 (s, 3H), 2.05 (m, 2H), 1.85 (m, 1H), 1.75 (m, 1H), 1.60 (m, 1H), 1.45 (m, 1H), 0.65 (broad s, 2H).

### Preparation 78: 1-Ethyl-3-(2-methyl-allyl)-benzene

Magnesium (0.32 g, 13.49 mmol) is placed in an oven dried flask under an atmosphere of Argon. THF (30 ml) is added followed by a catalytic amount of iodine. 1-Bromo-3-ethylbenzene (2.08 g, 11.24 mmol) is added and the reaction mixture is gently warmed to initiate the reaction. The reaction mixture is stirred at room temperature for 18 hours. Copper bromide dimethylsulfide complex (0.23 g, 1.12 mmol) is added followed by the dropwise addition of methallyl chloride (1.53 g, 16.87 mmol) (exothermic). The reaction mixture is stirred at room temperature for 4 hours and quenched with saturated ammonium chloride solution. The reaction mixture is partitioned between ethyl acetate (200 ml) and water (200 ml). The organic layer is washed with water (150 ml) and brine (150 ml), dried over MgSO₄, filtered and the solvent removed *in vacuo* to give the title compound. ¹H nmr (CDCl₃, 400 MHz); 7.20-6.90 (m, 4H), 4.70 (d, 2H), 3.20 (s, 2H), 2.55 (m, 2H), 1.60 (s, 3H), 1.15 (m, 3H).

### Preparation 79: N-[2-(3-Ethyl-phenyl)-1,1-dimethyl-ethyl]-benzamide

Benzonitrile (283 mg, 2.75 mmol) is dissolved in acetic acid (3 ml) and concentrated sulfuric acid (1 ml). 1-Ethyl-3-(2-methyl-allyl)-benzene (400 mg, 2.50 mmol) is added dropwise and the reaction mixture is stirred at room temperature for 18 hours. The reaction mixture is quenched with ice and partitioned between ethyl acetate (100 ml) and water (100 ml). The organic layer is washed with saturated sodium hydrogencarbonate (100 ml), water (100 ml) and brine (100 ml), dried over MgSO₄, filtered and the solvent removed *in vacuo*. The title compound is obtained after purification by flash column chromatography (silica, iso-hexane / ethyl acetate 10:1). ¹H nmr (CDCl₃, 400 MHz); 7.65(m, 2H), 7.50-7.40 (m, 3H), 7.20 (m,1H), 7.10-7.00 (m, 3H), 5.75 (broad s, 1H), 3.15 (s, 2H), 2.60 (q, 2H), 1.50 (s, 6H), 1.15 (t, 3H).

### Preparation 80: Benzyl-[2-(3-ethyl-phenyl)-1,1-dimethyl-ethyl]-amine

N-[2-(3-Ethyl-phenyl)-1,1-dimethyl-ethyl]-benzamide (300 mg, 1.12 mmol) is dissolved in THF (2 ml). Borane-THF complex (1M in THF) (3.37 ml, 3.37 mmol) is added and the reaction mixture is refluxed for 48 hours. The reaction mixture is quenched with concentrated hydrochloric acid (1 ml) and stirred at room temperature for 30 minutes. The reaction mixture is partitioned between ethyl acetate (100 ml) and 2M NaOH (100 ml). The organic layer is washed with water (100 ml) and brine (100 ml), dried over MgSO₄, filtered and the solvent removed *in vacuo* to give the title compound. ¹H nmr (CDCl₃, 400 MHz); 7.50-6.80 (m, 9H), 3.15 (s, 2H), 2.60 (s, 2H), 2.45 (q, 2H), 1.00 (t, 3H), 0.95 (s, 6H),

### Preparation 81: 2-(3-Ethyl-phenyl)-1,1-dimethyl-ethylamine

Benzyl-[2-(3-ethyl-phenyl)-1,1-dimethyl-ethyl]-amine (220 mg, 0.87 mmol) is dissolved in methanol (50 ml) under an atmosphere of Argon. 10% Pd-C (50 mg) is added and the reaction mixture is stirred at room temperature under an atmosphere of hydrogen at 1 atmosphere. The reaction is shown to be complete by TLC after 18 hours. The catalyst is filtered off and the solvent removed *in vacuo*. The residue is partitioned between dichloromethane (50mL) and water (50 ml). The organic layer is washed with water (50 ml) and brine (50 ml), dried over MgSO₄, filtered and the solvent removed *in vacuo* to give the title compound. ¹H nmr (CDCl₃, 400 MHz); 7.15-6.90 (m, 4H), 2.60 (m, 4H), 1.15 (t, 3H), 1.05 (s, 6H).

### Preparation 82: Trans-2-(Trityl-amino)-cyclopentanol

Trans-2-amino-cyclopentanol (1.83 g, 18.09 mmol), triphenylmethyl chloride (4.54 g, 16.28 mmol) and triethylamine (6.30 ml, 45.22 mmol) are placed in a flask with dichloromethane (100 ml). The reaction mixture is refluxed for 18 hours, allowed to cool and the solvent removed *in vacuo*. The title compound is obtained after purification by flash column chromatography (silica, iso-hexane / ethyl acetate 5:1). ¹H nmr (CDCl₃, 400 MHz); 7.35 (m, 5H), 7.10-6.95 (m, 10H), 3.40 (m, 1H), 2.55 (m, 1H), 1.55 (m, 2H), 1.40-0.90 (m, 6H).

### Preparation 83: Trans-2-(4-Chloro-benzyloxy)-cyclopentyl-trityl-amine

Trans-2-(Trityl-amino)-cyclopentanol (500 mg, 1.45 mmol) is placed in an oven dried flask under an atmosphere of argon. DMF (2.18 ml) and THF (11 ml) are added followed by sodium hydride (63 mg, 2.62 mmol). The reaction mixture is stirred at room temperature for 1 hour. 4-Chlorobenzyl bromide (327 mg, 1.59 mmol) and sodium iodide (20 mg, 0.13 mmol) are added and the reaction mixture is stirred at room temperature for 18 hours. The reaction mixture is quenched with water and partitioned between ethyl acetate (100 ml) and water (100 ml). The organic layer is washed with water (50 ml) and brine (50 ml), dried over MgSO₄, filtered and the solvent removed *in vacuo*. The title compound is obtained after purification by flash column chromatography (silica, iso-hexane / ethyl acetate 9:1).). ¹H nmr (CDCl₃, 400 MHz); 7.55 (m, 5H), 7.35-7.15 (m, 14H), 4.45 (d, 1H), 4.30 (d, 1H), 3.55 (m, 1H), 3.00 (m, 1H), 1.85-0.60 (m, 7H).

### Preparation 84: Trans-2-(4-Chloro-benzyloxy)-cyclopentylamine

Trans-2-(4-Chloro-benzyloxy)-cyclopentyl-trityl-amine (383 mg, 0.82 mmol) is dissolved in ethanol (1.6 ml) and dichloromethane (1.5 ml). Concentrated hydrochloric acid (0.1 ml) is added and the reaction mixture is refluxed for 1.5 hours, allowed to cool and the solvent removed *in vacuo*. The residue is partitioned between ethyl acetate (50 ml) and 2M hydrochloric acid (50 ml). The aqueous layer is basified to pH12 with 2M NaOH and extracted with ethyl acetate (3 x 50 ml). The organic layer is washed with water (50 ml) and brine (50 ml), dried over MgSO₄, filtered and the solvent removed in *vacuo* to give the title compound. ¹H nmr (CDCl₃, 400 MHz); 7.15 (m, 4H), 4.40 (d, 1H), 4.30 (d, 1H), 3.40 (m, 1H), 3.10 (m, 1H), 1.80 (m, 2H), 1.60-1.40 (m, 3H), 1.20 (m, 1H).

### Preparation 85: (S)-2-((1S,2S)-2-Benzyloxy-cyclopentylamino)-1-(4-tert-butoxy-2-isopropoxybenzothiazol-7-yl)-ethanol

The title compound is prepared from (S)-1-(4-tert-butoxy-2-isopropoxy-benzothiazol-7-yl)-2-chloro-ethanol and (1S,2S)-2-Benzyloxy-cyclopentylamine using procedures analogous to those of Preparations 30 and43. MS (ES+) *m*/*e* 499 (MH⁺).

### Preparation 86: (1R,2S)-2-Aminocyclopentanol hydrochloride

The title compound is prepared from by the procedure of Schaus, Scott E.; Larrow, Jay F.; Jacobsen, Eric N. Practical Synthesis of Enantiopure Cyclic 1,2-Amino Alcohols via Catalytic Asymmetric Ring Opening of Meso Epoxides. Journal of Organic Chemistry (1997), 62(12), 4197-4199.

### Preparation 87: (1S,2R)-2-(2-Hydroxy-cyclopentyl)-isoindole-1,3-dione

(1R,2S)-2-Aminocyclopentanol hydrochloride (10 g, 72.73 mmol), phthalic anhydride (10.76 g, 72.73 mmol) and diisopropylamine (11.26 g, 87.27 mmol) are placed in a flask and heated to 130°C. The reaction is shown to be complete by TLC after 2 hours. The reaction mixture is allowed to cool and is partitioned between ethyl acetate (200 ml) and 2M hydrochloric acid (200 ml). The organic layer is washed with water (1000 ml), sat. NaHCO₃ (100 ml) and brine (100 ml), dried over MgSO₄, filtered and the solvent removed *in vacuo.* The title compound is obtained after purification by flash column chromatography (silica, iso-hexane / ethyl acetate 1:1). ¹H nmr (CDCl₃, 400 MHz); 7.85 (m, 2H), 7.70 (m, 2H), 4.50 (m, 1H), 4.30 (m, 1H), 2.95 (d, 1H), 2.45 (m, 1H), 2.00 (m, 3H), 1.85 (m, 1H), 1.60 (m, 1H).

### Preparation 88: (1S,2R)-2-(2-Benzyloxy-cyclopentyl)-isoindole-1,3-dione

(1S,2R)-2-(2-Hydroxy-cyclopentyl)-isoindole-1,3-dione (7.50 g, 32.47 mmol) is dissolved in dry DMF (15 ml) under an atmosphere of argon. The solution is cooled to 0°C and sodium hydride (0.78 g, 32.47 mmol) is added. The reaction mixture is stirred at room temperature for 30 minutes and then cooled on ice. Benzyl bromide (6.11 g, 35.71 mmol) is added dropwise and the reaction mixture is stirred at room temperature for 18 hours. The reaction is shown to be complete by TLC. The reaction mixture is partitioned between ethyl acetate (200 ml) and water (200 ml). The organic layer is washed with brine (100 ml), dried over MgSO₄, filtered and the solvent removed *in vacuo*. The title compound is obtained after purification by flash column chromatography (silica, iso-hexane / ethyl acetate 10:1). ¹H nmr (CDCl₃, 400 MHz); 7.80 (m, 2H), 7.65 (m, 2H), 7.10 (m, 5H), 4.65 (q, 1H), 4.50 (d, 1H), 4.35 (d, 1H), 4.00 (q, 1H), 2.70 (m, 1H), 2.00 (m, 4H), 1.50 (m, 1H).

### Preparation 89: (1S,2R)-2-Benzyloxy-cyclopentylamine

(1S,2R)-2-(2-Benzyloxy-cyclopentyl)-isoindole-1,3-dione 5.50 g, 17.13 mmol) is dissolved in EtOH (175 ml). Acetic acid (3.08 g, 51.40 mmol) and hydrazine monohydrate (2.57 g, 51.40 mmol) are added and the reaction mixture is refluxed for 2 hours, allowed to cool, any solids are filtered off and the solvent removed *in vacuo*. The residue is partitioned between ethyl acetate (100 ml) and 2M hydrochloric acid (100 ml). The aqueous layer is basified to pH12 with 2M NaOH and extracted with ethyl acetate (3 x 50 ml). The organic layer is washed with brine (100 ml), dried over MgSO₄, filtered and the solvent removed in *vacuo* to give the title compound. ¹H nmr (CDCl₃, 400 MHz); 7.80 (m, 2H), 7.65 (m, 2H), 7.10 (m, 5H), 4.65 (q, 1H), 4.50 (d, 1H), 4.35 (d, 1H), 4.00 (q, 1H), 2.70 (m, 1H), 2.00 (m, 4H), 1.50 (m, 1H).

### Preparation 90: (1R,2S)-2-Benzyloxy-cyclopentylamine

The title compound is prepared from (1S,2R)-2-Aminocyclopentanol hydrochloride using procedures analogous to those of Preparations 86, 87 and 88. ¹H nmr (CDCl₃, 400 MHz); 7.80 (m, 2H), 7.65 (m, 2H), 7.10 (m, 5H), 4.65 (q, 1H), 4.50 (d, 1H), 4.35 (d, 1H), 4.00 (q, 1H), 2.70 (m, 1H), 2.00 (m, 4H), 1.50 (m, 1H).

### Preparation 91: (R)-2-((1S,2R)-2-Benzyloxy-cyclopentylamino)-1-(4-tert-butoxy-2-isopropoxybenzothiazol-7-yl)-ethanol

The title compound is prepared from (R)-1-(4-tert-butoxy-2-isopropoxy-benzothiazol-7-yl)-2-chloro-ethanol and (1S,2R)-2-Benzyloxy-cyclopentylamine using procedures analogous to those of Preparations 30 and 43. MS (ES+) *m*/*e* 499 (MH⁺).

### Preparation 92: (R)-2-((1S,2S)-2-Benzyloxy-cyclopentylamino)-1-(4-tert-butoxy-2-isopropoxybenzothiazol-7-yl)-ethanol

4-tert.butoxy-2-isopropoxy-7-(R)-oxiranyl-benzothiazole (13.2 g, 42.94 mmol) and (1S,2S)-2-Benzyloxy-cyclopentylamine (10.68 g, 55.82 mmol) are placed in a flask with Diglyme (40 ml) and the reaction mixture is heated at 115°C. The reaction is shown to be complete by TLC after 17 hours. The reaction mixture is cooled and partitioned between heptane (200 ml) and water (200 ml). The organic layer is dried over MgSO₄, filtered and the solvent removed *in vacuo*. The title compound is purified by crystallisation from isopropanol. MS (ES+) *m*/*e* 499 (MH⁺).

### Examples 1 to 9

The following examples concern especially preferred compounds of formula I that are also compounds of formula XII 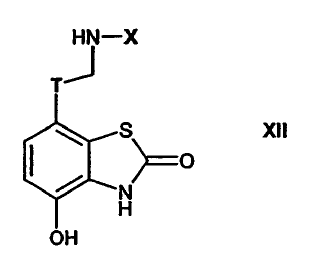 wherein T and X are as shown in the following table, the method of preparation being described hereinafter. The table also shows characterising mass spectrometry data ([MH]+). The compounds of Examples 1 to 5 and 8 are prepared as free salts. The other compounds are made as hydrochloride salts.

**TABLE 1**

| **Ex.** | **T** | **X** | **MS [MH]+** |
|---|---|---|---|
| 1 | 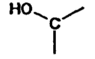 | 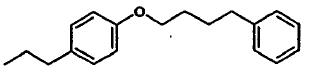 | 479 |
| 2 | 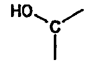 | 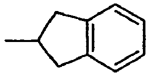 | 343 |
| 3 | 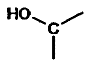 | 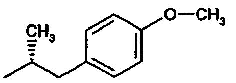 | 375 |
| 4 | 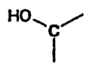 | 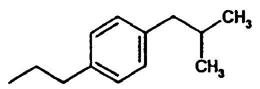 | 387 |
| 5 | 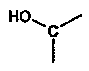 | 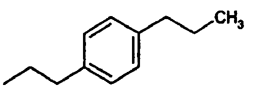 | 373 |
| 6 | 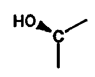 | 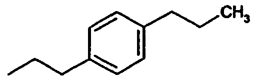 | 373 |
| 7 | 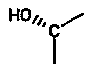 | 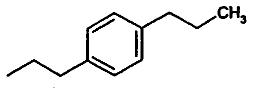 | 373 |
| 8 | 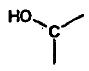 | 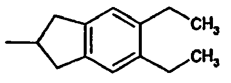 | 399 |
| 9 | 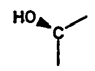 | 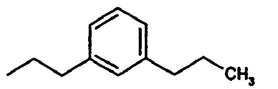 | 373 |

### Example 1: 4-hydroxy-7-(1-hydroxy-2-{2-[4-(4-phenyl-butoxy)-phenyl]-ethylamino}-ethyl)-3H-benzothiazol-2-one

Palladium black (0.2 g) is added portion-wise to a solution of 7-[2-(benzyl-{2-[4-(4-phenyl-butoxy)-phenyl]-ethyl}-amino)-1-hydroxy-ethyl]-4-hydroxy-3H-benzothiazol-2-one (0.29 g) in formic acid (10 ml) at room temperature. After 1 hour the catalyst is removed by filtration and the filtrate partitioned between CH₃CO₂CH₂CH₃ and aqueous NaHCO₃. Evaporation of the CH₃CO₂CH₂CH₃ layers and recrystallisation from hexane / CH₃CO₂CH₂CH₃ gives the title compound. MS (ES+) 479.

### Example 2: 4-hardroxy-7-[1-hydroxy-2-(indan-2-ylamino)-ethyl]-3H-benzothiazol-2-one

This compound is prepared from the product of Preparation 15 by a procedure analogous to that of Example 1. MS (ES+) 343.

### Example 3: 4-hydroxy-7-{1-hydroxy-2-[(R)-2-(4-methoxy-phenyl)-1-methyl-ethylamino]-ethyl}-3H-benzothiazol-2-one

The title compound is prepared from 2-{benzyl-[(R)-2-(4-methoxy-phenyl)-1-methyl-ethyl]-amino}-N-methoxy-N-methyl-acetamide and (2-tert-butoxy-5-fluoro-phenyl)-thiocarbamic acid O-isopropyl by following procedures analogous to those of Preparations 10, 11 and 12 and Example 1. MS (ES+) 375.

### Example 4: 4-hydroxy-7-{1-hydroxy-2-[2-(4-isobutyl-phenyl)-ethylamino]-ethyl}-3H-benzothiazol-2-one

The title compound is prepared from 2-{benzyl-[2-(4-isobutyl-phenyl)-ethyl]-amino}-N-methoxy-N-methyl-acetamide and (2-tert-butoxy-5-fluoro-phenyl)-thiocarbamic acid O-isopropyl ester by procedures analogous to those of Preparations 10, 11 and 12 and Example 1. MS (ES+) 387.

### Example 5: 4-hydroxy-7-{1-hydroxy-2-[2-(4-propyl-phenyl)-ethylamino]-ethyl}-3H-benzothiazol-2-one

The title compound is prepared from 2-{benzyl-[2-(4-isobutyl-phenyl)-ethyl]-amino}-N-methoxy-N-methyl-acetamide and (2-tert-butoxy-5-fluoro-phenyl)-thiocarbamic acid O-isopropyl ester by procedures analogous to those of Preparations 10, 11 and 12 and Example 1. ¹H nmr (MeOH-*d₄*, 400 MHz); 7.16-6.97 (m, 4H), 6.86 (d, 1H, *J* = 8 Hz), 6.65 (d, 1H, *J* = 8 Hz), 4.87-4.85 (m, 1H), 3.20-3.12 (m, 2H), 3.10-3.02 (m, 2H), 2.89-2.87 (m, 2H), 2.49-2.44 (m, 2H), 1.57-1.46 (m, 2H), 0.84-0.79 (m, 3H).

### Example 6: 4-hydroxy-7-{(R)-1-hydroxy-2-[2-(4-propyl-phenyl)-ethylamino]-ethyl}-3H-benzothiazol-2-one hydrochloride

Palladium black (5 g) is added portionwise to a solution of (R)-2-{benzyl-[2-(4-propyl-phenyl)-ethyl]-amino}-1-(4-tert-butoxy-2-isopropoxy-benzothiazol-7-yl)-ethanol (4.00 g in formic acid (25 ml) at room temperature. After 1 hour, filtration and evaporation gives the formate salt, which is dissolved in boiling ethyl acetate : MeOH (1 : 1), treated with decolourising charcoal and filtered hot. The solution is allowed to cool, 1 M HCl in Et₂O (10 ml) is added and the solvent is removed *in vacuo.* The solid is triturated with ethyl acetate, filtered and dried. MS (ES+) *m*/*e* 373 (MH⁺).

### Example 7: 4-hydroxy-7-{(S)-1-hydroxy-2-[2-(4-propyl-phenyl)-ethylamino]-ethyl}-3H-benzothiazol-2-one hydrochloride

The title compound is prepared from (S)-1-(4-tert-butoxy-2-isopropoxy-benzothiazol-7-yl)-2-chloro-ethanol by procedures analogous to those of Preparations 30 and 31 and Example 6. MS (ES+) *m*/*e* 373 (MH⁺).

### Example 8: 7-[2-(5,6-diethyl-indan-2-ylamino)-1-hydroxy-ethyl]-4-hydroxy-3H-benzothiazol-2-one

The title compound is prepared from 2-[benzyl-(5,6-diethyl-indan-2-yl)-amino]-N-methoxy-N-methyl-acetamide and (2-tert-butoxy-5-fluoro-phenyl)-thiocarbamic acid O-isopropyl ester by procedures analogous to those of Prep. 10,11 and 12 and Example 1. MS (ES+) 399.

### Example 9: 4-hydroxy-7-{(R)-1-hydroxy-2-[2-(3-propyl-phenyl)-ethylamino]-ethyl}-3H-benzothiazol-2-one hydrochloride

The title compd is prepared from (R)-2-{benzyl-[2-(3-propyl-phenyl)-ethyl]-amino}-1-(4-tert-butoxy-2-isopropoxy-benzothiazol-7-yl)-ethanol by a procedure analogous to that of Ex. 6.

### Examples 10 to 47

The following examples concern especially preferred compounds of formula I that are also compounds of formula XIII 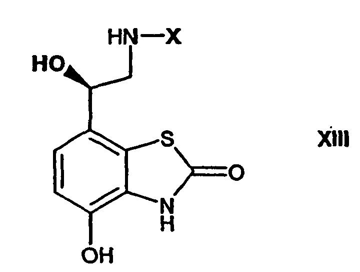 wherein X (of formula R¹-Ar-R²) is as shown in Table 2 below, the method of preparation being described hereinafter. The table also shows characterising mass spectrometry data ([MH]+). The compounds of Examples 12 and 16 to 18 are prepared as formate salts. The other compounds are made as trifluoroacetate salts. "Prep." refers to the number of one or more of any Preparations of starting compounds detailed above that are used to prepare the compound of the Example.

Compounds are analysed by high performance liquid chromatography (hplc) with mass spectral detection and "R. Time" represents the retention time for the compound with "MS" as the base peak from the mass spectra of the component eluting at that time point, from either a; CHROMOLITH RP18 SPEEDROD™ chromato-graphic column of dimensions 50 x 4.6 mm with the eluent system; A = 0.1 % HCOOH in water, B = 0.1 % HCOOH in acetonitrile, 5 to 95% B in 2.5 minutes at 3 ml/min at 25° C, or, where the reading is followed by an asterisk*, a CHROMOLITH RP18 SPEEDROD™ chromatographic column of dimensions 50 x 4.6 mm with the eluent system A = 0.1 % TFA in water, B = 0.1 % TFA in acetonitrile, 0 to 95% B in 2.5 minutes at 3 ml/min at 25° C.

**TABLE 2**

| **Ex.** | **X** | **Prep.** | **R. Time** | **MS [MH]+** |
|---|---|---|---|---|
| 10 | 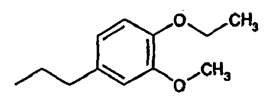 | 39,40 | 1.26 | 405.10 |
| 11 | 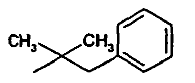 | 43 | 1.30 | 358.95 |
| 12 | 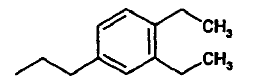 | 37, 38 | 1.45 | 387.45 |
| 13 | 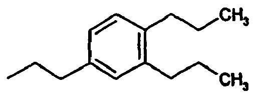 | 41,42 | 1.6 | 415.03 |
| 14 | 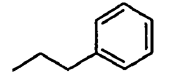 | - | 1.21 | 330.95 |
| 15 | 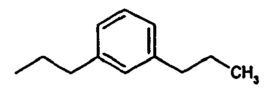 | 44 | 1.43 | 373.16 |
| 16 | 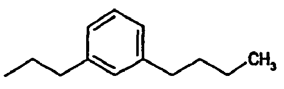 | 45 | 1.55 | 387.13 |
| 17 | 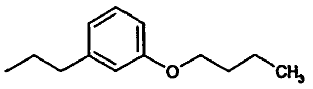 | 49 | 1.63 | 403.1 |
| 18 | 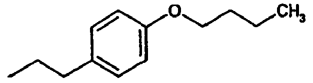 | 46,47, 48 | 1.5 | 403.09 |
| 19 | 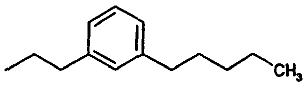 | 50 | 1.58 | 401.12 |
| 20 | 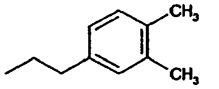 | - | 1.37 | 359.09 |
| 21 | 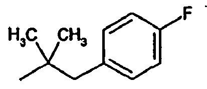 | - | 1.37 | 377.06 |
| 22 | 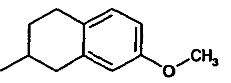 | - | 1.34 | 387.07 |
| 23 | 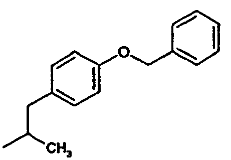 | - | 1.55 | 451.12 |
| 24 | 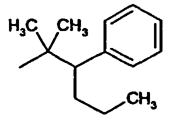 | - | 1.48 | 401.11 |
| 25 | 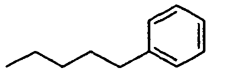 | - | 1.39 | 359.08 |
| 26 | 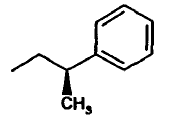 | - | 1.28 | 345.05 |
| 27 | 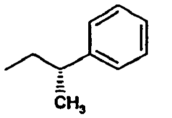 | - | 1.28 | 345.05 |
| 28 | 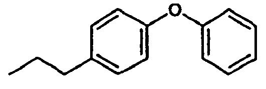 | - | 1.48 | 423.09 |
| 29 | 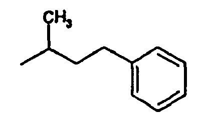 | - | 1.35 | 359.06 |
| 30 | 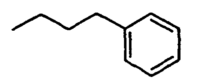 | - | 1.32 | 345.05 |
| 31 | 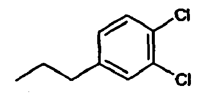 | - | 1.44 | 398.99 |
| 32 | 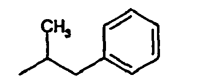 | - | 1.3 | 345.02 |
| 33 | 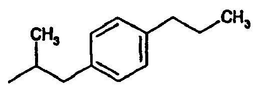 | 55, 56, 57, 58 | 1.53 | 387.08 |
| 34 | 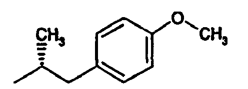 | 59 | 1.32 | 375.76 |
| 35 | 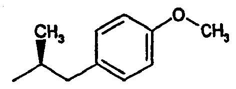 | 60 | 1.3 | 375.76 |
| 36 | 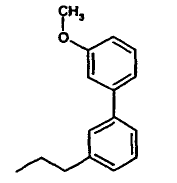 | 51, 52, 53, 54 | 1.48 | 437.87 |
| 37 | 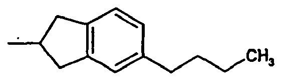 | 61, 62, 63,64 | 1.55 | 398.9 |
| 38 | 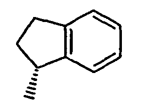 | - | 1.22* | 342.86 |
| 39 | 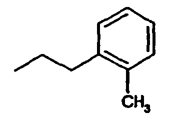 | - | 1.29* | 344.87 |
| 40 | 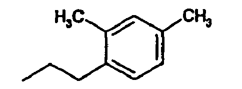 | - | 1.41* | 358.99 |
| 41 | 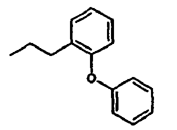 | - | 1.47* | 422.96 |
| 42 | 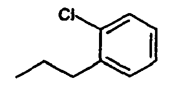 | - | 1.52* | 364.96 |
| 43 | 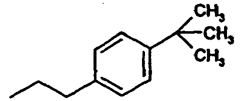 | - | 1.73* | 387.07 |
| 44 | 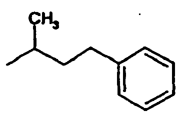 | - | 1.52* | 359.01 |
| 45 | 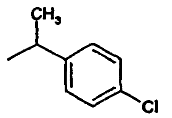 | - | 1.5* | 364.97 |
| 46 | 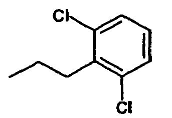 | - | 1.52* | 400.94 (50%) |
| 47 | 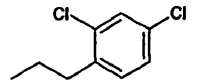 | - | 1.6* | 400.93 (25%) |

### Example 10: 7-{(R)-2-[2-(4-Ethoxy-3-methoxy-phenyl)-ethylamino]-1-hydroxy-ethyl}-4-hydroxy-3H-benzothiazol-2-one trifluoroacetate

Palladium black (0.4 g) is added portion-wise to a solution of (R)-2-{Benzyl-[2-(4-ethoxy-3-methoxy-phenyl)-ethyl]-amino}-1-(4-tert-butoxy-2-isopropoxy-benzothiazol-7-yl)-ethanol (0.45 g) in formic acid (5 ml) at room temperature. After 24 hours the catalyst is removed by filtration. Evaporation of the formic acid and purification by reverse phase chromatography (ISOLUTE FLASH C18, 0-50% methylcyanide in water (0.1% TFA)) gives the title compound. MS (ES+) *m*/*e* 405 (MH⁺).

### Example 11: 7-[(R)-2-(1,1-Dimethyl-2-phenyl-ethylamino)-1-hydroxy-ethyl]-4-hydroxy-3H-benzothiazol-2-one

A solution of (R)-1-(4-tert-Butoxy-2-isopropoxy-benzothiazol-7-yl)-2-(1,1dimethyl-2-phenyl-ethylamino)-ethanol in formic acid (10 ml) is stirred at room temperature. The reaction is shown to be complete by LCMS after 48 hours. Evaporation of the formic acid and purification by reverse phase chromatography (ISOLUTE FLASH C18, 0-50% methylcyanide in water (0.1% TFA)) gives the title compound. MS (ES+) *m*/*e* 359 (MH⁺).

### Example 12: 7-{(R)-2-[2-(3,4-Diethyl-phenyl)-ethylamino]-1-hydroxy-ethyl]-4-hydroxy-3H-benzothiazol-2-one

Palladium black (0.4 g) is added portion-wise to a solution of (R)-2-{2-(3,4-Diethyl-phenyl)-ethylamino]-1-hydroxy-ethyl]-amino}-1-(4-tert-butoxy-2-isopropoxy-benzothiazol-7-yl)-ethanol (prepared from the product of Preparation 38 following a procedure analogous to that of Preparation 31) (0.40 g) in formic acid (5 ml) at room temperature. After 24 hours the catalyst is removed by filtration. Evaporation of the formic acid and purification by reverse phase chromatography (ISOLUTE FLASH C18, 0-50% methylcyanide in water (0.1% formic acid)) gives the title compound. MS (ES+) *m*/*e* 387.05 (MH⁺).

The compounds of Examples 13, 15, 19 and 32 are made using procedures that are analogous to that used in Example 10.

The compounds of Examples 14, 20 to 31 and 33 to 47 are made using procedures that are analogous to that used in Example 11.

The compounds of Examples 16 to 18 are made using procedures that are analogous to that used in Example 12.

### Examples 48 to 131

The following examples concern especially preferred compounds of formula I that are also compounds of formula XIII wherein X (of formula R¹-Ar-R² or R^{a}-Y) is as shown in Table 3 below, the method of preparation being described hereinafter. The table also shows characterising mass spectrometry data ([MH]+). All of the compounds are prepared as trifluoroacetate salts. "Prep." refers to the number of one or more of any Preparations of starting compounds detailed above that are used to prepare the compound of the Example.

Compounds are analysed by high performance liquid chromatography (hplc) with mass spectral detection and "R. Time" represents the retention time for the compound with "MS" as the base peak from the mass spectra of the component eluting at that time point, from either a; CHROMOLITH RP18 SPEEDROD™ chromatographic column of dimensions 50 x 4.6 mm with the eluent system; A = 0.1% HCOOH in water, B = 0.1% HCOOH in acetonitrile, 5 to 95% B in 2.5 minutes at 3 ml/min at 25° C, or, where the reading is followed by an asterisk*, a CHROMOLITH RP18 SPEEDROD™ chromatographic column of dimensions 50 x 4.6 mm with the eluent system A = 0.1% TFA in water, B = 0.1% TFA in acetonitrile, 0 to 95% B in 2.5 minutes at 3 ml/min at 25° C.

**TABLE 3**

| **Ex.** | **X** | **Prep.** | **R. Time** | **MS [MH]+** |
|---|---|---|---|---|
| 48 | 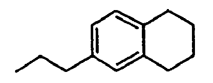 | 33, 34, 35, 36 | 1.44 | 385 |
| 49 | 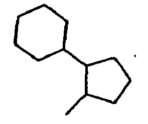 | - | 1.47 | 377.12 |
| 50 | 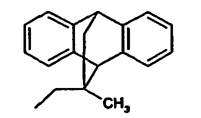 | - | 1.52 | 459.12 |
| 51 | 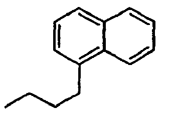 | - | 1.47 | 395.09 |
| 52 | 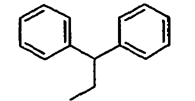 | - | 1.44 | 407.08 |
| 53 | 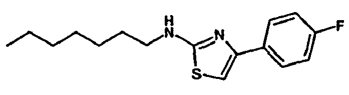 | - | 1.41 | 503.13 |
| 54 | 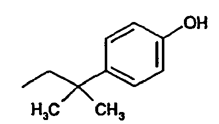 | - | 1.2 | 375.07 |
| 55 | 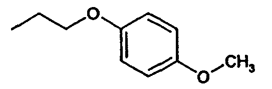 | - | 1.25 | 377.06 |
| 56 | 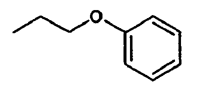 | - | 1.24 | 347.04 |
| 57 | 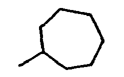 | - | 1.25 | 323.05 |
| 58 | 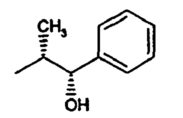 | - | 1.25 | 361.06 |
| 59 | 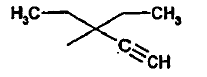 | - | 1.25 | 321.06 |
| 60 | 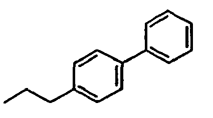 | - | 1.5 | 407.09 |
| 61 | 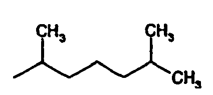 | - | 1.42 | 339.09 |
| 62 | 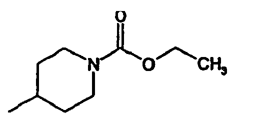 | - | 1.16 | 382.07 |
| 63 | 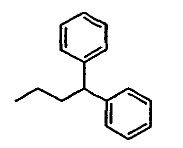 | - | 1.5 | 421.1 |
| 64 | 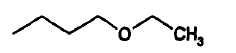 | - | 1.17* | 312.99 |
| 65 | 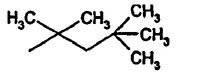 | - | 1.47* | 339.04 |
| 66 | 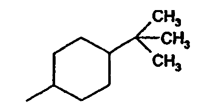 | - | 1.61* | 365.04 |
| 67 | 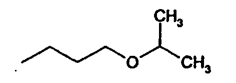 | - | 1.26* | 326.99 |
| 68 | 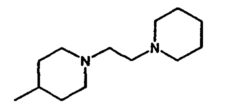 | - | 0.17 | 420.96 |
| 69 | 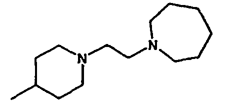 | - | 0.18 | 434.96 |
| 70 | 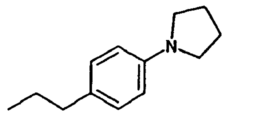 | 65,66, 67 | 1.25 | 399.9 |
| 71 | 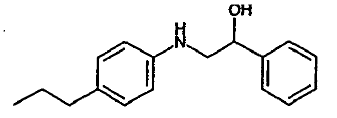 | 68 | 1.3 | 465.01 |
| 72 | 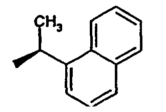 | - | 1.33* | 380.86 |
| 73 | 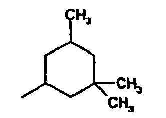 | - | 1.6* | 351.04 |
| 74 | 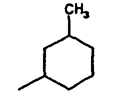 | - | 1.45* | 323.01 |
| 75 | 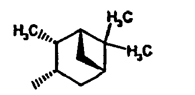 | - | 1.62* | 363.02 |
| 76 | 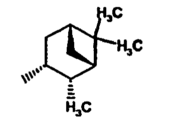 | - | 1.62* | 363.03 |
| 77 | 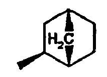 | - | 1.41* | 320.98 |
| 78 | 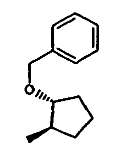 | - | 1.55* | 401.01 |
| 79 | 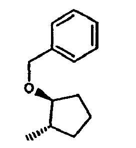 | - | 1.54* | 401.01 |
| 80 | 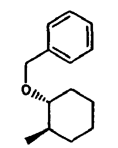 | - | 1.62* | 415.02 |
| 81 | 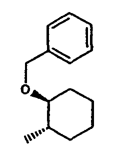 | - | 1.61* | 415.01 |
| 82 | 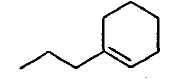 | - | 1.36* | 334.99 |
| 83 | 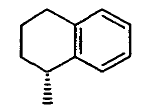 | - | 1.3* | 356.99 |
| 84 | 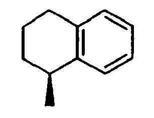 | - | 1.31* | 356.99 |
| 85 | 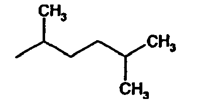 | - | 1.33* | 324.99 |
| 86 | 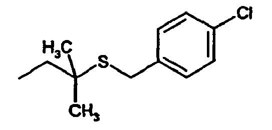 | - | 1.49* | 438.93 |
| 87 | 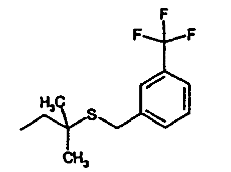 | - | 1.53* | 472.94 |
| 88 | 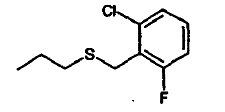 | - | 1.41* | 428.89 |
| 89 | 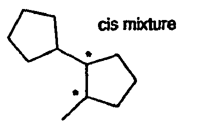 | 69 | 1.42 | 363.02 |
| 90 | 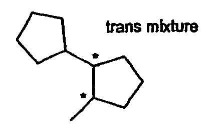 | 69 | 1.43 | 363.01 |
| 91 | 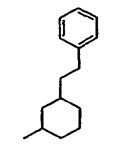 | - | 1.55 | 413.03 |
| 92 | 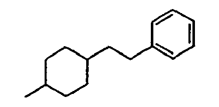 | - | 1.56 | 413.02 |
| 93 | 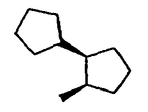 | 70 | 1.42 | 393.01 |
| 94 | 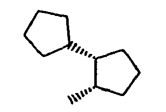 | 70 | 1.43 | 363.02 |
| 95 | 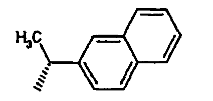 | - | 1.57* | 381.01 |
| 96 | 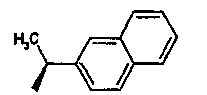 | - | 1.57* | 381.01 |
| 97 | 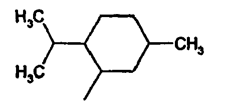 | - | 1.67* | 365.07 |
| 98 | 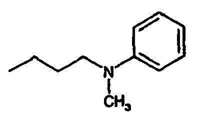 | - | 1.28* | 374.04 |
| 99 | 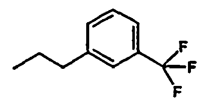 | - | 1.63* | 398.99 |
| 100 | 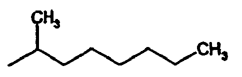 | - | 1.65* | 339.06 |
| 101 | 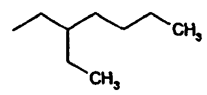 | - | 1.64* | 339.06 |
| 102 | 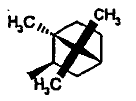 | - | 1.59* | 363.06 |
| 103 | 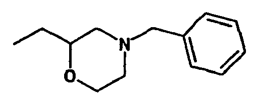 | - | 1.29* | 416.06 |
| 104 | 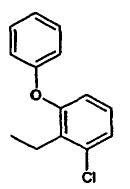 | - | 1.65* | 442.98 |
| 105 | 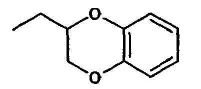 | - | 1.47* | 374.99 (80%) |
| 106 | 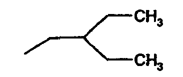 | - | 1.46* | 311.03 |
| 107 | 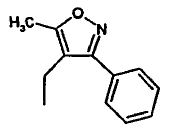 | - | 1.46* | 398.01 |
| 108 | 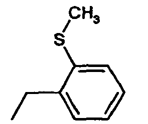 | - | 1.46* | 362.97 |
| 109 | 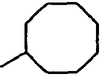 | - | 1.53* | 337.05 |
| 110 | 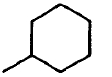 | - | 1.39* | 309.01 |
| 111 | 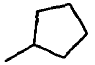 | - | 1.32* | 295 |
| 112 | 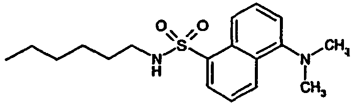 | - | 1.45* | 545.1 |
| 113 | 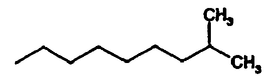 | - | 1.67* | 353.06 |
| 114 | 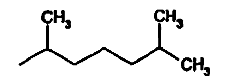 | - | 1.6* | 339.04 |
| 115 | 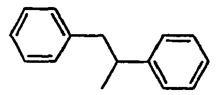 | - | 1.61* | 407.02 |
| 116 | 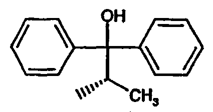 | - | 1.57* | 437.02 |
| 117 | 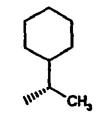 | - | 1.5* | 337.02 |
| 118 | 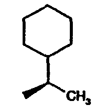 | - | 1.5* | 337.03 |
| 119 | 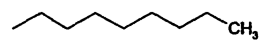 | - | 1.67* | 339.05 |
| 120 | 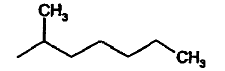 | - | 1.53* | 325.03 |
| 121 | 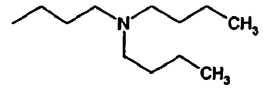 | - | 1.35* | 396.1 |
| 122 | 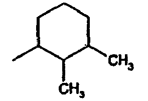 | - | 1.5* | 337.03 |
| 123 | 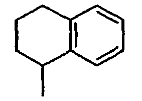 | - | 1.47* | 357.02 |
| 124 | 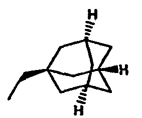 | - | 1.6* | 375.05 |
| 125 | 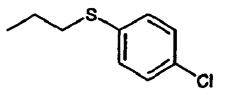 | - | 1.59* | 396.94 |
| 126 | 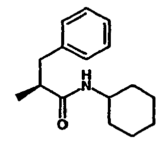 | - | 1.63* | 456.08 |
| 127 | 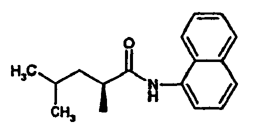 | - | 1.65* | 466.09 |
| 128 | 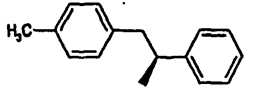 | - | 1.67* | 421.04 |
| 129 | 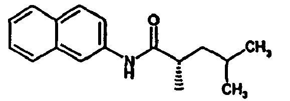 | - | 1.71* | 466.07 |
| 130 | 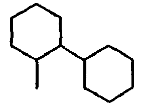 | - | 1.7* | 391.08 |
| 131 | 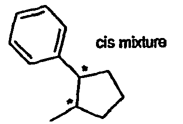 | 71 | 1.21 | 371 |

### Example 48: 4-Hydroxy-7-{(R)-1-hydroxy-2-[2-(5,6,7,8-tetrahydro-naphthalen-2-yl)-ethylamino]-ethyl}-3H-benzothiazol-2-one formate

Palladium black (0.4 g) is added portion-wise to a solution of (R)-2-{benzyl-[2-(5,6,7,8-tetrahydro-naphthalen-2-yl)-ethyl]-amino}-1-(4-tert-butoxy-2-isopropoxy-benzothiazol-7-yl)-ethanol (0.40 g) in formic acid (5 ml) at room temperature. After 24 hours the catalyst is removed by filtration. Evaporation of the formic acid and purification by reverse phase chromatography (ISOLUTE FLASH C18, 0-50% methylcyanide in water (0.1% formic acid)) gives the title compound. MS (ES+) *m*/*e* 385 (MH+).

The compounds of Examples 49 to 78 are made using procedures that are analogous to that used to prepare the compound of Example 11.

### Example 79: 7-[(R)-2-((1S,2S)-2-Benzyloxy-cyclopentylamino)-1-hydroxy-ethyl]-4-hydroxy-3H-benzothiazol-2-one

(R)-2-((1S,2S)-2-Benzyloxy-cyclopentylamino)-1-(4-tert-butoxy-2-isopropoxy-benzothiazol-7-yl)-ethanol (10 g, 20 mmol) is dissolved in isopropanol (100 ml). 1M HCl (50 ml) is added and the reaction mixture is heated at 80°C for 24 hours. The isopropanol is removed *in vacuo* and the residue is partitioned between ethyl acetate (50 ml) and sat. NaHCO₃. The organic layer is washed with brine (50 ml), dried over MgSO₄, filtered and the solvent removed *in vacuo*. The title compound is purified by crystallisation from ethanol. MS (ES+) *m*/*e* 401 (MH⁺).

The compounds of Examples 80 to 131 are made using procedures that are analogous to that used to prepare the compound of Example 11.

### Examples 132 to 157

The following examples concern especially preferred compounds of formula I that are also compounds of formula XII wherein T and X (of formula R¹-Ar-R² or R^{a}-Y) is as shown in Table 4 below, the method of preparation being described hereinafter. The table also shows characterising mass spectrometry data ([MH]+). The compounds of Examples 154 to 157 are prepared as free bases. The other compounds are made as trifluoroacetate salts. "Prep." refers to the number of one or more of any Preparations of starting compounds detailed above that are used to prepare the compound of the Example.

Compounds are analysed by high performance liquid chromatography (hplc) with mass spectral detection and "R. Time" represents the retention time for the compound with "MS" as the base peak from the mass spectra of the component eluting at that time point, from either a; CHROMOLITH RP18 SPEEDROD™ chromatographic column of dimensions 50 x 4.6 mm with the eluent system; A = 0.1% HCOOH in water, B = 0.1 % HCOOH in acetonitrile, 5 to 95% B in 2.5 minutes at 3 ml/min at 25° C, or, where the reading is followed by an asterisk*, a CHROMOLITH RP18 SPEEDROD™ chromatographic column of dimensions 50 x 4.6 mm with the eluent system A = 0.1 % TFA in water, B = 0.1 % TFA in acetonitrile, 0 to 95% B in 2.5 minutes at 3 ml/min at 25° C.

**TABLE 4**

| **Ex.** | **T** | **X** | **Prep.** | **R. Time** | **MS [MH]+** |
|---|---|---|---|---|---|
| 132 | 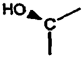 | 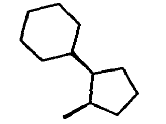 | 75 | 1.47 | 377.28 |
| 133 | 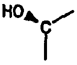 | 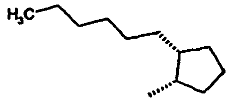 | 75 | 1.83* | 379.33 |
| 134 | 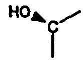 | 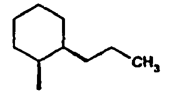 | 75 | 1.43 | 351.23 |
| 135 | 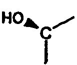 | 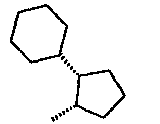 | 75 | 1.49 | 377.28 |
| 136 | 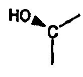 | 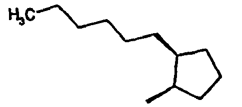 | 75 | 1.80* | 379.34 |
| 137 | 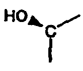 | 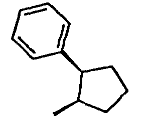 | 75 | 1.32 | 371.22 |
| 138 | 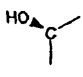 | 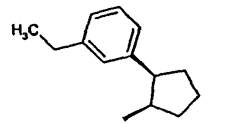 | 75 | 1.41 | 399.27 |
| 139 | 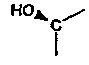 | 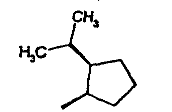 | 77 | 1.19 | 337.25 |
| 140 | 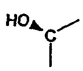 | 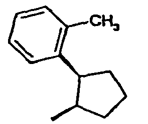 | 77 | 1.31 | 385.25 |
| 141 | 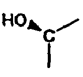 | 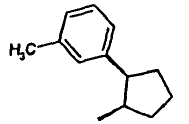 | 77 | 1.33 | 385.25 |
| 142 | 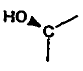 | 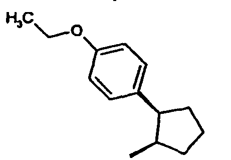 | 77 | 1.61* | 415.31 |
| 143 | 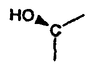 | 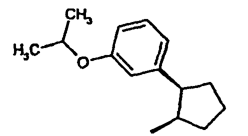 | 77 | 1.63* | 429.32 |
| 144 | 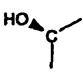 | 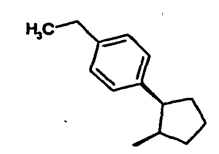 | 75 | 1.62* | 399.29 |
| 145 | 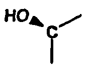 | 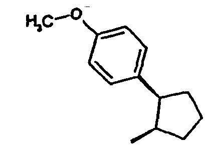 | 75 | 1.52* | 401.26 |
| 146 | 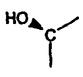 | 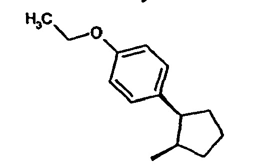 | 75 | 1.55* | 415.27 |
| 147 | 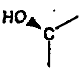 | 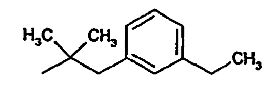 | 81 | 1.47 | 387.25 |
| 148 | 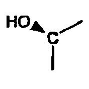 | 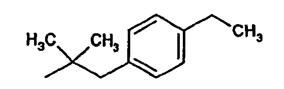 | 81 | 1.65* | 387.29 |
| 149 | 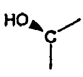 | 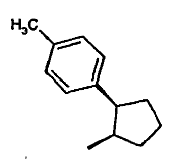 | 77 | 1.33 | 385.26 |
| 150 | 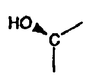 | 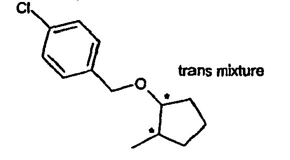 | 84 | 1.61* | 435.26 |
| 151 | 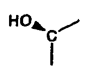 | 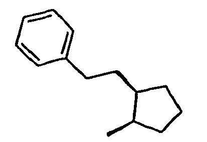 | 77 | 1.51 | 399.27 |
| 152 | 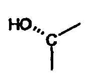 | 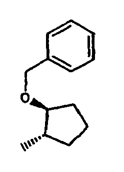 | - | 1.59 | 401.33 |
| 153 | 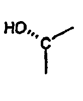 | 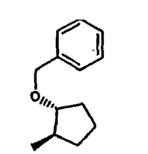 | - | 1.58 | 401.33 |
| 154 | 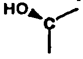 | 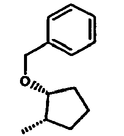 | 89 | 1.54 | 401.18 |
| 155 | 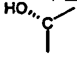 | 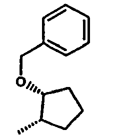 | 89 | 1.52 | 401.18 |
| 156 | 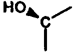 | 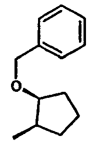 | 90 | 1.53 | 401.17 |
| 157 | 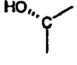 | 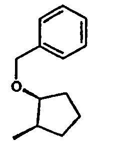 | 90 | 1.54 | 401.18 |

The compounds of Examples 132 to 153 are made using procedures that are analogous to that used to prepare the compound of Example 11.

### Example 154: 7-[(R)-2-((1S,2R)-2-Benzyloxy-cyclopentylamino)-1-hydroxy-ethyl]-4-hydroxy-3H-benzothiazol-2-one

(R)-2-((1S,2R)-2-Benzyloxy-cyclopentylamino)-1-(4-tert-butoxy-2-isopropoxy-benzothiazol-7-yl)-ethanol (460 mg, 0.92 mmol) is dissolved in isopropanol (5 ml). 1M HCl (2.5mL) is added and the reaction mixture is heated at 80°C for 24 hours. The isopropanol is removed *in vacuo* and the residue is partitioned between ethyl acetate (50 ml) and sat. NaHCO₃. The organic layer is washed with brine (50 ml), dried over MgSO₄, filtered and the solvent removed *in vacuo* to give the title compound. MS (ES+) *m*/*e* 401 (MH⁺).

The compounds of Examples 155 to 157 are made using procedures that are analogous to that used to prepare the compound of Example 154.

## Claims

1. A compound of formula I 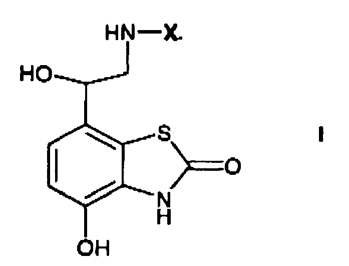 in free or salt or solvate form, wherein
X is -R¹-Ar-R² or -R^{a}-Y;
Ar denotes a phenylene group optionally substituted by halo, hydroxy, C₁-C₁₀-alkyl, C₁-C₁₀-alkoxy, C₁-C₁₀-alkoxy-C₁-C₁₀-alkyL, phenyl, C₁-C₁₀-alkyl substituted by phenyl, C₁-C₁₀-alkoxy substituted by phenyl, C₁-C₁₀-alkyl-substituted phenyl or by C₁-C₁₀-alkoxy-substituted phenyl;
R¹ and R² are attached to adjacent carbon atoms in Ar, and
either R¹ is C₁-C₁₀-alkylene and R² is hydrogen, C₁-C₁₀-alkyl, C₁-C₁₀-alkoxy or halogen, or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₁-C₁₀-alkoxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₁-C₁₀-alkoxy, C₃-C₁₀-cycloalkyl, C₇-C₁₄-aralkyl, C₇-C₁₄-aralkyloxy or C₆-C₁₀-aryl, where C₃-C₁₀-cycloalkyl, C₇-C₁₄-aralkyl, C₇-C₁₄-aralkyloxy or C₆-C₁₀-aryl are optionally substituted by halo, hydroxy, C₁-C₁₀-alkyl, C₁-C₁₀-alkoxy or halo-C₁-C₁₀-alkyl;
C₆-C₁₀-aryl optionally substituted by halo, hydroxy, C₁-C₁₀-alkyl, C₁-C₁₀-alkoxy, C₁-C₁₀-haloalkyl, phenoxy, C₁-C₁₀-alkylthio, C₆-C₁₀-aryl, 4- to 10- membered heterocyclic ring having at least one ring nitrogen, oxygen or sulphur atom, or by NR^{b}R^{c} where R^{b} and R^{c} are each independently C₁-C₁₀-alkyl optionally substituted by hydroxy, C₁-C₁₀-alkoxy or phenyl or R^{b} may additionally be hydrogen;
phenoxy optionally substituted by C₁-C₁₀-alkyl, C₁-C₁₀-alkoxy or by phenyl optionally substituted by C₁-C₁₀-alkyl or C₁-C₁₀-alkoxy;
a 4- to 10-membered heterocyclic ring having at least one ring nitrogen, oxygen or sulphur atom, said heterocyclic ring being optionally substituted by halo, C₁-C₁₀-alkyl, C₁-C₁₀-alkoxy, halo-C₁-C₁₀-alkyl, C₆-C₁₀-aryl, C₇-C₁₄-aralkyl, C₇-C₁₄-aralkyloxy, C₁-C₁₀-alkoxycarbonyl or a 4- to 10-membered heterocyclyl-C₁-C₁₀-alkyl;
-NR^{d}R^{e} where R^{d} is hydrogen or C₁-C₁₀-alkyl and R^{e} is C₁-C₁₀-alkyl optionally substituted by hydroxy, or R^{e} is C₆-C₁₀-aryl optionally substituted by halo, or R^{e} is a 4-to 10-membered heterocyclic ring having at least one ring nitrogen, oxygen or sulphur atom which ring is optionally substituted by phenyl or halo-substitured phenyl or R^{e} is C₆-C₁₀-arylsulfonyl optionally substituted by C₁-C₁₀-alkylamino or di(C₁-C₁₀-alkyl)amino;
-SR^{f} where R^{f} is C₆-C₁₀-aryl or C₇-C₁₄-aralkyl optionally substituted by halo, C₁-C₁₀-alkyl, C₁-C₁₀-alkoxy or C₁-C₁₀-haloalkyl; or
-CONHR^{g} where R^{g} is C₁-C₁₀-alkyl, C₃-C₁₀-cycloalkyl or C₆-C₁₀-aryl.

2. A compound according to claim 1, in which
X is -R¹-Ar-R² or -R^{a}-Y;
Ar denotes a phenylene group optionally substituted by halo, C₁-C₁₀-alkyl, C₁-C₁₀-alkoxy or by C₁-C₁₀-alkoxy substituted by phenyl;
R¹ and R² are attached to adjacent carbon atoms in Ar, and
either R¹ is C₁-C₁₀-alkylene and R² is hydrogen,
or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₃-C₁₀-cycloalkyl, C₇-C₁₄-arakyl, C₇-C₁₄-aralkyloxy optionally substituted by halo, or by C₆-C₁₀-aryl optionally substituted by C₁-C₁₀-alkyl or C₁-C₁₀-alkoxy; C₆-C₁₀-aryl optionally substituted by halo, hydroxy, C₁-C₁₀-alkyl, phenoxy, C₁-C₁₀-alkylthio, C₆-C₁₀-aryl, a 4- to 10-membered heterocyclic ring having at least one ring nitrogen atom, or by NR^{b}R^{c} where R^{b} and R^{c} are each independently C₁-C₁₀-alkyl optionally substituted by hydroxy or phenyl or R^{b} may additionally be hydrogen; phenoxy optionally substituted by C₁-C₁₀-alkoxy; a 4- to 10-membered heterocyclic ring having at least one ring nitrogen or oxygen atom, said heterocyclic ring being optionally substituted by C₁-C₁₀-alkyl, C₆-C₁₀-aryl, C₇-C₁₄-aralkyl, C₁-C₁₀-alkoxycarbonyl or by a 4- to 10-membered heterocyclyl-C₁-C₁₀-alkyl; -NR^{d}R^{e} where R^{d} is hydrogen or C₁-C₁₀-alkyl and R^{e} is C₁-C₁₀-alkyl, or R^{e} is a 4- to 10-membered heterocyclic ring having at least one ring nitrogen or oxygen atom which ring is optionally substituted by halo-substituted phenyl or R^{e} is C₆-C₁₀-arylsulfonyl optionally substituted by di(C₁-C₁₀-alkyl)amino; -SR^{f} where R^{f} is C₆-C₁₀-aryl or C₇-C₁₄-aralkyl optionally substituted by halo or C₁-C₁₀-haloalkyl; or -CONHR^{g} where R^{g} is C₃-C₁₀-cycloalkyl or C₆-C₁₀-aryl.

3. A compound according to claim 2, in which
X is -R¹-Ar-R² or -R^{a}-Y;
Ar denotes a phenylene group optionally substituted by halo, C₁-C₄-alkyl, C₁-C₄-alkoxy or by C₁-C₄-alkoxy substituted by phenyl;
R¹ and R² are attached to adjacent carbon atoms in Ar, and
either R¹ is C₁-C₄-alkylene and R² is hydrogen,
or R¹ and R² together with the carbon atoms in Ar to which they are attached denote a 5-, 6-or 7-membered cycloaliphatic ring, especially a 5-membered cycloaliphatic ring;
R^{a} is a bond or C₁-C₄-alkylene optionally substituted by hydroxy, C₆-C₈-aryl or C₇-C₁₀-aralkyl, and
Y is C₃-C₆-cycloalkyl optionally fused to one or more benzene rings and optionally substituted by C₁-C₆-alkyl, C₃-C₆-cycloalkyl, C₇-C₁₀-aralkyl, C₇-C₁₀-aralkyloxy optionally substituted by halo, or by C₆-C₈-aryl optionally substituted by C₁-C₄-alkyl or C₁-C₄-alkoxy; C₆-C₈-aryl optionally substituted by halo, hydroxy, C₁-C₄-alkyl, phenoxy, C₁-C₄-alkylthio, C₆-C₈-aryl, a 4- to 8-membered heterocyclic ring having at least one ring nitrogen atom, or by NR^{b}R^{c} where R^{b} and R^{c} are each independently C₁-C₄-alkyl optionally substituted by hydroxy or phenyl or R^{b} may additionally be hydrogen; phenoxy optionally substituted by C₁-C₄-alkoxy; a 4- to 8-membered heterocyclic ring having at least one ring nitrogen or oxygen atom, said heterocyclic ring being optionally substituted by C₁-C₄-alkyl, C₆-C₈-aryl, C₇-C₁₀-aralkyl, C₁-C₄-alkoxycarbonyl or by a 4- to 8-membered heterocyclyl-C₁-C₄-alkyl; -NR^{d}R^{e} where R^{d} is hydrogen or C₁-C₄-alkyl and R^{e} is C₁-C₄-alkyl, or R^{e} is a 4- to 8-membered heterocyclic ring having at least one ring nitrogen or sulphur atom which ring is optionally substituted by halo-substituted phenyl or R^{e} is C₆-C₈-arylsulfonyl optionally substituted by di(C₁-C₄-alkyl)amino; - SR^{f} where R^{f} is C₆-C₈-aryl or C₇-C₁₀-aralkyl optionally substituted by halo or C₁-C₄-haloalkyl; or -CONHR^{g} where R^{g} is C₃-C₆-cycloalkyl or C₆-C₈-aryl.

4. A compound according to claim 1 in free or salt or solvate form, wherein
X is -R¹-Ar-R² or -R^{a}-Y;
Ar denotes a phenylene group optionally substituted by halo, hydroxy, C₁-C₁₀-alkyl, C₁-C₁₀-alkoxy, C₁-C₁₀-alkoxy-C₁-C₁₀-alkyl, phenyl, C₁-C₁₀-alkyl substituted by phenyl, C₁-C₁₀-alkoxy substituted by phenyl, C₁-C₁₀-alkyl-substituted phenyl or by C₁-C₁₀-alkoxy-substituted phenyl; R¹ and R² are attached to adjacent carbon atoms in Ar, and
either R¹ is C₁-C₁₀-alkylene and R² is hydrogen, C₁-C₁₀-alkyl, C₁-C₁₀-alkoxy or halogen or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₁-C₁₀-alkoxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₁-C₁₀-alkoxy, C₃-C₁₀-cycloalkyl, C₇-C₁₄-aralkyl, C₇-C₁₄-aralkyloxy or C₆-C₁₀-aryl optionally substituted by halo, hydroxy, C₁-C₁₀-alkyl, C₁-C₁₀-alkoxy or halo-C₁-C₁₀-alkyl;
C₆-C₁₀-aryl optionally substituted by halo, hydroxy, C₁-C₁₀-alkyl, C₁-C₁₀-alkoxy, C₁-C₁₀-haloalkyl, phenoxy, C₁-C₁₀-alkylthio, C₆-C₁₀-aryl, 4- to 10- membered heterocyclic ring having at least one ring nitrogen, oxygen or sulphur atom, or by NR^{b}R^{c} where R^{b} and R^{c} are each independently C₁-C₁₀-alkyl optionally substituted by hydroxy, C₁-C₁₀-alkoxy or phenyl or R^{b} may additionally be hydrogen;
phenoxy optionally substituted by C₁-C₁₀-alkyl, C₁-C₁₀-alkoxy or by phenyl optionally substituted by C₁-C₁₀-alkyl or C₁-C₁₀-alkoxy;
a 4- to 10-membered heterocyclic ring having at least one ring nitrogen, oxygen or sulphur atom, said heterocyclic ring being optionally substituted by halo, C₁-C₁₀-alkyl, C₁-C₁₀-alkoxy, halo-C₁-C₁₀-alkyl, C₆-C₁₀-aryl, C₇-C₁₄-aralkyl, C₇-C₁₄-aralkyloxy, C₁-C₁₀-alkoxycarbonyl or a 4- to 10-membered heterocyclyl-C₁-C₁₀-alkyl;
-NR^{d}R^{e} where R^{d} is hydrogen or C₁-C₁₀-alkyl and R^{e} is C₁-C₁₀-alkyl optionally substituted by hydroxy, or R^{e} is C₆-C₁₀-aryl optionally substituted by halo, or R^{e} is a 4-to 10-membered heterocyclic ring having at least one ring nitrogen, oxygen or sulphur atom which ring is optionally substituted by phenyl or halo-substituted phenyl or R^{e} is C₆-C₁₀-arylsulfonyl optionally substituted by C₁-C₁₀-alkylamino or di(C₁-C₁₀-alkyl)-amino;
-SR^{f} where R^{f} is C₆-C₁₀-aryl or C₇-C₁₄-aralkyl optionally substituted by halo, C₁-C₁₀-alkyl, C₁-C₁₀-alkoxy or C₁-C₁₀-haloalkyl; or
-CONHR^{g} where R^{g} is C₁-C₁₀-alkyl, C₃-C₁₀-cycloalkyl or C₆-C₁₀-aryl.

5. A compound according to claim 4, in which
X is -R¹-Ar-R² or -R^{a}-Y;
Ar denotes a phenylene group optionally substituted by halo, C₁-C₁₀-alkyl, C₁-C₁₀-alkoxy or by C₁-C₁₀-alkoxy substituted by phenyl;
R¹ and R² are attached to adjacent carbon atoms in Ar, and
either R¹ is C₁-C₁₀-alkylene and R² is hydrogen,
or R¹ and R² together with the carbon atoms in Ar to which they are attached denote a 5-, 6-or 7-membered cycloaliphatic ring;
R^{a} is a bond or C₁-C₁₀-alkylene optionally substituted by hydroxy, C₆-C₁₀-aryl or C₇-C₁₄-aralkyl; and
Y is C₃-C₁₀-cycloalkyl optionally fused to one or more benzene rings and optionally substituted by C₁-C₁₀-alkyl, C₃-C₁₀-cycloalkyl, C₇-C₁₄-aralkyl, C₇-C₁₄-aralkyloxy or C₆-C₁₀-aryl; C₆-C₁₀-aryl optionally substituted by halo, hydroxy, C₁-C₁₀-alkyl, phenoxy, C₁-C₁₀-alkylthio, C₆-C₁₀-aryl, a 4- to 10-membered heterocyclic ring having at least one ring nitrogen atom, or by NR^{b}R^{c} where R^{b} and R^{c} are each independently C₁-C₁₀-alkyl optionally substituted by hydroxy or phenyl or R^{b} may additionally be hydrogen; phenoxy optionally substituted by C₁-C₁₀-alkoxy; a 4- to 10-membered heterocyclic ring having at least one ring nitrogen or oxygen atom, said heterocyclic ring being optionally substituted by C₁-C₁₀-alkyl, C₆-C₁₀-aryl, C₇-C₁₄-aralkyl, C₁-C₁₀-alkoxycarbonyl or by a 4- to 10-membered hererocyclyl-C₁-C₁₀-alkyl; -NR^{d}R^{e} where R^{d} is hydrogen or C₁-C₁₀-alkyl and R^{e} is C₁-C₁₀-alkyl, or R^{e} is a 4- to 10-membered heterocyclic ring having at least one ring nitrogen or oxygen atom which ring is optionally substituted by halo-substituted phenyl or R^{e} is C₆-C₁₀-arylsulfonyl optionally substituted by di(C₁-C₁₀-alkyl)amino; -SR^{f} where R^{f} is C₆-C₁₀-aryl or C₇-C₁₄-aralkyl optionally substituted by halo or C₁-C₁₀-haloalkyl; or -CONHR^{g} where R^{g} is C₃-C₁₀-cycloalkyl or C₆-C₁₀-aryl.

6. A compound according to claim 4, in which
X is -R¹-Ar-R² or R^{a}-Y;
Ar denotes a phenylene group optionally substituted by halo, C₁-C₄-alkyl, C₁-C₄-alkoxy or by C₁-C₄-alkoxy substituted by phenyl;
R¹ and R² are attached to adjacent carbon atoms in Ar, and
either R¹ is C₁-C₄-alkylene and R² is hydrogen,
or R¹ and R² together with the carbon atoms in Ar to which they are attached denote a 5-, 6-or 7-membered cycloaliphatic ring, especially a 5-membered cycloaliphatic ring;
R² is a bond or C₁-C₄-alkylene optionally substituted by hydroxy, C₆-C₈-aryl or C₇-C₁₀-aralkyl; and
Y is C₃-C₆-cycloalkyl optionally fused to one or more benzene rings and optionally substituted by C₁-C₄-alkyl, C₃-C₆-cycloalkyl, C₇-C₁₀-aralkyl, C₇-C₁₀-aralkyloxy or C₆-C₈-aryl; C₆-C₈-aryl optionally substituted by halo, hydroxy, C₁-C₄-alkyl, phenoxy, C₁-C₄-alkylthio, C₆-C₈-aryl, a 4- to 8-membered heterocyclic ring having at least one ring nitrogen atom, or by NR^{b}R^{c} where R^{b} and R^{c} are each independently C₁-C₄-alkyl optionally substituted by hydroxy or phenyl or R^{b} may additionally be hydrogen; phenoxy optionally substituted by C₁-C₄-alkoxy; a 4- to 8-membered heterocyclic ring having at least one ring nitrogen or oxygen atom, said heterocyclic ring being optionally substituted by C₁-C₄-alkyl, C₆-C₈-aryl, C₇-C₁₀-aralkyl, C₁-C₄-alkoxy-carboxyl or by a 4- to 8-membered heterocyclyl-C₁-C₄-alkyl; -NR^{d}R^{e} where R^{d} is hydrogen or C₁-C₄-alkyl and R^{e} is C₁-C₄-alkyl, or R^{e} is a 4- to 8-membered heterocyclic ring having at least one ring nitrogen or sulphur atom which ring is optionally substituted by halo-substituted phenyl or R^{e} is C₆-C₈-arylsulfonyl optionally substituted by di(C₁-C₄-alkyl)amino; -SR^{f} where R^{f} is C₆-C₈-aryl or C₇-C₁₀-aralkyl optionally substituted by halo or C₁-C₄-haloalkyl; or - CONHR^{g} where R^{g} is C₃-C₆-cycloalkyl or C₆-C₈-aryl.

7. A compound according to claim 1 that is also a compound of formula II 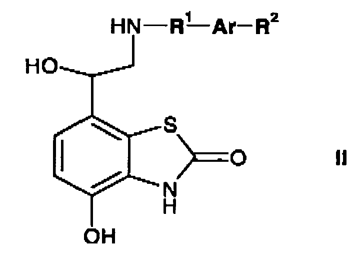 in free or salt or solvate form, where
Ar denotes a phenylene group optionally substituted by one or more substituents selected from halogen, C₁-C₈-alkyl, C₁-C₈-alkoxy, C₁-C₈-alkoxy-C₁-C₈-alkyl, or C₁-C₈-alkoxy substituted by phenyl, C₁-C₈-alkyl-substituted phenyl or by C₁-C₈-alkoxy-substituted phenyl,
R¹ and R² are attached to adjacent carbon atoms in Ar, and
either R¹ is C₁-C₈-alkylene and R² is hydrogen, C₁-C₈-alkyl, C₁-C₈-alkoxy or halogen or R¹ and R² together with the carbon atoms in Ar to which they are attached denote a 5-, 6- or 7-membered cycloaliphatic ring.

8. A compound according to claim 7 that is also a compound of formula III 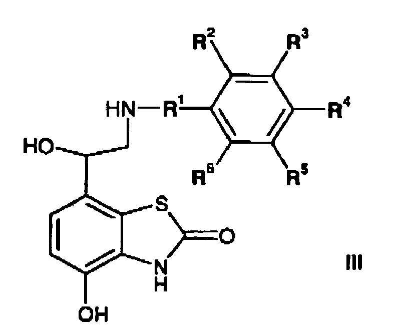 in free or salt or solvate form, where R¹ is C₂-C₄-alkylene and R² is hydrogen, or R¹ and R² together with the carbon atoms to which they are attached on the indicated benzene ring denote a 5-membered cycloaliphatic ring, R³ and R⁶ are each hydrogen, R⁴ is hydrogen, C₁-C₄-alkyl, C₁-C₄-alkoxy or C₁-C₄-alkoxy substituted by phenyl and R⁵ is hydrogen or C₁-C₄-alkyl.

9. A compound of formula I as defined in claim 1 that is selected from the group consiting of:
4-hydroxy-7-(1-hydroxy-2-[2-[4-(4-phenyl-butoxy)-phenyl]-ethylamino]-ethyl)-3H-benzothiazol-2-one,
4-hydroxy-7-[1-hydroxy-2-(indan-2-ylamino)-ethyl]-3H-benzothiazol-2-one,
4-hydroxy-7-{1-hydroxy-2-[(R)-2-(methoxy-phenyl)-1-methyl-ethylamino]-ethyl}-3H-benzothiazol-2-one,
4-hydroxy-7-[1-hydroxy-2-[2-(4-isobutyl-phenyl)-ethylamino]-ethyl]-3H-benzothiazol-2-one,
4-hydroxy-7-{1-hydroxy-2-[2-(4-propyl-phenyl)-ethylamino]-ethyl}-3H-benzothiazol-2-one,
4-hydroxy-7-{(R)-1-hydroxy-2-[2-(4-propyl-phenyl)-ethylamino]-ethyl}-3H-benzothiazol-2-one hydrochloride,
4-hydroxy-7-{(S)-1-hydroxy-2-[2-(4-propyl-phenyl)-ethylamino]-ethyl}-3H-benzothiazol-2-one hydrochloride,
7-[2-(5,6-diethyl-indan-2-ylamino)-1-hydroxy-ethyl]-4-hydroxy-3H-benzothiazol-2-one and
4-hydroxy-7-{(R)-1-hydroxy-2-[2-(3-propyl-phenyl)-ethylamino]-ethyl}-3H-benzothiazol-2-one hydrochloride.

10. A compound of formula I as defined in claim 1 that is selected from the group consiting of:
7-[(R)-2-[2-(4-Ethoxy-3-methoxy-phenyl)-ethylamino]-1-hydroxy-ethyl]-4-hydroxy-3H-benzothiazol-2-one trifluoroacetate,
7-[(R)-2-(1,1-Dimethyl-2-phenyl-ethylamino)-1-hydroxy-ethyl]-4-hydroxy-3H-benzothiazol-2-one,
7-[(R)-2-[2-(3,4-Diethyl-phenyl)-ethylamino]-1-hydroxy-ethyl]-4-hydroxy-3H-benzothiazol-2-one
4-Hydroxy-7-{(R)-1-hydroxy-2-[2-(5,6,7,8-tetrahydro-naphthalen-2-yl)-ethylamino]-ethyl}-3H-benzothiazol-2-one formate and
7-[(R)-2-((1S,2S)-2-Benzyloxy-cyclopentylamino)-1-hydroxy-ethyl]-4-hydroxy-3H-benzothiazol-2-one.

11. A compound of formula I as defined in claim 1 that is
7-[(R)-2-((1S,2R)-2-Benzyloxy-cyclopentylamino)-1-hydroxy-ethyl]-4-hydroxy-3H-benzothiazol-2-one.

12. A compound according to any preceding claim for use as a pharmaceutical.

13. A pharmaceutical composition comprising as active ingredient a compound according to any one of claims 1 to 11, optionally together with a pharmaceutically acceptable diluent or carrier therefor.

14. A pharmaceutical composition comprising as active ingredient a compound according to any one of claims 1 to 11 in combination with an anti-inflammatory, bronchodilatory or antihistamine drug substance, optionally together with a pharmaceutically acceptable diluent or carrier.

15. Use of a compound according to any one of claims 1 to 11 for the manufacture of a medicament for the treatment of a condition which is prevented or alleviated by activation of the β₂-adrenoreceptor.

16. Use of a compound according to any one of claims 1 to 11 for the manufacture of a medicament for the treatment of an obstructive or inflammatory airways disease.

17. A process for the preparation of a compound of formula I as claimed in claim 1 which comprises:
(i) either (A) reacting a compound of formula IV 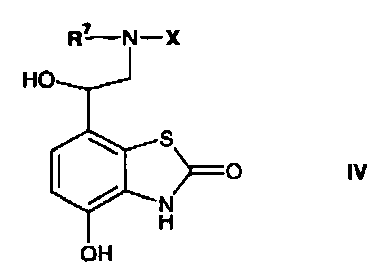 where X is as defined in Claim 1 and R⁷ denotes a protecting group, to replace R⁷ by hydrogen,
or (B) reacting a compound of formula V 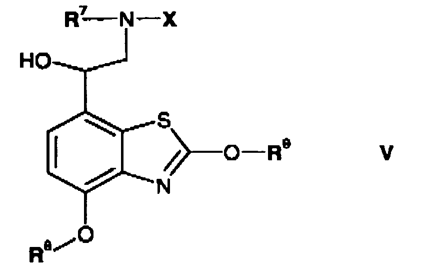 where X and R⁷ are as hereinbefore defined and R⁸ and R⁹ each independently denote a protecting group, to convert groups R⁷, R⁸ and R⁹ to hydrogen; and
(ii) recovering the compound of formula I in free or salt or solvate form.

## Patentansprüche

1. Zusammensetzung der formel I 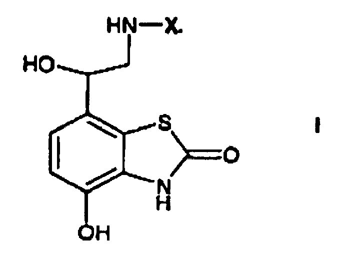 in freier oder salziger oder gelöster Form, wobei gilt:
X ist -R¹-Ar-R² oder -R^{a}-Y;
Ar bezeichnet eine Phenylen-Gruppe, wahlweise substituiert durch Halo, Hydroxy, C₁-C₁₀-Alkyl, C₁-C₁₀-Alkoxy, C₁-C₁₀-Alkoxy-C₁-C₁₀-Alkyl, Phenyl, durch Phenyl substituiertes C₁-C₁₀-Alkyl, durch Phenyl substituiertes C₁-C₁₀-Alkoxy, C₁-C₁₀-Alkyl-substituiertes Phenyl oder durch C₁-C₁₀-Alkoxy-substituiertes Phenyl;
R¹ und R² sind an benachbarte Kohlenstoffatome in Ar gebunden, und entweder ist R¹ C₁-C₁₀-Alkylene und R² Wasserstoff, C₁-C₁₀-Alkyl, C₁-C₁₀-Alkoxy oder Halogen,
oder R¹ und R² bezeichnen zusammen mit den Kohlenstoffatomen in Ar, an die sie gebunden sind, einen 5-, 6- oder 7-gliedrigen cycloaliphatischen Ring;
R^{a} ist eine Bindung oder C₁-C₁₀-Alkylen, wahlweise substituiert durch Hydroxy, C₁-C₁₀-Alkoxy, C₆-C₁₀-Aryl oder C₇-C₁₄-Aralkyl; und
Y ist C₃-C₁₀-Cycloalkyl, wahlweise fusioniert an einen oder mehrere Benzenring(e) und wahlweise substituiert durch C₁-C₁₀-Alkyl, C₁-C₁₀-Alkoxy, C₃-C₁₀-Cycloalkyl, C₇-C₁₄-Aralkyl, C₇-C₁₄-Aralkyloxy oder C₆-C₁₀-Aryl, wobei C₃-C₁₀-Cycloalkyl, C₇-C₁₄-Aralkyl, C₇-C₁₄-Aralkyloxy oder C₆-C₁₀-Aryl wahlweise substituiert sind durch Halo, Hydroxy, C₁-C₁₀-Alkyl, C₁-C₁₀-Alkoxy oder Halo-C₁-C₁₀-Alkyl;
C₆-C₁₀-Aryl, wahlweise substituiert durch Halo, Hydroxy, C₁-C₁₀-Alkyl, C₁-C₁₀-Alkoxy, C₁-C₁₀-Haloalkyl, Phenoxy, C₁-C₁₀-Alkylthio, C₆-C₁₀-Aryl, einen 4- bis 10-gliedrigen heterozyklischen Ring mit mindestens einem Ring Stickstoff-, Sauerstoff- oder Schwefelatom, oder durch NR^{b}R^{c}, wobei R^{b} und R^{c} jeweils unabhängig C₁-C₁₀-Alkyl, wahlweise substituiert durch Hydroxy, C₁-C₁₀-Alkoxy oder Phenyl ist oder R^{b} zusätzlich Wasserstoff sein kann;
Phenoxy, wahlweise substituiert durch C₁-C₁₀-Alkyl, C₁-C₁₀-Alkoxy oder durch Phenyl, wahlweise substituiert durch C₁-C₁₀-Alkyl oder C₁-C₁₀-Alkoxy;
ein 4- bis 10-gliedriger heterozyklischer Ring mit mindestens einem Ring Stickstoff-, Sauerstoff- oder Schwefelatom, wobei der heterozyklische Ring wahlweise substituiert ist durch Halo, C₁-C₁₀-Alkyl, C₁-C₁₀-Alkoxy, Halo-C₁-C₁₀-Alkyl, C₆-C₁₀-Aryl, C₇-C₁₄-Aralkyl, C₇-C₁₄-Aralkyloxy, C₁-C₁₀-Alkoxycarbonyl oder ein 4- bis 10-gliedriges Heterocyclyl- C₁-C₁₀-Alkyl;
-NR^{d}R^{e}, wobei R^{d} Wasserstoff oder C₁-C₁₀-Alkyl ist und R^{e} C₁-C₁₀-Alkyl, wahlweise substituiert durch Hydroxy ist, oder R^{e} C₆-C₁₀-Aryl, wahlweise substituiert durch Halo ist, oder R^{e} ein 4- bis 10-gliedriger heterozyklischer Ring mit mindestens einem Ring Stickstoff-, Sauerstoff- oder Schwefelatom ist, wobei der Ring wahlweise substituiert ist durch Phenyl oder mit Halo substituiertes Phenyl, oder R^{e} C₆-C₁₀-Arylsulfonyl, wahlweise substituiert durch C₁-C₁₀-Alkylamino oder di(C₁-C₁₀-Alkyl)Amino ist;
-SR^{f}, wobei R^{f} C₆-C₁₀-Aryl oder C₇-C₁₄-Aralkyl, wahlweise substituiert durch Halo, C₁-C₁₀-Alkyl, C₁-C₁₀-Alkoxy oder C₁-C₁₀-Haloalkyl ist; oder
-CONHR^{g}, wobei R^{g} C₁-C₁₀-Alkyl, C₃-C₁₀-Cycloalkyl oder C₆-C₁₀-Aryl ist.

2. Zusammensetzung nach Anspruch 1, wobei gilt:
X ist -R¹-Ar-R² oder -R^{a}-Y;
Ar bezeichnet eine Phenylen-Gruppe, wahlweise substituiert durch Halo, C₁-C₁₀-Alkyl, C₁-C₁₀-Alkoxy oder durch mit Phenyl substituiertes C₁-C₁₀-Alkoxy;
R¹ und R² sind an benachbarte Kohlenstoffatome in Ar gebunden, und
entweder ist R¹ C₁-C₁₀-Alkylene und R² Wasserstoff,
oder R¹ und R² bezeichnen zusammen mit den Kohlenstoffatomen in Ar, an die sie gebunden sind, einen 5-, 6- oder 7-gliedrigen cycloaliphatischen Ring;
R^{a} ist eine Bindung oder C₁-C₁₀-Alkylen, wahlweise substituiert durch Hydroxy, C₆-C₁₀-Aryl oder C₇-C₁₄-Aralkyl; und
Y ist C₃-C₁₀-Cycloalkyl wahlweise fusioniert an einen oder mehrere Benzenring(e) und wahlweise substituiert durch C₁-C₁₀-Alkyl, C₃-C₁₀-Cycloalkyl, C₇-C₁₄-Aralkyl, C₇-C₁₄-Aralkyloxy, wahlweise substituiert durch Halo, oder durch C₆-C₁₀-Aryl, wahlweise substituiert durch C₁-C₁₀-Alkyl oder C₁-C₁₀-Alkoxy; C₆-C₁₀-Aryl, wahlweise substituiert durch Halo, Hydroxy, C₁-C₁₀-Alkyl, Phenoxy, C₁-C₁₀-Alkylthio, C₆-C₁₀-Aryl, einen 4- bis 10-gliedrigen heterozyklischen Ring mit mindestens einem Ring Stickstoffatom, oder durch NR^{b}R^{c}, wobei R^{b} und R^{c} jeweils unabhängig C₁-C₁₀-Alkyl, wahlweise substituiert durch Hydroxy oder Phenyl ist oder R^{b} zusätzlich Wasserstoff sein kann; Phenoxy, wahlweise substituiert durch C₁-C₁₀-Alkoxy; ein 4- bis 10-gliedriger heterozyklischer Ring mit mindestens einem Ring Stickstoff- oder Sauerstoffatom, wobei der heterozyklische Ring wahlweise substituiert ist durch C₁-C₁₀-Alkyl, C₆-C₁₀-Aryl, C₇-C₁₄-Aralkyl, C₁-C₁₀-Alkoxycarbonyl oder ein 4- bis 10-gliedriges Heterocyclyl-C₁-C₁₀-Alkyl; -NR^{d}R^{e}, wobei R^{d} Wasserstoff oder C₁-C₁₀-Alkyl ist und R^{e} C₁-C₁₀-Alkyl ist oder R^{e} ein 4- bis 10-gliedriger heterozyklischer Ring mit mindestens einem Ring Stickstoff- oder Sauerstoffatom ist, wobei der Ring wahlweise substituiert wird durch mit Halo substituiertes Phenyl, oder R^{e} C₆-C₁₀-Arylsulfonyl, wahlweise substituiert durch di(C₁-C₁₀-Alkyl)Amino ist; -SR^{f}, wobei R^{f} C₆-C₁₀-Aryl oder C₇-C₁₄-Aralkyl, wahlweise substituiert durch Halo oder C₁-C₁₀-Haloalkyl ist; oder -CONHR^{g}, wobei R^{g} C₃-C₁₀-Cycloalkyl oder C₆-C₁₀-Aryl ist.

3. Zusammensetzung nach Anspruch 2, wobei gilt:
X ist -R¹-Ar-R² oder -R^{a}-Y;
Ar bezeichnet eine Phenylen-Gruppe, wahlweise substituiert durch Halo, C₁-C₄-Alkyl, C₁-C₄-Alkoxy oder durch mit Phenyl substituiertes C₁-C₄-Alkoxy;
R¹ und R² sind an benachbarte Kohlenstoffatome in Ar gebunden, und entweder ist R¹ C₁-C₄-Alkylene und R² Wasserstoff,
oder R¹ und R² bezeichnen zusammen mit den Kohlenstoffatomen in Ar, an die sie gebunden sind, einen 5-, 6- oder 7-gliedrigen cycloaliphatischen Ring, insbesondere einen 5-gliedrigen cycloaliphatischen Ring;
R^{a} ist eine Bindung oder C₁-C₄-Alkylen, wahlweise substituiert durch Hydroxy, C₆-C₈-Aryl oder C₇-C₁₀-Aralkyl; und
Y ist C₃-C₆-Cycloalkyl, wahlweise fusioniert an einen oder mehrere Benzenring(e) und wahlweise substituiert durch C₁-C₆-Alkyl, C₃-C₆-Cycloalkyl, C₇-C₁₀-Aralkyl, C₇-C₁₀-Aralkyloxy, wahlweise substituiert durch Halo, oder durch C₆-C₈-Aryl, wahlweise substituiert durch C₁-C₄-Alkyl oder C₁-C₄-Alkoxy; C₆-C₈-Aryl wahlweise substituiert durch Halo, Hydroxy, C₁-C₄-Alkyl, Phenoxy, C₁-C₄-Alkylthio, C₆-C₈-Aryl, einen 4- bis 8-gliedrigen heterozyklischen Ring mit mindestens einem Ring Stickstoffatom, oder durch NR^{b}R^{c}, wobei R^{b} und R^{c} jeweils unabhängig C₁-C₄-Alkyl, wahlweise substituiert durch Hydroxy oder Phenyl ist oder R^{b} zusätzlich Wasserstoff sein kann; Phenoxy, wahlweise substituiert durch C₁-C₄-Alkoxy; ein 4- bis 8-gliedriger heterozyklischer Ring mit mindestens einem Ring Stickstoff- oder Sauerstoffatom, wobei der heterozyklische Ring wahlweise substituiert ist durch C₁-C₄-Alkyl, C₆-C₈-Aryl, C₇-C₁₀-Aralkyl, C₁-C₄-Alkoxycarbonyl oder ein 4- bis 8-gliedriges Heterocyclyl-C₁-C₄-Alkyl; -NR^{d}R^{e}, wobei R^{d} Wasserstoff oder C₁-C₄-Alkyl ist und R^{e} C₁-C₄-Alkyl ist oder R^{e} ein 4- bis 8-gliedriger heterozyklischer Ring mit mindestens einem Ring Stickstoff- oder Schwefelatom ist, wobei der Ring wahlweise substituiert ist durch mit Halo substituiertes Phenyl, oder R^{e} C₆-C₈-Arylsulfonyl, wahlweise substituiert durch di(C₁-C₁₀-Alkyl)Amino ist; -SR^{f}, wobei R^{f} C₆-C₈-Aryl oder C₇-C₁₀-Aralkyl, wahlweise substituiert durch Halo oder C₁-C₄-Haloalkyl ist; oder -CONHR^{g}, wobei R^{g} C₃-C₆-Cycloalkyl oder C₆-C₈-Aryl ist.

4. Zusammensetzung nach Anspruch 1 in freier oder salziger oder gelöster Form, wobei gilt
X ist -R¹-Ar-R² oder -R^{a}-Y;
Ar bezeichnet eine Phenylen-Gruppe, wahlweise substituiert durch Halo, Hydroxy, C₁-C₁₀-Alkyl, C₁-C₁₀-Alkoxy, C₁-C₁₀-Alkoxy-C₁-C₁₀-Alkyl, Phenyl, durch Phenyl substituiertes C₁-C₁₀-Alkyl, durch Phenyl substituiertes C₁-C₁₀-Alkoxy, C₁-C₁₀-Alkyl-substituiertes Phenyl oder durch C₁-C₁₀-Alkoxy-substituiertes Phenyl;
R¹ und R² sind an benachbarte Kohlenstoffatome in Ar gebunden, und
entweder ist R¹ C₁-C₁₀-Alkylene und R² Wasserstoff, C₁-C₁₀-Alkyl, C₁-C₁₀-Alkoxy oder Halogen,
oder R¹ und R² bezeichnen zusammen mit den Kohlenstoffatomen in Ar, an die sie gebunden sind, einen 5-, 6- oder 7-gliedrigen cycloaliphatischen Ring;
R^{a} ist eine Bindung oder C₁-C₁₀-Alkylen, wahlweise substituiert durch Hydroxy, C₁-C₁₀-Alkoxy, C₆-C₁₀-Aryl oder C₇-C₁₄-Aralkyl; und
Y ist C₃-C₁₀-Cycloalkyl, wahlweise fusioniert an einen oder mehrere Benzenring(e) und wahlweise substituiert durch C₁-C₁₀-Alkyl, C₁-C₁₀-Alkoxy, C₃-C₁₀-Cycloalkyl, C₇-C₁₄-Aralkyl, C₇-C₁₄-Aralkyloxy oder C₆-C₁₀-Aryl, wahlweise substituiert durch Halo, Hydroxy, C₁-C₁₀-Alkyl, C₁-C₁₀-Alkoxy oder Halo-C₁-C₁₀-Alkyl;
C₆-C₁₀-Aryl wahlweise substituiert durch Halo, Hydroxy, C₁-C₁₀-Alkyl, C₁-C₁₀-Alkoxy, C₁-C₁₀-Haloalkyl, Phenoxy, C₁-C₁₀-Alkylthio, C₆-C₁₀-Aryl, einen 4- bis 10-gliedrigen heterozyklischen Ring mit mindestens einem Ring Stickstoff-, Sauerstoff- oder Schwefelatom, oder durch NR^{b}R^{c}, wobei R^{b} und R^{c} jeweils unabhängig C₁-C₁₀-Alkyl, wahlweise substituiert durch Hydroxy, C₁-C₁₀-Alkoxy oder Phenyl ist oder R^{b} zusätzlich Wasserstoff sein kann;
Phenoxy, wahlweise substituiert durch C₁-C₁₀-Alkyl, C₁-C₁₀-Alkoxy oder durch Phenyl, wahlweise substituiert durch C₁-C₁₀-Alkyl oder C₁-C₁₀-Alkoxy;
ein 4- bis 10-gliedriger heterozyklischer Ring mit mindestens einem Ring Stickstoff-, Sauerstoff- oder Schwefelatom, wobei der heterozyklische Ring wahlweise substituiert ist durch Halo, C₁-C₁₀-Alkyl, C₁-C₁₀-Alkoxy, Halo-C₁-C₁₀-Alkyl, C₆-C₁₀-Aryl, C₇-C₁₄-Aralkyl, C₇-C₁₄-Aralkyloxy, C₁-C₁₀-Alkoxycarbonyl oder ein 4- bis 10-gliedriges Heterocyclyl- C₁-C₁₀-Alkyl;
-NR^{d}R^{e}, wobei R^{d} Wasserstoff oder C₁-C₁₀-Alkyl ist und R^{e} C₁-C₁₀-Alkyl, wahlweise substituiert durch Hydroxy ist, oder R^{e} C₆-C₁₀-Aryl, wahlweise substituiert durch Halo ist, oder R^{e} ein 4- bis 10-gliedriger heterozyklischer Ring mit mindestens einem Ring Stickstoff-, Sauerstoff- oder Schwefelatom ist, wobei der Ring wahlweise substituiert ist durch Phenyl oder mit Halo substituiertes Phenyl, oder R^{e} C₆-C₁₀-Arylsulfonyl, wahlweise substituiert durch C₁-C₁₀-Alkylamino oder di(C₁-C₁₀-Alkyl)Amino ist;
-SR^{f}, wobei R^{f} C₆-C₁₀-Aryl oder C₇-C₁₄-Aralkyl, wahlweise substituiert durch Halo, C₁-C₁₀-Alkyl, C₁-C₁₀-Alkoxy oder C₁-C₁₀-Haloalkyl ist; oder
-CONHR^{g}, wobei R^{g} C₁-C₁₀-Alkyl, C₃-C₁₀-Cycloalkyl oder C₆-C₁₀-Aryl ist.

5. Zusammensetzung nach Anspruch 4, wobei gilt:
X ist -R¹-Ar-R² oder -R^{a}-Y;
Ar bezeichnet eine Phenylen-Gruppe, wahlweise substituiert durch Halo, C₁-C₁₀-Alkyl, C₁-C₁₀-Alkoxy oder durch mit Phenyl substituiertes C₁-C₁₀-Alkoxy;
R¹ und R² sind an benachbarte Kohlenstoffatome in Ar gebunden, und entweder ist R¹ C₁-C₁₀-Alkylene und R² Wasserstoff,
oder R¹ und R² bezeichnen zusammen mit den Kohlenstoffatomen in Ar, an die sie gebunden sind, einen 5-, 6- oder 7-gliedrigen cycloaliphatischen Ring;
R^{a} ist eine Bindung oder C₁-C₁₀-Alkylen, wahlweise substituiert durch Hydroxy, C₆-C₁₀-Aryl oder C₇-C₁₄-Aralkyl; und
Y ist C₃-C₁₀-Cycloalkyl, wahlweise fusioniert an einen oder mehrere Benzenring(e) und wahlweise substituiert durch C₁-C₁₀-Alkyl, C₃-C₁₀-Cycloalkyl, C₇-C₁₄-Aralkyl, C₇-C₁₄-Aralkyloxy oder C₆-C₁₀-Aryl; C₆-C₁₀-Aryl, wahlweise substituiert durch Halo, Hydroxy, C₁-C₁₀-Alkyl, Phenoxy, C₁-C₁₀-Alkylthio, C₆-C₁₀-Aryl, einen 4- bis 10-gliedrigen heterozyklischen Ring mit mindestens einem Ring Stickstoffatom, oder durch NR^{b}R^{c}, wobei R^{b} und R^{c} jeweils unabhängig C₁-C₁₀-Alkyl, wahlweise substituiert durch Hydroxy oder Phenyl ist oder R^{b} zusätzlich Wasserstoff sein kann; Phenoxy, wahlweise substituiert durch C₁-C₁₀-Alkoxy; ein 4- bis 10-gliedriger heterozyklischer Ring mit mindestens einem Ring Stickstoff- oder Sauerstoffatom, wobei der heterozyklische Ring wahlweise substituiert ist durch C₁-C₁₀-Alkyl, C₆-C₁₀-Aryl, C₇-C₁₄-Aralkyl, C₁-C₁₀-Alkoxycarbonyl oder ein 4- bis 10-gliedriges Heterocyclyl-C₁-C₁₀-Alkyl; -NR^{d}R^{e}, wobei R^{d} Wasserstoff oder C₁-C₁₀-Alkyl ist und R^{e} C₁-C₁₀-Alkyl ist oder R^{e} ein 4- bis 10-gliedriger heterozyklischer Ring mit mindestens einem Ring Stickstoff- oder Sauerstoffatom ist, wobei der Ring wahlweise substituiert wird durch mit Halo substituiertes Phenyl, oder R^{e} C₆-C₁₀-Arylsulfonyl, wahlweise substituiert durch di(C₁-C₁₀-Alkyl)Amino ist; -SR^{f}, wobei R^{f} C₆-C₁₀-Aryl oder C₇-C₁₄-Aralkyl, wahlweise substituiert durch Halo oder C₁-C₁₀-Haloalkyl ist; oder -CONHR^{g}, wobei R^{g} C₃-C₁₀-Cycloalkyl oder C₆-C₁₀-Aryl ist.

6. Zusammensetzung nach Anspruch 4, wobei gilt:
X ist -R¹-Ar-R² oder -R^{a}-Y;
Ar bezeichnet eine Phenylen-Gruppe, wahlweise substituiert durch Halo, C₁-C₄-Alkyl, C₁-C₄-Alkoxy oder durch mit Phenyl substituiertes C₁-C₄-Alkoxy;
R¹ und R² sind an benachbarte Kohlenstoffatome in Ar gebunden, und entweder ist R¹ C₁-C₄-Alkylene und R² Wasserstoff,
oder R¹ und R² bezeichnen zusammen mit den Kohlenstoffatomen in Ar, an die sie gebunden sind, einen 5-, 6- oder 7-gliedrigen cycloaliphatischen Ring, insbesondere einen 5-gliedrigen cycloaliphatischen Ring;
R^{a} ist eine Bindung oder C₁-C₄-Alkylen, wahlweise substituiert durch Hydroxy, C₆-C₈-Aryl oder C₇-C₁₀-Aralkyl; und
Y ist C₃-C₆-Cycloalkyl, wahlweise fusioniert an einen oder mehrere Benzenring(e) und wahlweise substituiert durch C₁-C₄-Alkyl, C₃-C₆-Cycloalkyl, C₇-C₁₀-Aralkyl, C₇-C₁₀-Aralkyloxy oder C₆-C₈-Aryl; C₆-C₈-Aryl, wahlweise substituiert durch Halo, Hydroxy, C₁-C₄-Alkyl, Phenoxy, C₁-C₄-Alkylthio, C₆-C₈-Aryl, einen 4- bis 8-gliedrigen heterozyklischen Ring mit mindestens einem Ring Stickstoffatom, oder durch NR^{b}R^{c}, wobei R^{b} und R^{c} jeweils unabhängig C₁-C₄-Alkyl, wahlweise substituiert durch Hydroxy oder Phenyl ist oder R^{b} zusätzlich Wasserstoff sein kann; Phenoxy, wahlweise substituiert durch C₁-C₄-Alkoxy; ein 4- bis 8-gliedriger heterozyklischer Ring mit mindestens einem Ring Stickstoff- oder Sauerstoffatom, wobei der heterozyklische Ring wahlweise substituiert ist durch C₁-C₄-Alkyl, C₆-C₈-Aryl, C₇-C₁₀-Aralkyl, C₁-C₄-Alkoxycarbonyl oder ein 4- bis 8-gliedriges Heterocyclyl-C₁-C₄-Alkyl; -NR^{d}R^{e}, wobei R^{d} Wasserstoff oder C₁-C₄-Alkyl ist und R^{e} C₁-C₄-Alkyl ist oder R^{e} ein 4- bis 8-gliedriger heterozyklischer Ring mit mindestens einem Ring Stickstoff- oder Schwefelatom ist, wobei der Ring wahlweise substituiert ist durch mit Halo substituiertes Phenyl, oder R^{e} C₆-C₈-Arylsulfonyl, wahlweise substituiert durch di(C₁-C₄-Alkyl)Amino ist; -SR^{f}, wobei R^{f} C₆-C₈-Aryl oder C₇-C₁₀-Aralkyl, wahlweise substituiert durch Halo oder C₁-C₄-Haloalkyl ist; oder -CONHR^{g}, wobei R^{g} C₃-C₆-Cycloalkyl oder C₆-C₈-Aryl ist.

7. Zusammensetzung nach Anspruch 1, die auch eine Zusammensetzung der Formel II ist 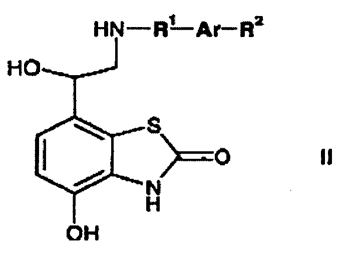 in freier oder salziger oder gelöster Form, wobei gilt
Ar bezeichnet eine Phenylengruppe, wahlweise substituiert durch einen oder mehrere Substituenten, der/die aus Halogen, C₁-C₈-Alkyl, C₁-C₈-Alkoxy, C₁-C₈-Alkoxy-C₁-C₈-Alkyl oder durch Phenyl substituiertes C₁-C₈-Alkoxy, C₁-C₈-Alkyl-substituiertes Phenyl oder durch C₁-C₈-Alkyl-substituiertes Phenyl ausgewählt ist/sind,
R¹ und R² sind an benachbarte Kohlenstoffatome in Ar gebunden, und entweder ist R¹ C₁-C₈-Alkylene und R² Wasserstoff, C₁-C₈-Alkyl, C₁-C₈-Alkoxy oder Halogen, oder R¹ und R² bezeichnen zusammen mit den Kohlenstoffatomen in Ar, an die sie gebunden sind, einen 5-, 6- oder 7-gliedrigen cycloaliphatischen Ring.

8. Zusammensetzung nach Anspruch 7, die auch eine Zusammensetzung der Formel III ist 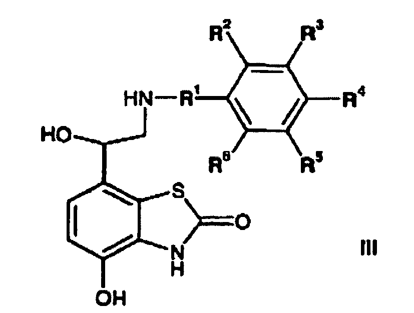 in freier oder salziger oder gelöster Form, wobei R¹ C₂-C₄-Alkylen und R² Wasserstoff ist oder R¹ und R² zusammen mit den Kohlenstoffatomen, an die sie in dem dargestellten Benzenring gebunden sind, einen 5-gliedrigen cycloaliphatischen Ring bezeichnen, R³ und R⁶ beide Wasserstoff sind, R⁴ Wasserstoff, C₁-C₄-Alkyl, C₁-C₄-Alkoxy oder C₁-C₄-Alkoxy substituiert durch Phenyl ist und R⁵ Wasserstoff oder C₁-C₄-Alkyl ist.

9. Zusammensetzung der Formel I wie in Anspruch 1 definiert, die ausgewählt ist aus der Gruppe bestehend aus:
4-Hydroxy-7-(1-Hydroxy-2-[2-[4-(4-Phenyl-Butoxy)-Phenyl]-Ethylamino]-Ethyl)-3H-Benzothiazol-2-on,
4-hydroxy-7-[1-Hydroxy-2-(Indan-2-Ylamino)-Ethyl]-3H-Benzothiazol-2-on,
4-Hydroxy-7-{1-Hydroxy-2-[(R)-2-(4-Methoxy-Phenyl)-1-Methyl-Ethylamino]-Ethyl}-3H-Benzothiazol-2-on,
4-Hydroxy-7-{1-Hydroxy-2-[2-(4-Isobutyl-Phenyl)-Ethylamino]-Ethyl}-3H-Benzothiazol-2-on,
4-Hydroxy-7-{1-Hydroxy-2-[2-(4-Propyl-Phenyl)-Ethylamino]-Ethyl}-3H-Benzothiazol-2-on,
4-Hydroxy-7-{(R)-1-Hydroxy-2-[2-(4-Propyl-Phenyl)-Ethylamino]-Ethyl}-3H-Benzothiazol-2-on-Hydrochlorid,
4-Hydroxy-7-{(S)-1-Hydroxy-2-[2-(4-Propyl-Phenyl)-Ethylamino]-Ethyl}-3H-Benzothiazol-2-on-Hydrochlorid,
7-[2-(5,6-Diethyl-Indan-2-Ylamino)-1-Hydroxy-Ethyl]-4-Hydroxy-3H-Benzothiazol-2-on und 4-Hydroxy-7-{(R)-1-Hydroxy-2-[2-(3-Propyl-Phenyl)-Ethylamino]-Ethyl}-3H-Benzothiazol-2-on-Hydrochlorid.

10. Zusammensetzung der Formel I wie in Anspruch 1 definiert, die ausgewählt ist aus der Gruppe bestehend aus:
7-{(R)-2-[2-(4-Ethoxy-3-Methoxy-Phenyl)-Ethylamino]-1-Hydroxy-Ethyl}-4-Hydroxy-3H-Benzothiazol-2-on-Trifluoroacetat,
7-[(R)-2-(1,1-Dimethyl-2-Phenyl-Ethylamino)-1-Hydroxy-Ethyl]-4-Hydroxy-3H-Benzothiazol-2-on,
7-{(R)-2-[2-(3,4-Diethyl-Phenyl)-Ethylamino]-1-Hydroxy-Ethyl}-4-Hydroxy-3H-Benzothiazol-2-on
4-Hydroxy-7-{(R)-1-Hydroxy-2-[2-(5,6,7,8-Tetrahydro-Naphthalen-2-yl)-Ethylamino]-Ethyl}-3H-Benzothiazol-2-on-Format und
7-[(R)-2-((1S,2S)-2-Benzyloxy-Cyclopentylamino)-1-Hydroxy-Ethyl]-4-Hydroxy-3H-Benzothiazol-2-on.

11. Zusammensetzung der Formel I wie in Anspruch 1 definiert, die 7-[(R)-2-((1S,2R)-2-Benzyloxy-Cyclopentylamino)-1-Hydroxy-Ethyl]-4-Hydroxy-3H-Benzothiazol-2-on ist.

12. Zusammensetzung nach einem der vorstehenden Ansprüche zur Verwendung als Arzneimittel.

13. Pharmazeutische Zusammensetzung, die als aktiven Bestandteil eine Zusammensetzung nach einem der Ansprüche 1 bis 11 enthält, wahlweise zusammen mit einem pharmazeutisch zulässigen Lösungsmittel oder Trägerstoff dafür.

14. Pharmazeutische Zusammensetzung, die als aktiven Bestandteil eine Zusammensetzung nach einem der Ansprüche 1 bis 11 in Kombination mit einer entzündungshemmenden, bronchodilatorischen oder Antihistamin-Arzneimittelsubstanz enthält, wahlweise zusammen mit einem pharmazeutisch zulässigen Lösungsmittel oder Trägerstoff.

15. Verwendung einer Zusammensetzung nach einem der Ansprüche 1 bis 11 zur Herstellung eines Arzneimittels für die Behandlung eines Zustands, der durch Aktivierung des β₂-Adrenorezeptors verhindert oder gemildert wird.

16. Verwendung einer Zusammensetzung nach einem der Ansprüche 1 bis 11 zur Herstellung eines Arzneimittels für die Behandlung einer obstruktiven oder entzündlichen Atemwegserkrankung.

17. Verfahren zur Herstellung einer Zusammensetzung der Formel I wie in Anspruch 1 angegeben, das umfasst:
(i) entweder (A) eine Zusammensetzung der Formel IV zur Reaktion zu bringen 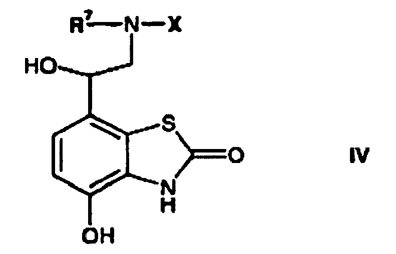 wobei X der Definition in Anspruch 1 entspricht und R⁷ eine schützende Gruppe bezeichnet, um R⁷ durch Wasserstoff zu ersetzen,
oder (B) eine Zusammensetzung der Formel V zur Reaktion zu bringen 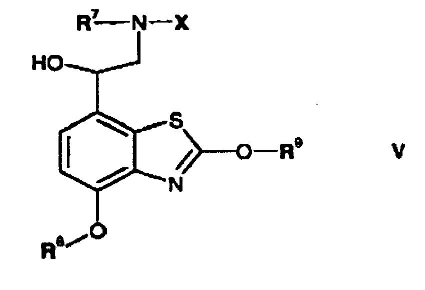 wobei X und R⁷ der vorstehenden Definition entsprechen und R⁸ und R⁹ jeweils unabhängig eine schützende Gruppe bezeichnen, um die Gruppen R⁷, R⁸ und R⁹ in Wasserstoff umzuwandeln; und
(ii) die Zusammensetzung der Formel 1 in freier, salziger oder gelöster Form zu gewinnen.

## Revendications

1. Composé selon la formule I 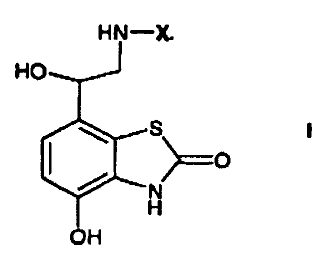 sous forme libre, de sel ou de solvate, où
X est -R¹-Ar-R² ou -R^{a}-Y,
Ar désigne un groupe phénylène optionnellement substitué par halo, hydroxy, C₁-C₁₀-alkyle, C₁-C₁₀-alkoxy, C₁-C₁₀-alkoxy-C₁-C₁₀-alkyle, phényle, C₁-C₁₀-alkyle substitué par phényle, C₁-C₁₀-alkoxy substitué par phényle, C₁-C₁₀-alkyle-phényle substitué ou par C₁-C₁₀-alkoxy-phényle substitué,
R¹ et R² sont attachés à des atomes de carbone adjacents dans Ar, et
soit R¹ est C₁-C₁₀-alkylène et R² est hydrogène, C₁-C₁₀-alkyle, C₁-C₁₀-alkoxy ou halogène soit R¹ et R² conjointement avec les atomes de carbone dans Ar auxquels ils sont attachés désignent un anneau cycloaliphatique à 5, 6 ou 7 chaînons,
R^{a} est une liaison ou C₁-C₁₀-alkylène optionnellement substitué par hydroxy, C₁-C₁₀-alkoxy, C₆-C₁₀-aryle ou C₇-C₁₄-aralkyle, et
Y est C₃-C₁₀-cycloalkyle optionnellement fusionné à un ou plusieurs anneaux benzéniques et optionnellement substitué par C₁-C₁₀-alkyle, C₁-C₁₀-alkoxy, C₃-C₁₀-cycloalkyle, C₇-C₁₄-aralkyle, C₇-C₁₄-aralkyloxy ou C₆-C₁₀-aryle, où C₃-C₁₀-cycloalkyle, C₇-C₁₄-aralkyle, C₇-C₁₄-aralkyloxy ou C₆-C₁₀-aryle sont optionnellement substitués par halo, hydroxy, C₁-C₁₀-alkyle, C₁-C₁₀-alkoxy ou halo-C₁-C₁₀-alkyle,
C₆-C₁₀-aryle optionnellement substitué par halo, hydroxy, C₁-C₁₀-alkyle, C₁-C₁₀-alkoxy, C₁-C₁₀-haloalkyle, phénoxy, C₁-C₁₀-alkylthio, C₆-C₁₀-aryle, un anneau hétérocyclique de 4 à 10 chaînons possédant au moins un atome de soufre, d'oxygène ou d'azote d'anneau, ou par NR^{b}R^{c} où R^{b} et R^{c} sont chacun indépendamment C₁-C₁₀-alkyle optionnellement substitué par hydroxy, C₁-C₁₀-alkoxy ou phényle ou R^{b} peut additionnellement être hydrogène,
phénoxy optionnellement substitué par C₁-C₁₀-alkyle, C₁-C₁₀-alkoxy ou par phényle optionnellement substitué par C₁-C₁₀-alkyle ou C₁-C₁₀-alkoxy,
un anneau hétérocyclique de 4 à 10 chaînons possédant au moins un atome de soufre, d'oxygène ou d'azote d'anneau, ledit anneau hétérocyclique étant optionnellement substitué par halo, C₁-C₁₀-alkyle, C₁-C₁₀-alkoxy, halo-C₁-C₁₀-alkyle, C₆-C₁₀-aryle, C₇-C₁₄-aralkyle, C₇-C₁₄-aralkyloxy, C₁-C₁₀-alkoxycarbonyl ou un hétérocyclyle-C₁-C₁₀-alkyle de 4 à 10 chaînons,
-NR^{d}R^{e} où R^{d} est hydrogène ou C₁-C₁₀-alkyle et R^{e} est C₁-C₁₀-alkyle optionnellement substitué par hydroxy, ou R^{e} est C₆-C₁₀-aryle optionnellement substitué par halo, ou R^{e} est un anneau hétérocyclique de 4 à 10 chaînons possédant au moins un atome de soufre, d'oxygène ou d'azote d'anneau dont l'anneau est optionnellement substitué par phényle ou phényle à substitution halo ou R^{e} est C₆-C₁₀-arylsulfonyle optionnellement substitué par C₁-C₁₀-alkylamino ou di(C₁-C₁₀-alkyle)amino,
-SR^{f} où R^{f} est C₆-C₁₀-aryle ou C₇-C₁₄-aralkyle optionnellement substitué par halo, C₁-C₁₀-alkyle, C₁-C₁₀-alkoxy ou C₁-C₁₀-haloalkyle, ou
-CONHR^{g} où R^{g} est C₁-C₁₀-alkyle, C₃-C₁₀-cycloalkyle ou C₆-C₁₀-aryle.

2. Composé selon la revendication 1, dans lequel
X est -R¹-Ar-R² ou -R^{a}-Y,
Ar désigne un groupe phénylène optionnellement substitué par halo, C₁-C₁₀-alkyle, C₁-C₁₀-alkoxy ou par C₁-C₁₀-alkoxy substitué par phényle,
R¹ et R² sont attachés à des atomes de carbone adjacents dans Ar, et
soit R¹ est C₁-C₁₀-alkylène et R² est hydrogène,
soit R¹ et R² conjointement avec les atomes de carbone dans Ar auxquels ils sont attachés désignent un anneau cycloaliphatique à 5, 6 ou 7 chaînons,
R^{a} est une liaison ou C₁-C₁₀-alkylène optionnellement substitué par hydroxy, C₆-C₁₀-aryle ou C₇-C₁₄-aralkyle, et
Y est C₃-C₁₀-cycloalkyle optionnellement fusionné à un ou plusieurs anneaux benzéniques et optionnellement substitué par C₁-C₁₀-alkyle, C₃-C₁₀-cycloalkyle, C₇-C₁₄-aralkyle, C₇-C₁₄-aralkyloxy optionnellement substitué par halo, ou par C₆-C₁₀-aryle optionnellement substitué par C₁-C₁₀-alkyle ou C₆-C₁₀-alkoxy, C₆-C₁₀-aryle optionnellement substitué par halo, hydroxy, C₁-C₁₀-alkyle, phénoxy, C₁-C₁₀-alkylthio, C₆-C₁₀-aryle, un anneau hétérocyclique de 4 à 10 chaînons possédant au moins un atome d'azote d'anneau, ou par NR^{b}R^{c} où R^{b} et R^{c} sont chacun indépendamment C₁-C₁₀-alkyle optionnellement substitué par hydroxy ou phényle ou R^{b} peut additionnellement être hydrogène, phénoxy optionnellement substitué par C₁-C₁₀-alkoxy, un anneau hétérocyclique de 4 à 10 chaînons possédant au moins un atome d'oxygène ou d'azote d'anneau, ledit anneau hétérocyclique étant optionnellement substitué par C₁-C₁₀-alkyle, C₆-C₁₀-aryle, C₇-C₁₄-aralkyle, C₁-C₁₀-alkoxycarbonyl ou par un hétérocyclyle-C₁-C₁₀-alkyle de 4 à 10 chaînons, -NR^{d}R^{e} où R^{d} est hydrogène ou C₁-C₁₀-alkyle et R^{e} est C₁-C₁₀-alkyle, ou R^{e} est un anneau hétérocyclique de 4 à 10 chaînons possédant au moins un atome d'oxygène ou d'azote d'anneau dont l'anneau est optionnellement substitué par phényle à substitution halo ou R^{e} est C₆-C₁₀-arylsulfonyle optionnellement substitué par di(C₁-C₁₀-alkyle)amino, - SR^{f} où R^{f} est C₆-C₁₀-aryle ou C₇-C₁₄-aralkyle optionnellement substitué par halo ou C₁-C₁₀-haloalkyle, ou -CONHR^{g} où R^{g} est C₃-C₁₀-cycloalkyle ou C₆-C₁₀-aryle.

3. Composé selon la revendication 2, dans lequel
X est -R¹-Ar-R² ou -R^{a}-Y,
Ar désigne un groupe phénylène optionnellement substitué par halo, C₁-C₄-alkyle, C₁-C₄-alkoxy ou par C₁-C₄-alkoxy substitué par phényle,
R¹ et R² sont attachés à des atomes de carbone adjacents dans Ar, et
soit R¹ est C₁-C₄-alkylène et R² est hydrogène,
soit R¹ et R² conjointement avec les atomes de carbone dans Ar auxquels ils sont attachés désignent un anneau cycloaliphatique à 5, 6 ou 7 chaînons, plus particulièrement un anneau cycloaliphatique à 5 chaînons,
R^{a} est une liaison ou C₁-C₄-alkylène optionnellement substitué par hydroxy, C₆-C₈-aryle ou C₇-C₁₀-aralkyle, et
Y est C₃-C₆-cycloalkyle optionnellement fusionné à un ou plusieurs anneaux benzéniques et optionnellement substitué par C₁-C₆-alkyle, C₃-C₆-cycloalkyle, C₇-C₁₀-aralkyle, C₇-C₁₀-aralkyloxy optionnellement substitué par halo, ou par C₆-C₈-aryle optionnellement substitué par C₁-C₄-alkyle ou C₁-C₄-alkoxy, C₆-C₈-aryle optionnellement substitué par halo, hydroxy, C₁-C₄-alkyle, phénoxy, C₁-C₄-alkylthio, C₆-C₈-aryle, un anneau hétérocyclique de 4 à 8 chaînons possédant au moins un atome d'azote d'anneau, ou par NR^{b}R^{c} où R^{b} et R^{c} sont chacun indépendamment C₁-C₄-alkyle optionnellement substitué par hydroxy ou phényle ou R^{b} peut additionnellement être hydrogène, phénoxy optionnellement substitué par C₁-C₄-alkoxy, un anneau hétérocyclique de 4 à 8 chaînons possédant au moins un atome d'oxygène ou d'azote d'anneau, ledit anneau hétérocyclique étant optionnellement substitué par C₁-C₄-alkyle, C₆-C₈-aryle, C₇-C₁₀-aralkyle, C₁-C₄-alkoxycarbonyl ou par un hétérocyclyle-C₁-C₄-alkyle de 4 à 8 chaînons, -NR^{d}R^{e} où R^{d} est hydrogène ou C₁-C₄-alkyle et R^{e} est C₁-C₄-alkyle, ou R^{e} est un anneau hétérocyclique de 4 à 8 chaînons possédant au moins un atome de soufre ou d'azote d'anneau dont l'anneau est optionnellement substitué par phényle à substitution halo ou R^{e} est C₆-C₈-arylsulfonyle optionnellement substitué par di(C₁-C₄-alkyle)amino, -SR^{f} où R^{f} est C₆-C₈-aryle ou C₇-C₁₀-aralkyle optionnellement substitué par halo ou C₁-C₄-haloalkyle, ou -CONHR^{g} où R^{g} est C₃-C₆-cycloalkyle ou C₆-C₈-aryle.

4. Composé selon la revendication 1 sous forme libre, de sel ou de solvate, où
X est -R¹-Ar-R² ou -R^{a}-Y,
Ar désigne un groupe phénylène optionnellement substitué par halo, hydroxy, C₁-C₁₀-alkyle, C₁-C₁₀-alkoxy, C₁-C₁₀-alkoxy-C₁-C₁₀-alkyle, phényle, C₁-C₁₀-alkyle substitué par phényle, C₁-C₁₀-alkoxy substitué par phényle, C₁-C₁₀-alkyle-phényle substitué ou par C₁-C₁₀-alkoxy-phényle substitué,
R¹ et R² sont attachés à des atomes de carbone adjacents dans Ar, et
soit R¹ est C₁-C₁₀-alkylène et R² est hydrogène, C₁-C₁₀-alkyle, C₁-C₁₀-alkoxy ou halogène soit R¹ et R² conjointement avec les atomes de carbone dans Ar auxquels ils sont attachés désignent un anneau cycloaliphatique à 5, 6 ou 7 chaînons,
R^{a} est une liaison ou C₁-C₁₀-alkylène optionnellement substitué par hydroxy, C₁-C₁₀-alkoxy, C₆-C₁₀-aryle ou C₇-C₁₄-aralkyle, et
Y est C₃-C₁₀-cycloalkyle optionnellement fusionné à un ou plusieurs anneaux benzéniques et optionnellement substitué par C₁-C₁₀-alkyle, C₁-C₁₀-alkoxy, C₃-C₁₀-cycloalkyle, C₇-C₁₄-aralkyle, C₇-C₁₄-aralkyloxy ou C₆-C₁₀-aryle optionnellement substitué par halo, hydroxy, C₁-C₁₀-alkyle, C₁-C₁₀-alkoxy ou halo-C₁-C₁₀-alkyle, C₆-C₁₀-aryle optionnellement substitué par halo, hydroxy, C₁-C₁₀-alkyle, C₁-C₁₀-alkoxy, C₁-C₁₀-haloalkyle, phénoxy, C₁-C₁₀-alkylthio, C₆-C₁₀-aryle, un anneau hétérocyclique de 4 à 10 chaînons possédant au moins un atome de soufre, d'oxygène ou d'azote d'anneau, ou par NR^{b}R^{c} où R^{b} et R^{c} sont chacun indépendamment C₁-C₁₀-alkyle optionnellement substitué par hydroxy, C₁-C₁₀-alkoxy ou phényle ou R^{b} peut additionnellement être hydrogène,
phénoxy optionnellement substitué par C₁-C₁₀-alkyle, C₁-C₁₀-alkoxy ou par phényle optionnellement substitué par C₁-C₁₀-alkyle ou C₁-C₁₀-alkoxy,
un anneau hétérocyclique de 4 à 10 chaînons possédant au moins un atome de soufre, d'oxygène ou d'azote d'anneau, ledit anneau hétérocyclique étant optionnellement substitué par halo, C₁-C₁₀-alkyle, C₁-C₁₀-alkoxy, halo-C₁-C₁₀-alkyle, C₆-C₁₀-aryle, C₇-C₁₄-aralkyle, C₇-C₁₄-aralkyloxy, C₁-C₁₀-alkoxycarbonyl ou un hétérocyclyle-C₁-C₁₀-alkyle de 4 à 10 chaînons,
-NR^{d}R^{e} où R^{d} est hydrogène ou C₁-C₁₀-alkyle et R^{e} est C₁-C₁₀-alkyle optionnellement substitué par hydroxy, ou R^{e} est C₆-C₁₀-aryle optionnellement substitué par halo, ou R^{e} est un anneau hétérocyclique de 4 à 10 chaînons possédant au moins un atome de soufre, d'oxygène ou d'azote d'anneau dont l'anneau est optionnellement substitué par phényle ou phényle à substitution halo ou R^{e} est C₆-C₁₀-arylsulfonyle optionnellement substitué par C₁-C₁₀-alkylamino ou di(C₁-C₁₀-alkyle)-amino,
-SR^{f} où R^{f} est C₆-C₁₀-aryle ou C₇-C₁₄-aralkyle optionnellement substitué par halo, C₁-C₁₀-alkyle, C₁-C₁₀-alkoxy ou C₁-C₁₀-haloalkyle, ou
-CONHR^{g} où R^{g} est C₁-C₁₀-alkyle, C₃-C₁₀-cycloalkyle ou C₆-C₁₀-aryle.

5. Composé selon la revendication 4, dans lequel
X est -R¹-Ar-R² ou -R^{a}-Y,
Ar désigne un groupe phénylène optionnellement substitué par halo, C₁-C₁₀-alkyle, C₁-C₁₀-alkoxy ou par C₁-C₁₀-alkoxy substitué par phényle,
R¹ et R² sont attachés à des atomes de carbone adjacents dans Ar, et
soit R¹ est C₁-C₁₀-alkylène et R² est hydrogène,
soit R¹ et R² conjointement avec les atomes de carbone dans Ar auxquels ils sont attachés désignent un anneau cycloaliphatique à 5, 6 ou 7 chaînons, R^{a} est une liaison ou C₁-C₁₀-alkylène optionnellement substitué par hydroxy, C₆-C₁₀-aryle ou C₇-C₁₄-aralkyle, et
Y est C₃-C₁₀-cycloalkyle optionnellement fusionné à un ou plusieurs anneaux benzéniques et optionnellement substitué par C₁-C₁₀-alkyle, C₃-C₁₀-cycloalkyle, C₇-C₁₄-aralkyle, C₇-C₁₄-aralkyloxy ou C₆-C₁₀-aryle, C₆-C₁₀-aryle optionnellement substitué par halo, hydroxy, C₁-C₁₀-alkyle, phénoxy, C₁-C₁₀-alkylthio, C₆-C₁₀-aryle, un anneau hétérocyclique de 4 à 10 chaînons possédant au moins un atome d'azote d'anneau, ou par NR^{b}R^{c} où R^{b} et R^{c} sont chacun indépendamment C₁-C₁₀-alkyle optionnellement substitué par hydroxy ou phényle ou R^{b} peut additionnellement être hydrogène, phénoxy optionnellement substitué par C₁-C₁₀-alkoxy, un anneau hétérocyclique de 4 à 10 chaînons possédant au moins un atome d'oxygène ou d'azote d'anneau, ledit anneau hétérocyclique étant optionnellement substitué par C₁-C₁₀-alkyle, C₆-C₁₀-aryle, C₇-C₁₄-aralkyle, C₁-C₁₀-alkoxycarbonyl ou par un hétérocyclyle-C₁-C₁₀-alkyle de 4 à 10 chaînons, -NR^{d}R^{e} où R^{d} est hydrogène ou C₁-C₁₀-alkyle et R^{e} est C₁-C₁₀-alkyle, ou R^{e} est un anneau hétérocyclique de 4 à 10 chaînons possédant au moins un atome d'oxygène ou d'azote d'anneau dont l'anneau est optionnellement substitué par phényle à substitution halo ou R^{e} est C₆-C₁₀-arylsulfonyle optionnellement substitué par di(C₁-C₁₀-alkyle)amino, -SR^{f} où R^{f} est C₆-C₁₀-aryle ou C₇-C₁₄-aralkyle optionnellement substitué par halo ou C₁-C₁₀-haloalkyle, ou - CONHR^{g} où R^{g} est C₃-C₁₀-cycloalkyle ou C₆-C₁₀-aryle.

6. Composé selon la revendication 4, dans lequel
X est -R¹-Ar-R² ou R^{a}-Y,
Ar désigne un groupe phénylène optionnellement substitué par halo, C₁-C₄-alkyle, C₁-C₄-alkoxy ou par C₁-C₄-alkoxy substitué par phényle,
R¹ et R² sont attachés à des atomes de carbone adjacents dans Ar, et
soit R¹ est C₁-C₄-alkylène et R² est hydrogène,
soit R¹ et R² conjointement avec les atomes de carbone dans Ar auxquels ils sont attachés désignent un anneau cycloaliphatique à 5, 6 ou 7 chaînons, plus particulièrement un anneau cycloaliphatique à 5 chaînons,
R^{a} est une liaison ou C₁-C₄-alkylène optionnellement substitué par hydroxy, C₆-C₈-aryle ou C₇-C₁₀-aralkyle, et
Y est C₃-C₆-cycloalkyle optionnellement fusionné à un ou plusieurs anneaux benzéniques et optionnellement substitué par C₁-C₄-alkyle, C₃-C₆-cycloalkyle, C₇-C₁₀-aralkyle, C₇-C₁₀-aralkyloxy ou C₆-C₈-aryle, C₆-C₈-aryle optionnellement substitué par halo, hydroxy, C₁-C₄-alkyle, phénoxy, C₁-C₄-alkylthio, C₆-C₈-aryle, un anneau hétérocyclique de 4 à 8 chaînons possédant au moins un atome d'azote d'anneau, ou par NR^{b}R^{c} où R^{b} et R^{c} sont chacun indépendamment C₁-C₄-alkyle optionnellement substitué par hydroxy ou phényle ou R^{b} peut additionnellement être hydrogène, phénoxy optionnellement substitué par C₁-C₄-alkoxy, un anneau hétérocyclique de 4 à 8 chaînons possédant au moins un atome d'oxygène ou d'azote d'anneau, ledit anneau hétérocyclique étant optionnellement substitué par C₁-C₄-alkyle, C₆-C₈-aryle, C₇-C₁₀-aralkyle, C₁-C₄-alkoxycarbonyl ou par un hétérocyclyle-C₁-C₄-alkyle de 4 à 8 chaînons, -NR^{d}R^{e} où R^{d} est hydrogène ou C₁-C₄-alkyle et R^{e} est C₁-C₄-alkyle, ou R^{e} est un anneau hétérocyclique de 4 à 8 chaînons possédant au moins un atome de soufre ou d'azote d'anneau dont l'anneau est optionnellement substitué par phényle à substitution halo ou R^{e} est C₆-C₈-arylsulfonyle optionnellement substitué par di(C₁-C₄-alkyle)amino, -SR^{f} où R^{f} est C₆-C₈-aryle ou C₇-C₁₀-aralkyle optionnellement substitué par halo ou C₁-C₄-haloalkyle, ou -CONHR^{g} où R^{g} est C₃-C₆-cycloalkyle ou C₆-C₈-aryle.

7. Composé selon la revendication 1 qui est également un composé selon la formule II 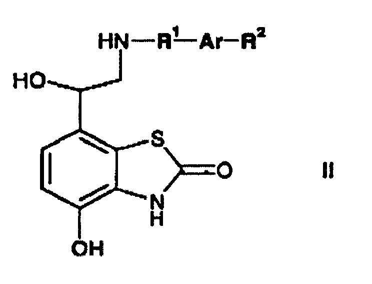 sous forme libre, de sel ou de solvate, où
Ar désigne un groupe phénylène optionnellement substitué par un ou plusieurs substituants sélectionnés parmi halogène, C₁-C₈-alkyle, C₁-C₈-alkoxy, C₁-C₈-alkoxy-C₁-C₈-alkyle, ou C₁-C₈-alkoxy substitué par phényle, C₁-C₈-alkyle-phényle substitué ou par C₁-C₈-alkoxy-phényle substitué,
R¹ et R² sont attachés à des atomes de carbone adjacents dans Ar, et
soit R¹ est C₁-C₈-alkylène et R² est hydrogène, C₁-C₈-alkyl, C₁-C₈-alkoxy ou halogène soit R¹ et R² conjointement avec les atomes de carbone dans Ar auxquels ils sont attachés désignent un anneau cycloaliphatique à 5, 6 ou 7 chaînons.

8. Composé selon la revendication 7 qui est également un composé selon la formule III 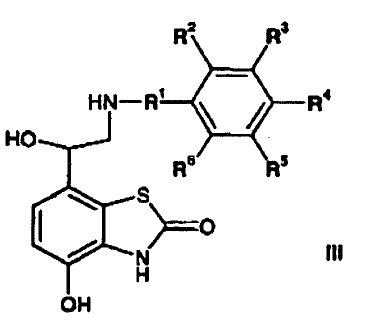 sous forme libre, de sel ou de solvate, où R¹ est C₂-C₄-alkylène et R² est hydrogène, ou R¹ et R² conjointement avec les atomes de carbone auxquels ils sont attachés sur l'anneau benzénique indiqué désignent un anneau cycloaliphatique à 5 chaînons, R³ et R⁶ sont chacun hydrogène, R⁴ est hydrogène, C₁-C₄-alkyle, C₁-C₄-alkoxy ou C₁-C₄-alkoxy substitué par phényle et R⁵ est hydrogène ou C₁-C₄-alkyle.

9. Composé selon la formule I tel que défini à la revendication 1 qui est sélectionné dans le groupe se composant de :
4-hydroxy-7-(1-hydroxy-2-[2-[4-(4-phényle-butoxy)-phényle]-éthylamino]-éthyle)-3H-benzothiazole-2-one,
4-hydroxy-7-[1-hydroxy-2-(indan-2-ylamino)-éthyle]-3H-benzothiazole-2-one,
4-hydroxy-7-[1-hydroxy-2-[(R)-2-(4-méthoxy-phényle)-1-méthyle-éthylamino]-éthyle)-3H-benzothiazole-2-one,
4-hydroxy-7-[1-hydroxy-2-[2-(4-isobutyle-phényle)-éthylamino]-éthyle]-3H-benzothiazole-2-one,
4-hydroxy-7-[1-hydroxy-2-[2-(4-propyle-phényle)-éthylamino]-éthyle]-3H-benzothiazole-2-one,
4-hydroxy-7-[(R)-1-hydroxy-2-[2-(4-propyle-phényle)-éthylamino]-éthyle]-3H-benzothiazole-2-one hydrochlorure,
4-hydroxy-7-[(S)-1-hydroxy-2-[2-(4-propyle-phényle)-éthylamino]-éthyle)-3H-benzothiazole-2-one hydrochlorure
7-[2-(5,6-diéthyle-indan-2-ylamino)-1-hydroxy-éthyle]-4-hydroxy-3H-benzothiazole-2-one et
4-hydroxy-7-[(R)-1-hydroxy-2-[2-(3-propyle-phényle)-éthylamino]-éthyle]-3H-benzothiazole-2-one hydrochlorure.

10. Composé selon la formule I tel que défini à la revendication 1 qui est sélectionné dans le groupe se composant de :
7-[(R)-2-[2-(4-Ethoxy-3-méthoxy-phényle)-éthylamino]-1-hydroxy-éthyle]-4-hydroxy-3H-benzothiazole-2-one trifluoroacétate,
7-[(R)-2-(1,1-Diméthyle-2-phényle-éthylamino)-1-hydroxy-éthyle]-4-hydroxy-3H-benzothiazole-2-one,
7-[(R)-2-(2-(3,4-Diéthyle-phényle)-éthylamino]-1-hydroxy-éthyle]-4-hydroxy-3H-benzothiazole-2-one,
4-Hydroxy-7-[(R)-1-hydroxy-2-[2-(5,6,7,8-tétrahydro-naphthalène-2-yl)-éthylaminol-éthyle]-3H-benzothiazole-2-one formiate et
7-[(R)-2-((1S, 2S)-2-Benzyloxy-cyclopentylamino)-1-hydroxy-éthyle]-4-hydroxy-3H-benzothiazole-2-one.

11. Composé selon la formule I tel que défini à la revendication 1 qui est 7-[(R)-2-((1S, 2R)-2-Benzyloxy-cyclopentylamino)-1-hydroxy-éthyle]-4-hydroxy-3H-benzothiazole-2-one.

12. Composé selon l'une quelconque des revendications précédentes destiné à un usage en tant que produit pharmaceutique.

13. Composition pharmaceutique comprenant en tant qu'ingrédient actif un composé selon l'une quelconque des revendications 1 à 11, optionnellement conjointement avec un diluant ou support pharmaceutiquement acceptable à cet effet.

14. Composition pharmaceutique comprenant en tant qu'ingrédient actif un composé selon l'une quelconque des revendications 1 à 11, en combinaison avec une substance médicamenteuse anti-inflammatoire, bronchodilatatrice ou antihistaminique, optionnellement conjointement avec un diluant ou support pharmaceutiquement acceptable à cet effet.

15. Utilisation d'un composé selon l'une quelconque des revendications 1 à 11 pour la fabrication d'un médicament pour le traitement d'un état qui est évité ou atténué par l'activation du récepteur adrénergique β₂.

16. Utilisation d'un composé selon l'une quelconque des revendications 1 à 11 pour la fabrication d'un médicament pour le traitement d'un syndrome obstructif ou inflammatoire des voies aériennes.

17. Processus destiné à la préparation d'un composé selon la formule I selon la revendication 1 qui comprend :
(i) soit (A) réagissant un composé selon la formule IV 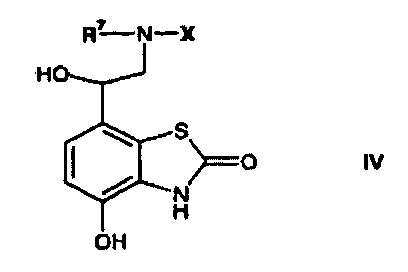 où X est tel que défini à la revendication 1 et R⁷ désigne un groupe de protection destiné à remplacer R⁷ par hydrogène,
ou (B) réagissant un composé selon la formule V 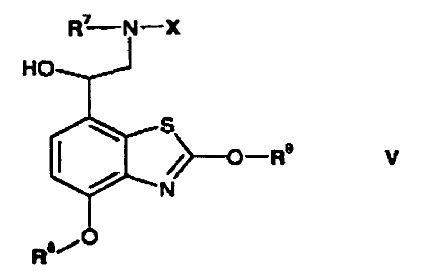 où X et R⁷ sont tels que définis plus haut dans le présent document et R⁸ et R⁹ désignent chacun indépendamment un groupe de protection destiné à convertir les groupes R⁷, R⁸ et R⁹ vers hydrogène, et
(ii) la récupération du composé selon la formule I sous forme libre, de sel ou de solvate.
